# EUROPEAN PATENT SPECIFICATION

(11) **EP 3 289 104 B1**
(45) Date of publication and mention of the grant of the patent: **04.11.2020**
(21) Application number: 16787255.5
(22) Date of filing: 29.04.2016
(51) Int. Cl.: A61P 35/00, C07K 14/705, C12N 15/113, C12Q 1/68, C12Q 1/6886, G01N 33/50, G01N 33/574

(54) **METHOD FOR TREATING HIGH-GRADE GLIOMAS**
VERFAHREN ZUR BEHANDLUNG VON HOCHWERTIGEN GLIOMEN
PROCÉDÉ POUR LE TRAITEMENT DE GLIOMES DE HAUT GRADE

(30) Priority: 29.04.2015 US 201562154173 P
(43) Date of publication of application: 07.03.2018
(73) Proprietor: New York University, New York City, NY 10012 (US)
(72) Inventor: PLACANTONAKIS, Dimitris, G., New York, NY 10021 (US); BAYIN, Nermin, Sumru, New York, NY 10010 (US); ZAGZAG, David, Rego Park, NY 11374 (US)
(74) Representative: Grünecker Patent- und Rechtsanwälte PartG mbB
(86) International application number: PCT/US2016/030201
(87) International publication number: WO 2016/176617

(56) References cited:
- US-A1- 2013 164 218
- NAOTOSHI SUGIMOTO ET AL: "Targeted activation of PKA and Epac promotes glioblastoma regression in vitro", MOLECULAR AND CLINICAL ONCOLOGY, vol. 1, no. 2, 1 January 2013 (2013-01-01) , pages 281-285, XP055506840, GR ISSN: 2049-9450, DOI: 10.3892/mco.2013.65
- KESSLER JACQUELINE ET AL: "HIF-1alpha inhibition by siRNA or chetomin in human malignant glioma cells: effects on hypoxic radioresistance and monitoring via CA9 expression", BMC CANCER, BIOMED CENTRAL, LONDON, GB, vol. 10, no. 1, 4 November 2010 (2010-11-04), page 605, XP021075428, ISSN: 1471-2407, DOI: 10.1186/1471-2407-10-605
- TAN CHALET ET AL: "Identification of a novel small-molecule inhibitor of the hypoxia-inducible factor 1 pathway", CANCER RESEARCH, AMERICAN ASSOCIATION FOR CANCER RESEARCH, US, vol. 65, no. 2, 15 January 2005 (2005-01-15), pages 605-612, XP002607741, ISSN: 0008-5472
- WILLIAM B VANTI ET AL: "Novel human G-protein-coupled receptors", BIOCHEMICAL AND BIOPHYSICAL RESEARCH COMMUNICATIONS, vol. 305, no. 1, 1 May 2003 (2003-05-01), pages 67-71, XP055124818, ISSN: 0006-291X, DOI: 10.1016/S0006-291X(03)00709-5
- RUDIN ET AL.: 'Comprehensive genomic analysis identifies SOX2 as a frequently amplified gene in small- cell lung cancer.' NATURE GENETICS. vol. 44, no. ISSUE, October 2012, pages 1111 - 1116, XP055335227
- ZHANG ET AL.: 'Abstract A93: Genetic Variations in aging related genes/pathways and colorectal cancer risk.' PROCEEDINGS OF THE ELEVENTH ANNUAL AACR INTERNATIONAL CONFERENCE ON FRONTIERS IN CANCER PREVENTION RESEARCH; November 2012, XP009507130 Retrieved from the Internet: <URL:http://cancerpreventionresearch.aacrjo urnals.org/content/5/11_Supplement/A93.abst ract> [retrieved on 2016-08-04]
- 'CANCER GENOME ATLAS NETWORK, Comprehensive genomic characterization of squamous cell lung cancers.' NATURE vol. 489, no. ISSUE, 27 September 2012, pages 519 - 525, XP002725593
- BOHNEKAMP ET AL.: 'Cell adhesion receptor GPR133 couples to Gs protein.' J BIOL CHEM vol. 286, no. 49, 09 December 2011, pages 41912 - 41916, XP055335228
- PARK ET AL.: 'Identification of rare germline copy number variations over-represented in five human cancer types.' MOL. CANCER vol. 14, 03 February 2015, page 25, XP021220445
- SHASHIDHAR ET AL.: 'GPR56 is a GPCR that is overexpressed in gliomas and functions in tumor cell adhesion.' ONCOGENE vol. 24, no. 10, 03 March 2005, pages 1673 - 1682, XP002533126
- BEREZOVSKY ET AL.: 'Sox2 Promotes Malignancy in Glioblastoma by Regulating Plasticity and Astrocytic Differentiation.' NEOPLASIA. vol. 16, no. 3, March 2014, pages 193 - 206, XP055335232
- ANDRADAS ET AL.: 'The orphan G protein-coupled receptor GPR55 promotes cancer cell proliferation via ERK.' ONCOGENE vol. 30, no. 2, 13 January 2011, pages 245 - 252, XP055335233
- BAYIN ET AL.: 'GPR133 Promotes Glioblastoma Growth in Hypoxia' CLINICAL NEUROSURGERY vol. 58, pages 158 - 159, XP009507045 Retrieved from the Internet: <URL:file:///C:/Users/ming.chen/Downloads/1 44_GPR133_Promotes_Glioblastoma_Growth_in.7 4.pdf> [retrieved on 2016-08-05]

### SEQUENCE LISTING

<110> NEW YORK UNIVERSITY
<120> METHOD FOR TREATING HIGH-GRADE GLIOMAS
<130> 243735.000148
<140>
   <141>
<150> 62/154,173
   <151> 2015-04-29
<160> 24
<170> PatentIn version 3.5
<210> 1
   <211> 570
   <212> PRT
   <213> Homo sapiens
<400> 1
   Met Glu Lys Leu Leu Arg Leu Cys Cys Trp Tyr Ser Trp Leu Leu Leu
   1               5                   10                  15
   Phe Tyr Tyr Asn Phe Gln Val Arg Gly Val Tyr Ser Arg Ser Gln Asp
               20                  25                  30
   His Pro Gly Phe Gln Val Leu Ala Ser Ala Ser His Tyr Trp Pro Leu
           35                  40                  45
   Glu Asn Val Asp Gly Ile His Glu Leu Gln Asp Thr Thr Gly Asp Ile
       50                  55                  60
   Val Glu Gly Lys Val Asn Lys Gly Ile Tyr Leu Lys Glu Glu Lys Gly
   65                  70                  75                  80
   Val Thr Leu Leu Tyr Tyr Gly Arg Tyr Asn Ser Ser Cys Ile Ser Lys
                   85                  90                  95
   Pro Glu Gln Cys Gly Pro Glu Gly Val Thr Phe Ser Phe Phe Trp Lys
               100                 105                 110
   Thr Gln Gly Glu Gln Ser Arg Pro Ile Pro Ser Ala Tyr Gly Gly Gln
           115                 120                 125
   Val Ile Ser Asn Gly Phe Lys Val Cys Ser Ser Gly Gly Arg Gly Ser
       130                 135                 140
   Val Glu Leu Tyr Thr Arg Asp Asn Ser Met Thr Trp Glu Ala Ser Phe
   145                 150                 155                 160
   Ser Pro Pro Gly Pro Tyr Trp Thr His Val Leu Phe Thr Trp Lys Ser
                   165                 170                 175
   Lys Glu Gly Leu Lys Val Tyr Val Asn Gly Thr Leu Ser Thr Ser Asp
               180                 185                 190
   Pro Ser Gly Lys Val Ser Arg Asp Tyr Gly Glu Ser Asn Val Asn Leu
           195                 200                 205
   Val Ile Gly Ser Glu Gln Asp Gln Ala Lys Cys Tyr Glu Asn Gly Ala
       210                 215                 220
   Phe Asp Glu Phe Ile Ile Trp Glu Arg Ala Leu Thr Pro Asp Glu Ile
   225                 230                 235                 240
   Ala Met Tyr Phe Thr Ala Ala Ile Gly Lys His Ala Leu Leu Ser Ser
                   245                 250                 255
   Thr Leu Pro Ser Leu Phe Met Thr Ser Thr Ala Ser Pro Val Met Pro
               260                 265                 270
   Thr Asp Ala Tyr His Pro Ile Ile Thr Asn Leu Thr Glu Glu Arg Lys
           275                 280                 285
   Thr Phe Gln Ser Pro Gly Val Ile Leu Ser Tyr Leu Gln Asn Val Ser
       290                 295                 300
   Leu Ser Leu Pro Ser Lys Ser Leu Ser Glu Gln Thr Ala Leu Asn Leu
   305                 310                 315                 320
   Thr Lys Thr Phe Leu Lys Ala Val Gly Glu Ile Leu Leu Leu Pro Gly
                   325                 330                 335
   Trp Ile Ala Leu Ser Glu Asp Ser Ala Val Val Leu Ser Leu Ile Asp
               340                 345                 350
   Thr Ile Asp Thr Val Met Gly His Val Ser Ser Asn Leu His Gly Ser
           355                 360                 365
   Thr Pro Gln Val Thr Val Glu Gly Ser Ser Ala Met Ala Glu Phe Ser
       370                 375                 380
   Val Ala Lys Ile Leu Pro Lys Thr Val Asn Ser Ser His Tyr Arg Phe
   385                 390                 395                 400
   Pro Ala His Gly Gln Ser Phe Ile Gln Ile Pro His Glu Ala Phe His
                   405                 410                 415
   Arg His Ala Trp Ser Thr Val Val Gly Leu Leu Tyr His Ser Met His
               420                 425                 430
   Tyr Tyr Leu Asn Asn Ile Trp Pro Ala His Thr Lys Ile Ala Glu Ala
           435                 440                 445
   Met His His Gln Asp Cys Leu Leu Phe Ala Thr Ser His Leu Ile Ser
       450                 455                 460
   Leu Glu Val Ser Pro Pro Pro Thr Leu Ser Gln Asn Leu Ser Gly Ser
   465                 470                 475                 480
   Pro Leu Ile Thr Val His Leu Lys His Arg Leu Thr Arg Lys Gln His
                   485                 490                 495
   Ser Glu Ala Thr Asn Ser Ser Asn Arg Val Phe Val Tyr Cys Ala Phe
               500                 505                 510
   Leu Asp Phe Ser Ser Gly Glu Gly Val Trp Ser Asn His Gly Cys Ala
           515                 520                 525
   Leu Thr Arg Gly Asn Leu Thr Tyr Ser Val Cys Arg Cys Thr His Leu
       530                 535                 540
   Thr Asn Phe Ala Ile Leu Met Gln Val Val Pro Leu Glu Leu Ala Arg
   545                 550                 555                 560
   Gly His Gln Val Ala Leu Ser Ser Ile Ser
                   565                 570
<210> 2
   <211> 256
   <212> PRT
   <213> Homo sapiens
<400> 2
   Met Glu Lys Gly Thr Glu Leu Leu Val Ser Pro Ser Gln Ser Gly Pro
   1               5                   10                  15
   Gly Gly Asp Gln Pro Leu Leu Val Lys His Arg Glu Asp Ser Ala Val
               20                  25                  30
   Val Leu Ser Leu Ile Asp Thr Ile Asp Thr Val Met Gly His Val Ser
           35                  40                  45
   Ser Asn Leu His Gly Ser Thr Pro Gln Val Thr Val Glu Gly Ser Ser
       50                  55                  60
   Ala Met Ala Glu Phe Ser Val Ala Lys Ile Leu Pro Lys Thr Val Asn
   65 70 75 80
   Ser Ser His Tyr Arg Phe Pro Ala His Gly Gln Ser Phe Ile Gln Ile
   85 90 95
   Pro His Glu Ala Phe His Arg His Ala Trp Ser Thr Val Val Gly Leu
   100 105 110
   Leu Tyr His Ser Met His Tyr Tyr Leu Asn Asn Ile Trp Pro Ala His
   115 120 125
   Thr Lys Ile Ala Glu Ala Met His His Gln Asp Cys Leu Leu Phe Ala
   130 135 140
   Thr Ser His Leu Ile Ser Leu Glu Val Ser Pro Pro Pro Thr Leu Ser
   145 150 155 160
   Gln Asn Leu Ser Gly Ser Pro Leu Ile Thr Val His Leu Lys His Arg
   165 170 175
   Leu Thr Arg Lys Gln His Ser Glu Ala Thr Asn Ser Ser Asn Arg Val
   180 185 190
   Phe Val Tyr Cys Ala Phe Leu Asp Phe Ser Ser Gly Glu Gly Val Trp
   195 200 205
   Ser Asn His Gly Cys Ala Leu Thr Arg Gly Asn Leu Thr Tyr Ser Val
   210 215 220
   Cys Arg Cys Thr His Leu Thr Asn Phe Ala Ile Leu Met Gln Val Val
   225 230 235 240
   Pro Leu Glu Leu Ala Arg Gly His Gln Val Ala Leu Ser Ser Ile Ser
   245 250 255
<210> 3
   <211> 89
   <212> PRT
   <213> Homo sapiens
<400> 3
   Met His Arg Val Cys Phe Leu Ser Phe Gln Thr Arg Lys Gln His Ser
   1 5 10 15
   Glu Ala Thr Asn Ser Ser Asn Arg Val Phe Val Tyr Cys Ala Phe Leu
   20 25 30
   Asp Phe Ser Ser Gly Glu Gly Val Trp Ser Asn His Gly Cys Ala Leu
   35 40 45
   Thr Arg Gly Asn Leu Thr Tyr Ser Val Cys Arg Cys Thr His Leu Thr
   50 55 60
   Asn Phe Ala Ile Leu Met Gln Val Val Pro Leu Glu Leu Ala Arg Gly
   65 70 75 80
   His Gln Val Ala Leu Ser Ser Ile Ser
   85
<210> 4
   <211> 602
   <212> PRT
   <213> Homo sapiens
<400> 4
   Met Glu Lys Leu Leu Arg Leu Cys Cys Trp Tyr Ser Trp Leu Leu Leu
   1               5                   10                  15
   Phe Tyr Tyr Asn Phe Gln Val Arg Gly Val Tyr Ser Arg Ser Gln Asp
               20                  25                  30
   His Pro Gly Phe Gln Val Leu Ala Ser Ala Ser His Tyr Trp Pro Leu
           35                  40                  45
   Glu Asn Val Asp Gly Ile His Glu Leu Gln Asp Thr Thr Gly Ala Ser
       50                  55                  60
   Arg Thr His Lys Leu Thr Val Leu Pro Ser Arg Asn Ala Thr Phe Val
   65                  70                  75                  80
   Tyr Ser Asn Asp Ser Ala Tyr Ser Asn Leu Ser Ala Thr Val Asp Ile
                   85                  90                  95
   Val Glu Gly Lys Val Asn Lys Gly Ile Tyr Leu Lys Glu Glu Lys Gly
               100                 105                 110
   Val Thr Leu Leu Tyr Tyr Gly Arg Tyr Asn Ser Ser Cys Ile Ser Lys
           115                 120                 125
   Pro Glu Gln Cys Gly Pro Glu Gly Val Thr Phe Ser Phe Phe Trp Lys
       130                 135                 140
   Thr Gln Gly Glu Gln Ser Arg Pro Ile Pro Ser Ala Tyr Gly Gly Gln
   145                 150                 155                 160
   Val Ile Ser Asn Gly Phe Lys Val Cys Ser Ser Gly Gly Arg Gly Ser
   165 170 175
   Val Glu Leu Tyr Thr Arg Asp Asn Ser Met Thr Trp Glu Ala Ser Phe
   180 185 190
   Ser Pro Pro Gly Pro Tyr Trp Thr His Val Leu Phe Thr Trp Lys Ser
   195 200 205
   Lys Glu Gly Leu Lys Val Tyr Val Asn Gly Thr Leu Ser Thr Ser Asp
   210 215 220
   Pro Ser Gly Lys Val Ser Arg Asp Tyr Gly Glu Ser Asn Val Asn Leu
   225 230 235 240
   Val Ile Gly Ser Glu Gln Asp Gln Ala Lys Cys Tyr Glu Asn Gly Ala
   245 250 255
   Phe Asp Glu Phe Ile Ile Trp Glu Arg Ala Leu Thr Pro Asp Glu Ile
   260 265 270
   Ala Met Tyr Phe Thr Ala Ala Ile Gly Lys His Ala Leu Leu Ser Ser
   275 280 285
   Thr Leu Pro Ser Leu Phe Met Thr Ser Thr Ala Ser Pro Val Met Pro
   290 295 300
   Thr Asp Ala Tyr His Pro Ile Ile Thr Asn Leu Thr Glu Glu Arg Lys
   305 310 315 320
   Thr Phe Gln Ser Pro Gly Val Ile Leu Ser Tyr Leu Gln Asn Val Ser
   325 330 335
   Leu Ser Leu Pro Ser Lys Ser Leu Ser Glu Gln Thr Ala Leu Asn Leu
   340 345 350
   Thr Lys Thr Phe Leu Lys Ala Val Gly Glu Ile Leu Leu Leu Pro Gly
   355 360 365
   Trp Ile Ala Leu Ser Glu Asp Ser Ala Val Val Leu Ser Leu Ile Asp
   370 375 380
   Thr Ile Asp Thr Val Met Gly His Val Ser Ser Asn Leu His Gly Ser
   385 390 395 400
   Thr Pro Gln Val Thr Val Glu Gly Ser Ser Ala Met Ala Glu Phe Ser
   405 410 415
   Val Ala Lys Ile Leu Pro Lys Thr Val Asn Ser Ser His Tyr Arg Phe
               420                 425                 430
   Pro Ala His Gly Gln Ser Phe Ile Gln Ile Pro His Glu Ala Phe His
           435                 440                 445
   Arg His Ala Trp Ser Thr Val Val Gly Leu Leu Tyr His Ser Met His
       450                 455                 460
   Tyr Tyr Leu Asn Asn Ile Trp Pro Ala His Thr Lys Ile Ala Glu Ala
   465                 470                 475                 480
   Met His His Gln Asp Cys Leu Leu Phe Ala Thr Ser His Leu Ile Ser
                   485                 490                 495
   Leu Glu Val Ser Pro Pro Pro Thr Leu Ser Gln Asn Leu Ser Gly Ser
               500                 505                 510
   Pro Leu Ile Thr Val His Leu Lys His Arg Leu Thr Arg Lys Gln His
           515                 520                 525
   Ser Glu Ala Thr Asn Ser Ser Asn Arg Val Phe Val Tyr Cys Ala Phe
       530                 535                 540
   Leu Asp Phe Ser Ser Gly Glu Gly Val Trp Ser Asn His Gly Cys Ala
   545                 550                 555                 560
   Leu Thr Arg Gly Asn Leu Thr Tyr Ser Val Cys Arg Cys Thr His Leu
                   565                 570                 575
   Thr Asn Phe Ala Ile Leu Met Gln Val Val Pro Leu Glu Leu Ala Arg
               580                 585                 590
   Gly His Gln Val Ala Leu Ser Ser Ile Ser
           595                 600
<210> 5
   <211> 21
   <212> DNA
   <213> Artificial Sequence
<220>
   <223> Description of Artificial Sequence: Synthetic oligonucleotide
<400> 5
   gggatcatag atgtgaatta a 21
<210> 6
   <211> 21
   <212> DNA
   <213> Artificial Sequence
<220>
   <223> Description of Artificial Sequence: Synthetic oligonucleotide
<400> 6
   ggagtcacgc ttctctatta c 21
<210> 7
   <211> 21
   <212> DNA
   <213> Artificial Sequence
<220>
   <223> Description of Artificial Sequence: Synthetic oligonucleotide
<400> 7
   cctgcaggga ctgttcatat t 21
<210> 8
   <211> 130
   <212> PRT
   <213> Homo sapiens
<400> 8
   Val Asn Lys Gly Ile Tyr Leu Lys Glu Glu Lys Gly Val Thr Leu Leu
   1               5                   10                  15
   Tyr Tyr Gly Arg Tyr Asn Ser Ser Cys Ile Ser Lys Pro Glu Gln Cys
               20                  25                  30
   Gly Pro Glu Gly Val Thr Phe Ser Phe Phe Trp Lys Thr Gln Gly Glu
           35                  40                  45
   Gln Ser Arg Pro Ile Pro Ser Ala Tyr Gly Gly Gln Val Ile Ser Asn
       50                  55                  60
   Gly Phe Lys Val Cys Ser Ser Gly Gly Arg Gly Ser Val Glu Leu Tyr
   65                  70                  75                  80
   Thr Arg Asp Asn Ser Met Thr Trp Glu Ala Ser Phe Ser Pro Pro Gly
                   85                  90                  95
   Pro Tyr Trp Thr His Val Leu Phe Thr Trp Lys Ser Lys Glu Gly Leu
               100                 105                 110
   Lys Val Tyr Val Asn Gly Thr Leu Val Ile Gly Ser Glu Gln Asp Gln
           115                 120                 125
   Ala Lys
   130
<210> 9
   <211> 110
   <212> PRT
   <213> Homo sapiens
<400> 9
   Ala Val Val Leu Ser Leu Ile Asp Thr Ile Asp Thr Val Met Gly His
   1               5                   10                  15
   Val Ser Ser Asn Leu His Gly Ser Thr Pro Gln Val Thr Val Glu Gly
               20                  25                  30
   Ser Ser Ala Met Ala Glu Phe Ser Val Ala Lys Ile Leu Pro Lys Thr
           35                  40                  45
   Val Asn Ser Ser His Tyr Arg Phe Pro Ala His Gly Gln Ser Phe Ile
       50                  55                  60
   Gln Ile Pro His Glu Ala Phe His Arg His Ala Trp Ser Thr Val Val
   65                  70                  75                  80
   Gly Leu Leu Tyr His Ser Met His Tyr Tyr Leu Asn Asn Ile Trp Pro
                   85                  90                  95
   Ala His Thr Lys Ile Ala Glu Ala Met His His Gln Asp Cys
               100                 105                 110
<210> 10
   <211> 15
   <212> PRT
   <213> Homo sapiens
<400> 10
   Thr Arg Lys Gln His Ser Glu Ala Thr Asn Ser Ser Asn Arg Cys
   1               5                   10                  15
<210> 11
   <211> 12
   <212> PRT
   <213> Homo sapiens
<400> 11
   Cys Ser Ser Gly Glu Gly Val Trp Ser Asn His Gly
   1               5                   10
<210> 12
   <211> 15
   <212> PRT
   <213> Homo sapiens
<400> 12
   Cys Ser Ser Ala Arg Thr Ser Asn Ala Lys Pro Phe His Ser Asp
   1               5                   10                  15
<210> 13
   <211> 16
   <212> PRT
   <213> Homo sapiens
<400> 13
   Leu Met Asn Gly Thr Arg Pro Gly Met Ala Ser Thr Lys Leu Ser Cys
   1               5                   10                  15
<210> 14
   <211> 21
   <212> DNA
   <213> Artificial Sequence
<220>
   <223> Description of Artificial Sequence: Synthetic oligonucleotide
<400> 14
   cctaaggtta agtcgccctc g 21
<210> 15
   <211> 874
   <212> PRT
   <213> Homo sapiens
<400> 15
   Met Glu Lys Leu Leu Arg Leu Cys Cys Trp Tyr Ser Trp Leu Leu Leu
   1               5                   10                  15
   Phe Tyr Tyr Asn Phe Gln Val Arg Gly Val Tyr Ser Arg Ser Gln Asp
               20                  25                  30
   His Pro Gly Phe Gln Val Leu Ala Ser Ala Ser His Tyr Trp Pro Leu
           35                  40                  45
   Glu Asn Val Asp Gly Ile His Glu Leu Gln Asp Thr Thr Gly Asp Ile
       50                  55                  60
   Val Glu Gly Lys Val Asn Lys Gly Ile Tyr Leu Lys Glu Glu Lys Gly
   65                  70                  75                  80
   Val Thr Leu Leu Tyr Tyr Gly Arg Tyr Asn Ser Ser Cys Ile Ser Lys
                   85                  90                  95
   Pro Glu Gln Cys Gly Pro Glu Gly Val Thr Phe Ser Phe Phe Trp Lys
               100                 105                 110
   Thr Gln Gly Glu Gln Ser Arg Pro Ile Pro Ser Ala Tyr Gly Gly Gln
           115                 120                 125
   Val Ile Ser Asn Gly Phe Lys Val Cys Ser Ser Gly Gly Arg Gly Ser
       130                 135                 140
   Val Glu Leu Tyr Thr Arg Asp Asn Ser Met Thr Trp Glu Ala Ser Phe
   145                 150                 155                 160
   Ser Pro Pro Gly Pro Tyr Trp Thr His Val Leu Phe Thr Trp Lys Ser
                   165                 170                 175
   Lys Glu Gly Leu Lys Val Tyr Val Asn Gly Thr Leu Ser Thr Ser Asp
               180                 185                 190
   Pro Ser Gly Lys Val Ser Arg Asp Tyr Gly Glu Ser Asn Val Asn Leu
           195                 200                 205
   Val Ile Gly Ser Glu Gln Asp Gln Ala Lys Cys Tyr Glu Asn Gly Ala
       210                 215                 220
   Phe Asp Glu Phe Ile Ile Trp Glu Arg Ala Leu Thr Pro Asp Glu Ile
   225                 230                 235                 240
   Ala Met Tyr Phe Thr Ala Ala Ile Gly Lys His Ala Leu Leu Ser Ser
                   245                 250                 255
   Thr Leu Pro Ser Leu Phe Met Thr Ser Thr Ala Ser Pro Val Met Pro
               260                 265                 270
   Thr Asp Ala Tyr His Pro Ile Ile Thr Asn Leu Thr Glu Glu Arg Lys
           275                 280                 285
   Thr Phe Gln Ser Pro Gly Val Ile Leu Ser Tyr Leu Gln Asn Val Ser
       290                 295                 300
   Leu Ser Leu Pro Ser Lys Ser Leu Ser Glu Gln Thr Ala Leu Asn Leu
   305                 310                 315                 320
   Thr Lys Thr Phe Leu Lys Ala Val Gly Glu Ile Leu Leu Leu Pro Gly
                   325                 330                 335
   Trp Ile Ala Leu Ser Glu Asp Ser Ala Val Val Leu Ser Leu Ile Asp
               340                 345                 350
   Thr Ile Asp Thr Val Met Gly His Val Ser Ser Asn Leu His Gly Ser
           355                 360                 365
   Thr Pro Gln Val Thr Val Glu Gly Ser Ser Ala Met Ala Glu Phe Ser
       370                 375                 380
   Val Ala Lys Ile Leu Pro Lys Thr Val Asn Ser Ser His Tyr Arg Phe
   385                 390                 395                 400
   Pro Ala His Gly Gln Ser Phe Ile Gln Ile Pro His Glu Ala Phe His
                   405                 410                 415
   Arg His Ala Trp Ser Thr Val Val Gly Leu Leu Tyr His Ser Met His
               420                 425                 430
   Tyr Tyr Leu Asn Asn Ile Trp Pro Ala His Thr Lys Ile Ala Glu Ala
           435                 440                 445
   Met His His Gln Asp Cys Leu Leu Phe Ala Thr Ser His Leu Ile Ser
       450                 455                 460
   Leu Glu Val Ser Pro Pro Pro Thr Leu Ser Gln Asn Leu Ser Gly Ser
   465                 470                 475                 480
   Pro Leu Ile Thr Val His Leu Lys His Arg Leu Thr Arg Lys Gln His
                   485                 490                 495
   Ser Glu Ala Thr Asn Ser Ser Asn Arg Val Phe Val Tyr Cys Ala Phe
               500                 505                 510
   Leu Asp Phe Ser Ser Gly Glu Gly Val Trp Ser Asn His Gly Cys Ala
           515                 520                 525
   Leu Thr Arg Gly Asn Leu Thr Tyr Ser Val Cys Arg Cys Thr His Leu
       530                 535                 540
   Thr Asn Phe Ala Ile Leu Met Gln Val Val Pro Leu Glu Leu Ala Arg
   545                 550                 555                 560
   Gly His Gln Val Ala Leu Ser Ser Ile Ser Tyr Val Gly Cys Ser Leu
                   565                 570                 575
   Ser Val Leu Cys Leu Val Ala Thr Leu Val Thr Phe Ala Val Leu Ser
               580                 585                 590
   Ser Val Ser Thr Ile Arg Asn Gln Arg Tyr His Ile His Ala Asn Leu
           595                 600                 605
   Ser Phe Ala Val Leu Val Ala Gln Val Leu Leu Leu Ile Ser Phe Arg
       610                 615                 620
   Leu Glu Pro Gly Thr Thr Pro Cys Gln Val Met Ala Val Leu Leu His
   625 630 635 640
   Tyr Phe Phe Leu Ser Ala Phe Ala Trp Met Leu Val Glu Gly Leu His
   645 650 655
   Leu Tyr Ser Met Val Ile Lys Val Phe Gly Ser Glu Asp Ser Lys His
   660 665 670
   Arg Tyr Tyr Tyr Gly Met Gly Trp Gly Phe Pro Leu Leu Ile Cys Ile
   675 680 685
   Ile Ser Leu Ser Phe Ala Met Asp Ser Tyr Gly Thr Ser Asn Asn Cys
   690 695 700
   Trp Leu Ser Leu Ala Ser Gly Ala Ile Trp Ala Phe Val Ala Pro Ala
   705 710 715 720
   Leu Phe Val Ile Val Val Asn Ile Gly Ile Leu Ile Ala Val Thr Arg
   725 730 735
   Val Ile Ser Gln Ile Ser Ala Asp Asn Tyr Lys Ile His Gly Asp Pro
   740 745 750
   Ser Ala Phe Lys Leu Thr Ala Lys Ala Val Ala Val Leu Leu Pro Ile
   755 760 765
   Leu Gly Thr Ser Trp Val Phe Gly Val Leu Ala Val Asn Gly Cys Ala
   770 775 780
   Val Val Phe Gln Tyr Met Phe Ala Thr Leu Asn Ser Leu Gln Gly Leu
   785 790 795 800
   Phe Ile Phe Leu Phe His Cys Leu Leu Asn Ser Glu Val Arg Ala Ala
   805 810 815
   Phe Lys His Lys Thr Lys Val Trp Ser Leu Thr Ser Ser Ser Ala Arg
   820 825 830
   Thr Ser Asn Ala Lys Pro Phe His Ser Asp Leu Met Asn Gly Thr Arg
   835 840 845
   Pro Gly Met Ala Ser Thr Lys Leu Ser Pro Trp Asp Lys Ser Ser His
   850 855 860
   Ser Ala His Arg Val Asp Leu Ser Ala Val
   865 870
<210> 16
   <211> 560
   <212> PRT
   <213> Homo sapiens
<400> 16
   Met Glu Lys Gly Thr Glu Leu Leu Val Ser Pro Ser Gln Ser Gly Pro
   1               5                   10                  15
   Gly Gly Asp Gln Pro Leu Leu Val Lys His Arg Glu Asp Ser Ala Val
               20                  25                  30
   Val Leu Ser Leu Ile Asp Thr Ile Asp Thr Val Met Gly His Val Ser
           35                  40                  45
   Ser Asn Leu His Gly Ser Thr Pro Gln Val Thr Val Glu Gly Ser Ser
       50                  55                  60
   Ala Met Ala Glu Phe Ser Val Ala Lys Ile Leu Pro Lys Thr Val Asn
   65                  70                  75                  80
   Ser Ser His Tyr Arg Phe Pro Ala His Gly Gln Ser Phe Ile Gln Ile
                   85                  90                  95
   Pro His Glu Ala Phe His Arg His Ala Trp Ser Thr Val Val Gly Leu
               100                 105                 110
   Leu Tyr His Ser Met His Tyr Tyr Leu Asn Asn Ile Trp Pro Ala His
           115                 120                 125
   Thr Lys Ile Ala Glu Ala Met His His Gln Asp Cys Leu Leu Phe Ala
       130                 135                 140
   Thr Ser His Leu Ile Ser Leu Glu Val Ser Pro Pro Pro Thr Leu Ser
   145                 150                 155                 160
   Gln Asn Leu Ser Gly Ser Pro Leu Ile Thr Val His Leu Lys His Arg
                   165                 170                 175
   Leu Thr Arg Lys Gln His Ser Glu Ala Thr Asn Ser Ser Asn Arg Val
               180                 185                 190
   Phe Val Tyr Cys Ala Phe Leu Asp Phe Ser Ser Gly Glu Gly Val Trp
           195                 200                 205
   Ser Asn His Gly Cys Ala Leu Thr Arg Gly Asn Leu Thr Tyr Ser Val
       210                 215                 220
   Cys Arg Cys Thr His Leu Thr Asn Phe Ala Ile Leu Met Gln Val Val
   225                 230                 235                 240
   Pro Leu Glu Leu Ala Arg Gly His Gln Val Ala Leu Ser Ser Ile Ser
                   245                 250                 255
   Tyr Val Gly Cys Ser Leu Ser Val Leu Cys Leu Val Ala Thr Leu Val
               260                 265                 270
   Thr Phe Ala Val Leu Ser Ser Val Ser Thr Ile Arg Asn Gln Arg Tyr
           275                 280                 285
   His Ile His Ala Asn Leu Ser Phe Ala Val Leu Val Ala Gln Val Leu
       290                 295                 300
   Leu Leu Ile Ser Phe Arg Leu Glu Pro Gly Thr Thr Pro Cys Gln Val
   305                 310                 315                 320
   Met Ala Val Leu Leu His Tyr Phe Phe Leu Ser Ala Phe Ala Trp Met
                   325                 330                 335
   Leu Val Glu Gly Leu His Leu Tyr Ser Met Val Ile Lys Val Phe Gly
               340                 345                 350
   Ser Glu Asp Ser Lys His Arg Tyr Tyr Tyr Gly Met Gly Trp Gly Phe
           355                 360                 365
   Pro Leu Leu Ile Cys Ile Ile Ser Leu Ser Phe Ala Met Asp Ser Tyr
       370                 375                 380
   Gly Thr Ser Asn Asn Cys Trp Leu Ser Leu Ala Ser Gly Ala Ile Trp
   385                 390                 395                 400
   Ala Phe Val Ala Pro Ala Leu Phe Val Ile Val Val Asn Ile Gly Ile
                   405                 410                 415
   Leu Ile Ala Val Thr Arg Val Ile Ser Gln Ile Ser Ala Asp Asn Tyr
               420                 425                 430
   Lys Ile His Gly Asp Pro Ser Ala Phe Lys Leu Thr Ala Lys Ala Val
           435                 440                 445
   Ala Val Leu Leu Pro Ile Leu Gly Thr Ser Trp Val Phe Gly Val Leu
       450                 455                 460
   Ala Val Asn Gly Cys Ala Val Val Phe Gln Tyr Met Phe Ala Thr Leu
   465                 470                 475                 480
   Asn Ser Leu Gln Gly Leu Phe Ile Phe Leu Phe His Cys Leu Leu Asn
   485 490 495
   Ser Glu Val Arg Ala Ala Phe Lys His Lys Thr Lys Val Trp Ser Leu
   500 505 510
   Thr Ser Ser Ser Ala Arg Thr Ser Asn Ala Lys Pro Phe His Ser Asp
   515 520 525
   Leu Met Asn Gly Thr Arg Pro Gly Met Ala Ser Thr Lys Leu Ser Pro
   530 535 540
   Trp Asp Lys Ser Ser His Ser Ala His Arg Val Asp Leu Ser Ala Val
   545 550 555 560
<210> 17
   <211> 393
   <212> PRT
   <213> Homo sapiens
<400> 17
   Met His Arg Val Cys Phe Leu Ser Phe Gln Thr Arg Lys Gln His Ser
   1 5 10 15
   Glu Ala Thr Asn Ser Ser Asn Arg Val Phe Val Tyr Cys Ala Phe Leu
   20 25 30
   Asp Phe Ser Ser Gly Glu Gly Val Trp Ser Asn His Gly Cys Ala Leu
   35 40 45
   Thr Arg Gly Asn Leu Thr Tyr Ser Val Cys Arg Cys Thr His Leu Thr
   50 55 60
   Asn Phe Ala Ile Leu Met Gln Val Val Pro Leu Glu Leu Ala Arg Gly
   65 70 75 80
   His Gln Val Ala Leu Ser Ser Ile Ser Tyr Val Gly Cys Ser Leu Ser
   85 90 95
   Val Leu Cys Leu Val Ala Thr Leu Val Thr Phe Ala Val Leu Ser Ser
   100 105 110
   Val Ser Thr Ile Arg Asn Gln Arg Tyr His Ile His Ala Asn Leu Ser
   115 120 125
   Phe Ala Val Leu Val Ala Gln Val Leu Leu Leu Ile Ser Phe Arg Leu
   130 135 140
   Glu Pro Gly Thr Thr Pro Cys Gln Val Met Ala Val Leu Leu His Tyr
   145                 150                 155                 160
   Phe Phe Leu Ser Ala Phe Ala Trp Met Leu Val Glu Gly Leu His Leu
                   165                 170                 175
   Tyr Ser Met Val Ile Lys Val Phe Gly Ser Glu Asp Ser Lys His Arg
               180                 185                 190
   Tyr Tyr Tyr Gly Met Gly Trp Gly Phe Pro Leu Leu Ile Cys Ile Ile
           195                 200                 205
   Ser Leu Ser Phe Ala Met Asp Ser Tyr Gly Thr Ser Asn Asn Cys Trp
       210                 215                 220
   Leu Ser Leu Ala Ser Gly Ala Ile Trp Ala Phe Val Ala Pro Ala Leu
   225                 230                 235                 240
   Phe Val Ile Val Val Asn Ile Gly Ile Leu Ile Ala Val Thr Arg Val
                   245                 250                 255
   Ile Ser Gln Ile Ser Ala Asp Asn Tyr Lys Ile His Gly Asp Pro Ser
               260                 265                 270
   Ala Phe Lys Leu Thr Ala Lys Ala Val Ala Val Leu Leu Pro Ile Leu
           275                 280                 285
   Gly Thr Ser Trp Val Phe Gly Val Leu Ala Val Asn Gly Cys Ala Val
       290                 295                 300
   Val Phe Gln Tyr Met Phe Ala Thr Leu Asn Ser Leu Gln Gly Leu Phe
   305                 310                 315                 320
   Ile Phe Leu Phe His Cys Leu Leu Asn Ser Glu Val Arg Ala Ala Phe
                   325                 330                 335
   Lys His Lys Thr Lys Val Trp Ser Leu Thr Ser Ser Ser Ala Arg Thr
               340                 345                 350
   Ser Asn Ala Lys Pro Phe His Ser Asp Leu Met Asn Gly Thr Arg Pro
           355                 360                 365
   Gly Met Ala Ser Thr Lys Leu Ser Pro Trp Asp Lys Ser Ser His Ser
       370                 375                 380
   Ala His Arg Val Asp Leu Ser Ala Val
   385                 390
<210> 18
   <211> 906
   <212> PRT
   <213> Homo sapiens
<400> 18
   Met Glu Lys Leu Leu Arg Leu Cys Cys Trp Tyr Ser Trp Leu Leu Leu
   1               5                   10                  15
   Phe Tyr Tyr Asn Phe Gln Val Arg Gly Val Tyr Ser Arg Ser Gln Asp
               20                  25                  30
   His Pro Gly Phe Gln Val Leu Ala Ser Ala Ser His Tyr Trp Pro Leu
           35                  40                  45
   Glu Asn Val Asp Gly Ile His Glu Leu Gln Asp Thr Thr Gly Ala Ser
       50                  55                  60
   Arg Thr His Lys Leu Thr Val Leu Pro Ser Arg Asn Ala Thr Phe Val
   65                  70                  75                  80
   Tyr Ser Asn Asp Ser Ala Tyr Ser Asn Leu Ser Ala Thr Val Asp Ile
                   85                  90                  95
   Val Glu Gly Lys Val Asn Lys Gly Ile Tyr Leu Lys Glu Glu Lys Gly
               100                 105                 110
   Val Thr Leu Leu Tyr Tyr Gly Arg Tyr Asn Ser Ser Cys Ile Ser Lys
           115                 120                 125
   Pro Glu Gln Cys Gly Pro Glu Gly Val Thr Phe Ser Phe Phe Trp Lys
       130                 135                 140
   Thr Gln Gly Glu Gln Ser Arg Pro Ile Pro Ser Ala Tyr Gly Gly Gln
   145                 150                 155                 160
   Val Ile Ser Asn Gly Phe Lys Val Cys Ser Ser Gly Gly Arg Gly Ser
                   165                 170                 175
   Val Glu Leu Tyr Thr Arg Asp Asn Ser Met Thr Trp Glu Ala Ser Phe
               180                 185                 190
   Ser Pro Pro Gly Pro Tyr Trp Thr His Val Leu Phe Thr Trp Lys Ser
           195                 200                 205
   Lys Glu Gly Leu Lys Val Tyr Val Asn Gly Thr Leu Ser Thr Ser Asp
       210                 215                 220
   Pro Ser Gly Lys Val Ser Arg Asp Tyr Gly Glu Ser Asn Val Asn Leu
   225                 230                 235                 240
   Val Ile Gly Ser Glu Gln Asp Gln Ala Lys Cys Tyr Glu Asn Gly Ala
                   245                 250                 255
   Phe Asp Glu Phe Ile Ile Trp Glu Arg Ala Leu Thr Pro Asp Glu Ile
               260                 265                 270
   Ala Met Tyr Phe Thr Ala Ala Ile Gly Lys His Ala Leu Leu Ser Ser
           275                 280                 285
   Thr Leu Pro Ser Leu Phe Met Thr Ser Thr Ala Ser Pro Val Met Pro
       290                 295                 300
   Thr Asp Ala Tyr His Pro Ile Ile Thr Asn Leu Thr Glu Glu Arg Lys
   305                 310                 315                 320
   Thr Phe Gln Ser Pro Gly Val Ile Leu Ser Tyr Leu Gln Asn Val Ser
                   325                 330                 335
   Leu Ser Leu Pro Ser Lys Ser Leu Ser Glu Gln Thr Ala Leu Asn Leu
               340                 345                 350
   Thr Lys Thr Phe Leu Lys Ala Val Gly Glu Ile Leu Leu Leu Pro Gly
           355                 360                 365
   Trp Ile Ala Leu Ser Glu Asp Ser Ala Val Val Leu Ser Leu Ile Asp
       370                 375                 380
   Thr Ile Asp Thr Val Met Gly His Val Ser Ser Asn Leu His Gly Ser
   385                 390                 395                 400
   Thr Pro Gln Val Thr Val Glu Gly Ser Ser Ala Met Ala Glu Phe Ser
                   405                 410                 415
   Val Ala Lys Ile Leu Pro Lys Thr Val Asn Ser Ser His Tyr Arg Phe
               420                 425                 430
   Pro Ala His Gly Gln Ser Phe Ile Gln Ile Pro His Glu Ala Phe His
           435                 440                 445
   Arg His Ala Trp Ser Thr Val Val Gly Leu Leu Tyr His Ser Met His
       450                 455                 460
   Tyr Tyr Leu Asn Asn Ile Trp Pro Ala His Thr Lys Ile Ala Glu Ala
   465                 470                 475                 480
   Met His His Gln Asp Cys Leu Leu Phe Ala Thr Ser His Leu Ile Ser
   485 490 495
   Leu Glu Val Ser Pro Pro Pro Thr Leu Ser Gln Asn Leu Ser Gly Ser
   500 505 510
   Pro Leu Ile Thr Val His Leu Lys His Arg Leu Thr Arg Lys Gln His
   515 520 525
   Ser Glu Ala Thr Asn Ser Ser Asn Arg Val Phe Val Tyr Cys Ala Phe
   530 535 540
   Leu Asp Phe Ser Ser Gly Glu Gly Val Trp Ser Asn His Gly Cys Ala
   545 550 555 560
   Leu Thr Arg Gly Asn Leu Thr Tyr Ser Val Cys Arg Cys Thr His Leu
   565 570 575
   Thr Asn Phe Ala Ile Leu Met Gln Val Val Pro Leu Glu Leu Ala Arg
   580 585 590
   Gly His Gln Val Ala Leu Ser Ser Ile Ser Tyr Val Gly Cys Ser Leu
   595 600 605
   Ser Val Leu Cys Leu Val Ala Thr Leu Val Thr Phe Ala Val Leu Ser
   610 615 620
   Ser Val Ser Thr Ile Arg Asn Gln Arg Tyr His Ile His Ala Asn Leu
   625 630 635 640
   Ser Phe Ala Val Leu Val Ala Gln Val Leu Leu Leu Ile Ser Phe Arg
   645 650 655
   Leu Glu Pro Gly Thr Thr Pro Cys Gln Val Met Ala Val Leu Leu His
   660 665 670
   Tyr Phe Phe Leu Ser Ala Phe Ala Trp Met Leu Val Glu Gly Leu His
   675 680 685
   Leu Tyr Ser Met Val Ile Lys Val Phe Gly Ser Glu Asp Ser Lys His
   690 695 700
   Arg Tyr Tyr Tyr Gly Met Gly Trp Gly Phe Pro Leu Leu Ile Cys Ile
   705 710 715 720
   Ile Ser Leu Ser Phe Ala Met Asp Ser Tyr Gly Thr Ser Asn Asn Cys
   725 730 735
   Trp Leu Ser Leu Ala Ser Gly Ala Ile Trp Ala Phe Val Ala Pro Ala
               740                 745                 750
   Leu Phe Val Ile Val Val Asn Ile Gly Ile Leu Ile Ala Val Thr Arg
           755                 760                 765
   Val Ile Ser Gln Ile Ser Ala Asp Asn Tyr Lys Ile His Gly Asp Pro
       770                 775                 780
   Ser Ala Phe Lys Leu Thr Ala Lys Ala Val Ala Val Leu Leu Pro Ile
   785                 790                 795                 800
   Leu Gly Thr Ser Trp Val Phe Gly Val Leu Ala Val Asn Gly Cys Ala
                   805                 810                 815
   Val Val Phe Gln Tyr Met Phe Ala Thr Leu Asn Ser Leu Gln Gly Leu
               820                 825                 830
   Phe Ile Phe Leu Phe His Cys Leu Leu Asn Ser Glu Val Arg Ala Ala
           835                 840                 845
   Phe Lys His Lys Thr Lys Val Trp Ser Leu Thr Ser Ser Ser Ala Arg
       850                 855                 860
   Thr Ser Asn Ala Lys Pro Phe His Ser Asp Leu Met Asn Gly Thr Arg
   865                 870                 875                 880
   Pro Gly Met Ala Ser Thr Lys Leu Ser Pro Trp Asp Lys Ser Ser His
                   885                 890                 895
   Ser Ala His Arg Val Asp Leu Ser Ala Val
               900                 905
<210> 19
   <211> 27
   <212> PRT
   <213> Homo sapiens
<400> 19
   Gln Thr Ala Leu Asn Leu Thr Lys Thr Phe Leu Lys Ala Val Gly Glu
   1               5                   10                  15
   Ile Leu Leu Leu Pro Gly Trp Ile Ala Leu Ser
               20                  25
<210> 20
   <211> 27
   <212> PRT
   <213> Homo sapiens
<400> 20
   Met Glu Lys Gly Thr Glu Leu Leu Val Ser Pro Ser Gln Ser Gly Pro
   1               5                   10                  15
   Gly Gly Asp Gln Pro Leu Leu Val Lys His Arg
               20                  25
<210> 21
   <211> 10
   <212> PRT
   <213> Homo sapiens
<400> 21
   Leu Ile Thr Val His Leu Lys His Arg Leu
   1               5                   10
<210> 22
   <211> 10
   <212> PRT
   <213> Homo sapiens
<400> 22
   Met His Arg Val Cys Phe Leu Ser Phe Gln
   1               5                   10
<210> 23
   <211> 33
   <212> PRT
   <213> Homo sapiens
<400> 23
   Gly Ala Ser Arg Thr His Lys Leu Thr Val Leu Pro Ser Arg Asn Ala
   1               5                   10                  15
   Thr Phe Val Tyr Ser Asn Asp Ser Ala Tyr Ser Asn Leu Ser Ala Thr
               20                  25                  30
   Val
<210> 24
   <211> 21
   <212> DNA
   <213> Artificial Sequence
<220>
   <223> Description of Artificial Sequence: Synthetic oligonucleotide
<400> 24
   gccgctcaat ttatgaatat t 21

## Description

### CROSS-REFERENCE TO RELATED APPLICATIONS

This application claims priority to U.S. Provisional Application No. 62/154,173, filed on April 29, 2015.

### STATEMENT AS TO FEDERALLY SPONSORED RESEARCH

The United States Government has certain rights to this invention by virtue of funding reserved from Grant No. 1R21NS087241-01 from the National Institutes of Health.

### SEQUENCE LISTING

The instant application contains a Sequence Listing which has been submitted electronically in ASCII format. Said ASCII copy, created on April 28, 2016, is named 243735.000148_SL.txt and is 42,231 bytes in size.

### FIELD OF THE INVENTION

The invention is directed to methods and compositions for treating cancers and to methods for inhibiting growth, self-renewal, or tumorigenicity of cancer cells by inhibiting GPR133 expression and/or function in cancer cells, including CD133-expressing glioma stem cells (GSCs). The invention is also directed to a method for determining if a subject diagnosed with a cancer is at an increased risk for cancer progression and/or reduced survival by determining an expression level of GPR133 in cancer cells.

### BACKGROUND OF THE INVENTION

High-grade gliomas, including glioblastoma (GBM), gliosarcoma, anaplastic astrocytoma, and anaplastic oligodendroglioma are incurable brain malignancies with a poor median survival (14-16 months for GBM patients) and limited therapeutic options (Stupp et al., 2005, NEJM 352:987-96).

Recent evidence suggests a cellular hierarchy within GBM, dominated by stem-like cells (GBM stem cells or GSCs) giving rise to diverse tumor lineages and supporting tumor growth (Singh et al., 2004, Nature 432:396-401). GBM is characterized by remarkable microenvironmental heterogeneity, highlighted by the coexistence of hypervascular areas and avascular hypoxic zones in tumors. Within any given GBM tumor, there exist at least two different subtypes of GSCs whose molecular, cellular and metabolic profiles match such distinct microenvironments. One of these GSC subtypes (referred to as Notch+) requires the Notch signaling pathway for self-renewal and tumorigenicity. Notch+ GSCs are metabolically dependent on oxidative phosphorylation and have the unique ability to differentiate to tumor-derived pericytes, thereby giving rise to large tumor vessels that generate a normoxic microenvironment and support their aerobic metabolism. A second GSC subtype (referred to CD133+), which is marked by cell surface expression of the CD133 glycoprotein and does not require the Notch pathway for self-renewal or tumorigenesis, has a restricted differentiation program and cannot generate pericytes. This restriction translates to poor tumor vascularity and hypoxic microenvironmental conditions.

GPR133 belongs to the adhesion class of GPCRs, but very little is known about its function. Polymorphisms in GPR133 have been linked to regulation of body height and weight, as well as RR and QT interval duration in cardiac electrophysiology. Studies in HEK293 and COS7 cells indicated that GPR133 is coupled to the Gₛ protein, which activates adenylate cyclase, thereby leading to elevation in intracellular cAMP. The N-terminal ectodomain of GPR133 was recently shown to contain a tethered agonist peptide (*Stachel* sequence), which is exposed and activates the receptor upon N-terminal proteolytic cleavage.

Current standard-of-care therapy in high-grade gliomas, including glioblastoma (GBM), gliosarcoma, anaplastic astrocytoma, and anaplastic oligodendroglioma, involves surgery and concurrent chemoradiotherapy with the alkylating agent temozolamide. However, it was found that GSCs utilize both cell-autonomous and microenvironment-regulated mechanisms to resist chemoradiotherapy and support tumor recurrence (Bao et al., 2006, Nature 444:756-60; Chen et al., 2012, Nature 488:522-6). It is, therefore, imperative, to develop novel therapies that target cancer stem cells in high-grade gliomas.

Adenocarcinomas are cancers that occur in glandular or glandular-like tissue. Most types of breast cancers are adenocarcinomas, including invasive ductal carcinoma, ductal carcinoma in situ, and invasive lobular carcinoma. Similarly, many types of colorectal cancers are adenocarcinomas, due to the high number of glands found within the colon. Lung squamous cell carcinoma (also called squamous cell carcinoma of the lung or SCC) is a type of non-small-cell lung carcinoma that most often arises centrally in larger bronchi and often metastasizes to locoregional lymph nodes (particularly the hilar nodes).

### SUMMARY OF THE INVENTION

There is a great need in the art to develop novel therapies for treating cancers such as high-grade gliomas, including glioblastoma (GBM), gliosarcoma, anaplastic astrocytoma, and anaplastic oligodendroglioma. The invention fulfills such need by providing a method for inhibiting growth, self-renewal, or tumorigenicity of cancer cells by inhibiting GPR133 expression and/or function in such cancer cells.

The invention provides the following embodiments 1-14:
1. A GPR133 inhibitor for use in inhibiting growth, self-renewal, or tumorigenicity of a glioma cancer cell by exposing said cancer cell to the GPR133 inhibitor.
2. The GPR133 inhibitor for use according to item 1, wherein said glioma cell is a high-grade glioma cell.
3. The GPR133 inhibitor for use according to item 1, wherein said high-grade glioma cell is a glioblastoma cell.
4. The GPR133 inhibitor for use according to item 1, wherein said glioma cancer cell is a CD133-expressing (CD133+) cell.
5. The GPR133 inhibitor for use according to any one of items 1-4, wherein said glioma cancer cell is a glioma stem cell.
6. The GPR133 inhibitor for use according to any one of items 1-5, wherein said GPR133 inhibitor is selected from the group consisting of a nucleic acid-based molecule, an antibody, a peptide, and a small molecule.
7. The GPR133 inhibitor for use according to item 6, wherein said GPR133 inhibitor is selected from the group consisting of interfering RNA (RNAi) molecules, dsRNA, RNA polymerase III transcribed DNAs, and antisense nucleic acids.
8. The GPR133 inhibitor for use according to item 7, wherein said RNAi molecule is shRNA or siRNA.
9. The GPR133 inhibitor for use according to item 8, wherein said shRNA comprises a nucleic acid sequence selected from the group consisting of GGGATCATAGATGTGAATTAA (SEQ ID NO: 5), GGAGTCACGCTTCTCTATTAC (SEQ ID NO: 6), and CCTGCAGGGACTGTTCATATT (SEQ ID NO: 7).
10. The GPR133 inhibitor for use according to any one of items 1-5, wherein said GPR133 inhibitor is selected from the group consisting of methods which involve the use of Clustered Regularly Interspaced Short Palindromic Repeats (CRISPR)/Cas9 gene systems, methods which involve the use of zinc finger nucleases (ZFNs), and methods which involve the use of transcription activator-like effector nucleases (TALENs).
11. The GPR133 inhibitor for use according to item 6, wherein said GPR133 inhibitor is an antibody.
12. The GPR133 inhibitor for use according to item 11, wherein said antibody recognizes an amino acid sequence selected from the group consisting of: AVVLSLIDTIDVMGHVSSNLHGSTPQVTVEGSSAMAEFSVAKILPKTVNSSHYRFPAHG QSFIQIPHEAFHRHAWSTVVGLLYHSMHYYLNNIWPAHTKIAEAMHHQDC (SEQ ID NO: 9), TRKQHSEATNSSNRC (SEQ ID NO: 10), CSSGEGVWSNHG (SEQ ID NO: 11), CSSARTSNAKPFHSD (SEQ ID NO: 12), and LMNGTRPGMASTKLSC (SEQ ID NO: 13).
13. The GPR133 inhibitor for use according to item 11, wherein said antibody selectively recognizes GPR133 isoform 1 (Uniprot Q6QNK2.1; SEQ ID NO: 15).
14. An in vitro method for determining whether a subject diagnosed with a glioma is at an increased risk for progression of said glioma and/or reduced survival comprising:
   (a) determining an expression level of GPR133 in glioma cancer cells of the subject,
   (b) comparing the expression level of GPR133 determined in step (a) to GPR133 expression level in corresponding normal cells of the same tissue origin or to a database's mean value of GPR133 mRNA expression in the same type of cancer, and
   (c) determining that the subject is at an increased risk for cancer progression and/or reduced survival if the expression level of GPR133 in cancer cells of the subject is higher than in the corresponding normal cells or the database's mean value of GPR133 mRNA expression in the same type of cancer.

In one aspect, the disclosure relates to a method for inhibiting growth, self-renewal, or tumorigenicity of a cancer cell, said method comprising exposing said cancer cell to a GPR133 inhibitor that is capable of inhibiting expression or function of GPR133 in said cancer cell. In one embodiment, the cancer cell is a glioma cell (e.g., a high-grade glioma cell such as, e.g., a glioblastoma cell). In another embodiment, the cancer cell is an adenocarcinoma cell (e.g., a breast adenocarcinoma cell or a colorectal adenocarcinoma cell) or a lung squamous cancer cell. In one embodiment, the cancer cell has elevated expression of GPR133 as compared to a corresponding normal cell of the same tissue origin.

In another aspect, the disclosure relates to a method of treating a cancer in a subject in need thereof comprising administering to said subject a therapeutically effective amount of GPR133 inhibitor, wherein said inhibitor inhibits expression or function of GPR133 in cancer cells of said subject. In one embodiment, the cancer is a glioma (e.g., a high-grade glioma such as, e.g., glioblastoma, gliosarcoma, anaplastic astrocytoma, or anaplastic oligodendroglioma). In one specific embodiment, the cancer is glioblastoma. In another embodiment, the cancer is an adenocarcinoma (e.g., a breast adenocarcinoma or a colorectal adenocarcinoma) or a lung squamous cancer. In one embodiment, the cancer is characterized by elevated expression of GPR133 mRNA in cancer cells of the subject as compared to a database's (e.g., NIH The Cancer Genome Atlas (TCGA) database) mean value of GPR133 mRNA expression in the same type of cancer.

In one embodiment of any of the above methods, the cancer cell is CD133-expressing (CD133+) cell. In one specific embodiment, said CD133+ cancer cell is located within a hypoxic area of a tumor in a subject. In another specific embodiment, said CD133+ cancer cell is located within a non-hypoxic area of a tumor in a subject. In one embodiment of any of the above methods, said cancer cell is a glioma stem cell.

Non-limiting examples of GPR133 inhibitors useful in any of the above methods include nucleic acid-based molecules, antibodies, peptides, and small molecules. In one embodiment, the GPR133 inhibitor is selected from the group consisting of interfering RNA (RNAi) molecules (e.g., shRNA or siRNA), dsRNA, RNA polymerase III transcribed DNAs, and antisense nucleic acids. In one specific embodiment, the GPR133 inhibitor is shRNA which comprises a nucleic acid sequence selected from the group consisting of GGGATCATAGATGTGAATTAA (SEQ ID NO: 5), GGAGTCACGCTTCTCTATTAC (SEQ ID NO: 6), and CCTGCAGGGACTGTTCATATT (SEQ ID NO: 7). In another embodiment, the GPR133 inhibitor is selected from the group consisting of methods which involve the use of Clustered Regularly Interspaced Short Palindromic Repeats (CRISPR)/Cas9 gene systems, methods which involve the use of zinc finger nucleases (ZFNs), and methods which involve the use of transcription activator-like effector nucleases (TALENs). In a further embodiment, the GPR133 inhibitor is an antibody. In one specific embodiment, the antibody recognizes an epitope within the extracellular domain of GPR133. In one specific embodiment, the antibody recognizes an amino acid sequence selected from the group consisting of: VNKGIYLKEEKGVTLLYYGRYNSSCISKPEQCGPEGVTFSFFWKTQGEQSRPIPSAYGGQ VISNGFKVCSSGGRGSVELYTRDNSMTWEASFSPPGPYWTHVLFTWKSKEGLKVYVNG TLVIGSEQDQAK (SEQ ID NO: 8), AVVLSLIDTIDTVMGHVSSNLHGSTPQVTVEGSSAM AEFSVAKILPKTVNSSHYRFPAHGQSFIQIPHEAFHRHAWSTVVGLLYHSMHYYLNNIW PAHTKIAEAMHHQDC (SEQ ID NO: 9), TRKQHSEATNSSNRC (SEQ ID NO: 10), CSSGEGVWSNHG (SEQ ID NO: 11), CSSARTSNAKPFHSD (SEQ ID NO: 12), and LMNGTRPGMASTKLSC (SEQ ID NO: 13). In one specific embodiment, the antibody selectively recognizes one GPR133 isoform (e.g., isoform 1 Uniprot Q6QNK2.1; SEQ ID NO: 15). In one embodiment, the antibody is a bispecific antibody. In one specific embodiment, said bispecific antibody recognizes GPR133 and CD3. In another specific embodiment, said bispecific antibody recognizes GPR133 and an endogenous blood-brain barrier (BBB) receptor. In a further specific embodiment, said bispecific antibody recognizes (i) GPR133 and (ii) an adenocarcinoma-specific receptor or a lung squamous cell carcinoma-specific receptor.

In one embodiment of any of the methods of the invention, the GPR133 inhibitor is a cAMP regulator (e.g., forskolin, IBMX, phosphodiesterase inhibitors, cAMP analogs [e.g., 8-Bromo-cAMP, cAMPS-Sp and 8-pCPT-2-O-Me-cAMP-AM], or the protein kinase A inhibitor H-89). In another embodiment of any of the methods of the invention, the GPR133 inhibitor is a regulator of oxygen tension or a regulatory molecule modulated by hypoxia (e.g., inhibitors of Hif1α such as, e.g., chetomin, cryptotanshinone, FM19G11, or PX12).

In one embodiment of any of the above methods, GPR133 inhibitor is administered to said subject by a route selected from the group consisting of systemic delivery, oral delivery, direct injection within the tumor, intra-arterial delivery within a vascular territory of the tumor, and viral vector-mediated delivery (e.g., using a lentiviral vector or a retroviral replicating vector).

In one embodiment of any of the above methods, the treatment method is used in combination with one or more additional treatments selected from the group consisting of radiation therapy, surgery, chemotherapy, immune cell therapy, cancer vaccine, oncolytic viruses, and alternating electric field therapy.

In a further aspect, the disclosure relates to a method of identifying a GPR133 inhibitor for treatment of a cancer, comprising contacting a candidate agent with a CD133-expressing (CD133+) cancer cell and determining expression level or a function of GPR133 in said cancer cell, wherein an inhibition of expression level or function of GPR133, as compared with said level or function prior to the agent exposure, indicates that said agent is a GPR133 inhibitor. In one embodiment, the cancer is selected from the group consisting of an adenocarcinoma (e.g., a breast adenocarcinoma or a colorectal adenocarcinoma), a lung squamous cell cancer, and a glioma (e.g., a high-grade glioma such as, e.g., glioblastoma, gliosarcoma, anaplastic astrocytoma, or anaplastic oligodendroglioma). In one specific embodiment, said cancer is glioblastoma and said CD133+ cancer cell is a glioblastoma cell.

In another aspect, the disclosure relates to a method for determining whether a subject diagnosed with a cancer (e.g., a glioma [e.g., glioblastoma, gliosarcoma, anaplastic astrocytoma, or anaplastic oligodendroglioma], an adenocarcinoma [e.g., a breast adenocarcinoma or a colorectal adenocarcinoma], or a lung squamous cell carcinoma) is at an increased risk for progression of said cancer and/or reduced survival comprising:
(a) determining an expression level of GPR133 in cancer cells of the subject,
(b) comparing the expression level of GPR133 determined in step (a) to GPR133 expression level in corresponding normal cells of the same tissue origin (e.g., expression level in normal cells of the same subject or a database's level for normal tissue [e.g., NIH The Cancer Genome Atlas (TCGA) database level]), and
(c) determining that the subject is at an increased risk for cancer progression and/or reduced survival if the expression level of GPR133 in cancer cells of the subject is higher than in the corresponding normal cells. In one specific embodiment, the cancer is glioblastoma.

In another embodiment, the disclosure relates to a method for determining whether a subject diagnosed with a cancer (e.g., a glioma [e.g., glioblastoma, gliosarcoma, anaplastic astrocytoma, or anaplastic oligodendroglioma], an adenocarcinoma [e.g., a breast adenocarcinoma or a colorectal adenocarcinoma], or a lung squamous cell carcinoma) is at an increased risk for progression of said cancer and/or reduced survival comprising:
(a) determining an expression level of GPR133 in cancer cells of the subject,
(b) comparing the expression level of GPR133 determined in step (a) to a database's (e.g., NIH The Cancer Genome Atlas (TCGA) database) mean value of GPR133 mRNA expression in the same type of cancer, and
(c) determining that the subject is at an increased risk for cancer progression and/or reduced survival if the expression level of GPR133 in cancer cells of the subject is higher than the database's mean value of GPR133 mRNA expression in the same type of cancer.

In one embodiment of any of the above methods, the subject is human.

These and other aspects of the present invention will be apparent to those of ordinary skill in the art in the following description, claims and drawings.

### BRIEF DESCRIPTION OF THE DRAWINGS

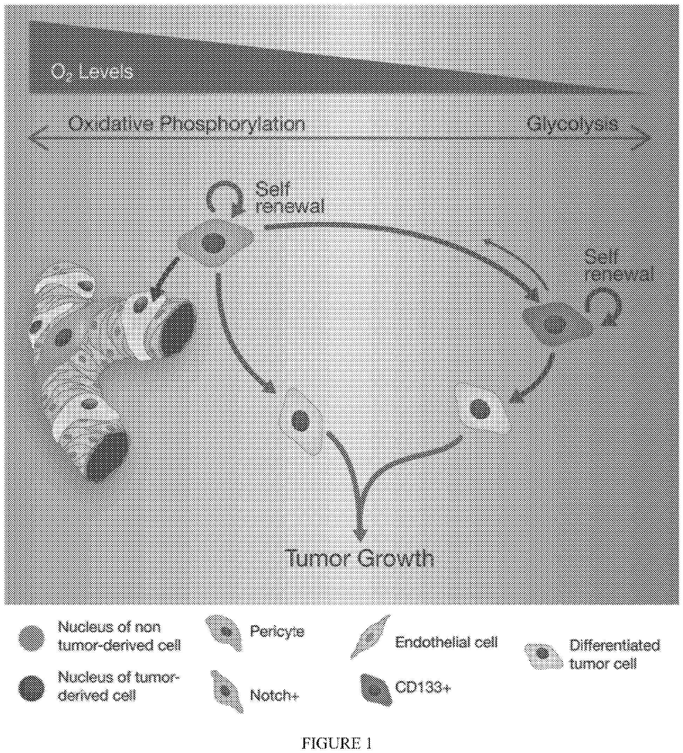

**Figure 1****.** Cellular hierarchy in GBM. In the proposed model, Notch+ GSCs give rise to all tumor lineages, including CD133+ GSCs and pericytes. The GSC subtypes manifest distinct metabolic preferences. Importantly, plasticity between Notch+ and CD133+ GSCs is tightly regulated, so that the CD133+ to Notch+ transition occurs only infrequently.

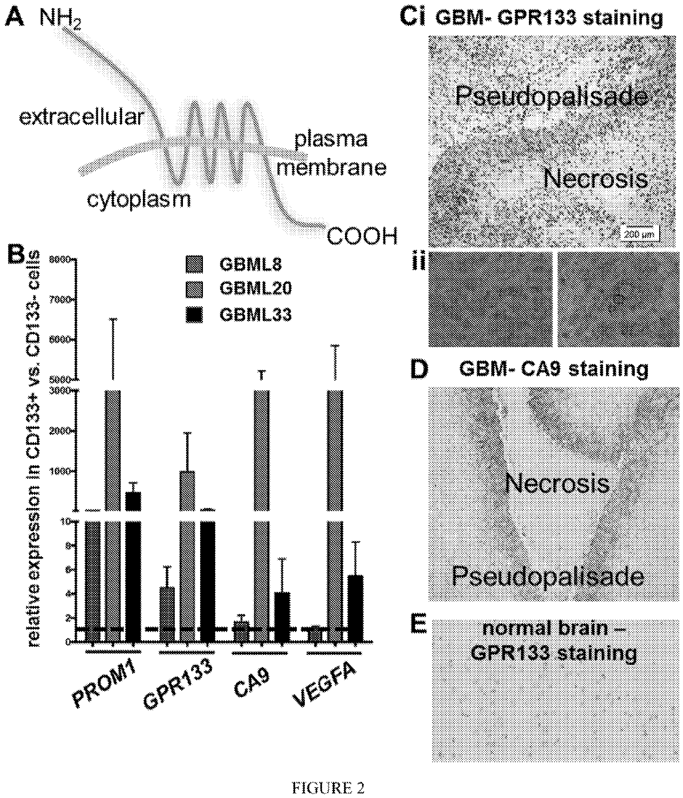

**Figures 2A-2E****.** GPR133 expression profile in GBM. **Figure 2A****.** Membrane topology of GPR133. **Figure 2B****.** *GPR133* transcript is enriched in CD133+ GSCs along with *PROM1* (*CD133*)*, CA9* and *VEGFA.* **Figure 2C****.** GPR133 immunostaining in human tumor tissue shows that GPR133 is expressed on the cell membrane of tumor cells localizing to hypoxic pseudopalisades. **Figure 2D****.** Pseudopalisades also express CA9 (Carbonic Anhydrase 9), a known marker of hypoxia. **Figure 2E****.** There is no GPR133 expression in normal brain.

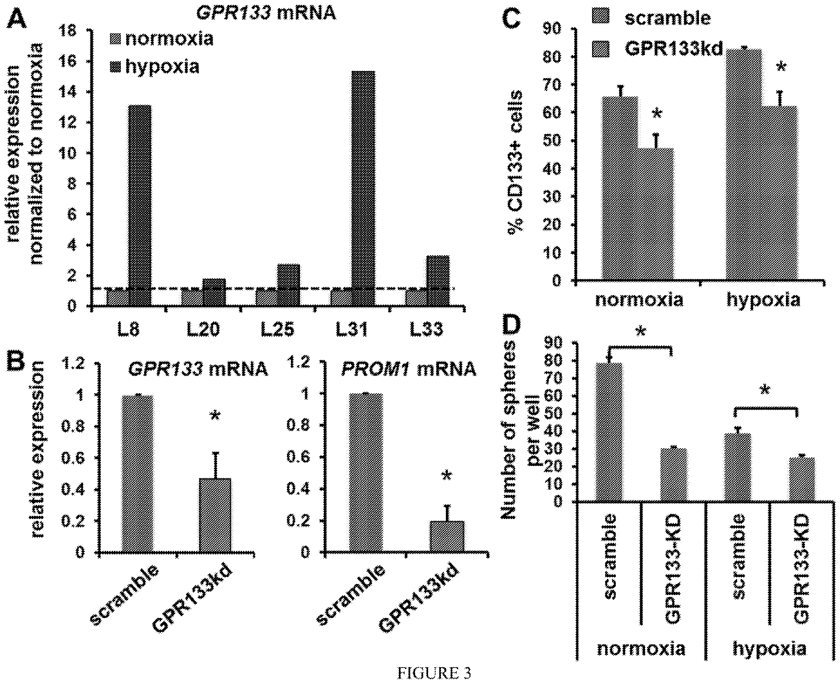

**Figures 3A-3D****.** GPR133 is required for tumor growth in hypoxia. **Figure 3A****.** *GPR133* is transcriptionally upregulated by hypoxia. **Figures 3B and 3C****.** GPR133 knockdown reduces *PROM1* (*CD133*) transcript (**Figure 3B**) and the abundance of CD133+ cells (**Figure 3C**) *in vitro.* **Figure 3D****.** GPR133 knockdown reduces tumor sphere formation in normoxia and hypoxia.

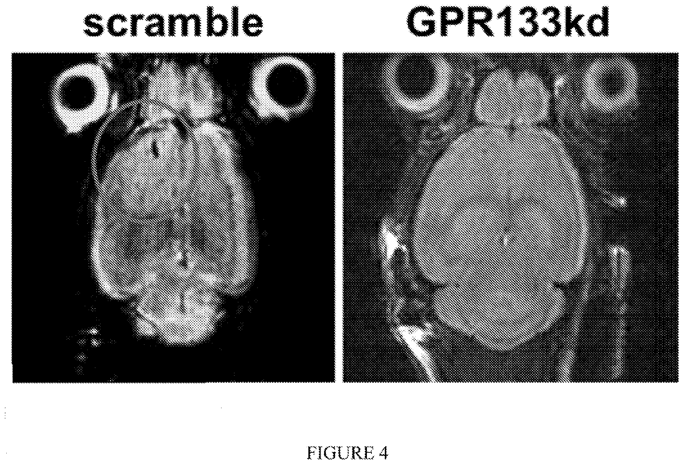

**Figure 4****.** GPR133 is required for *in vivo* tumorigenesis. GPR133 knockdown results in significantly smaller tumor xenografts.

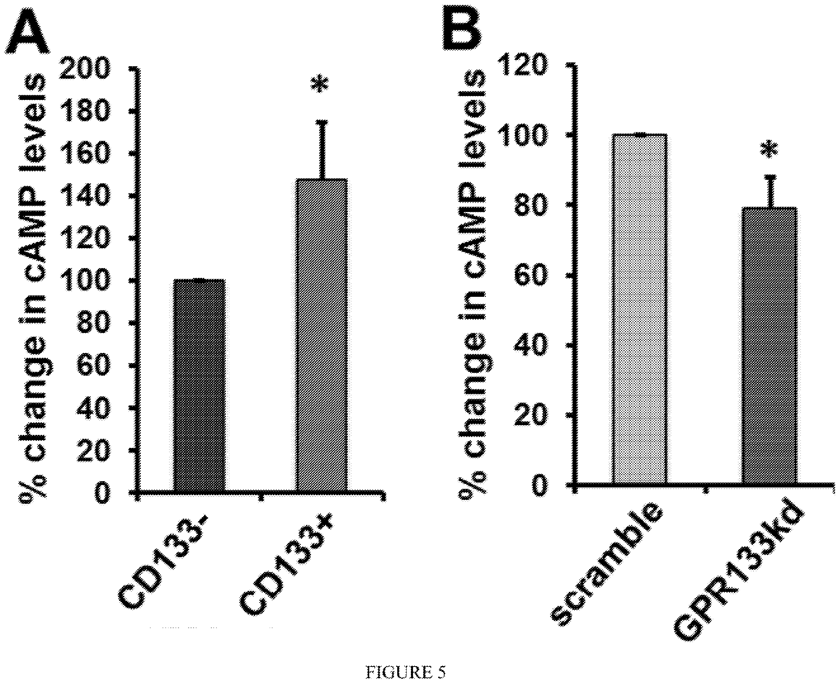

**Figures 5A-5B****.** GPR133 signaling is mediated by cAMP. **Figure 5A****.** Intracellular cAMP is significantly higher in CD133+ cells. **Figure 5B****.** GPR133 knockdown results in a significant reduction in cAMP levels.

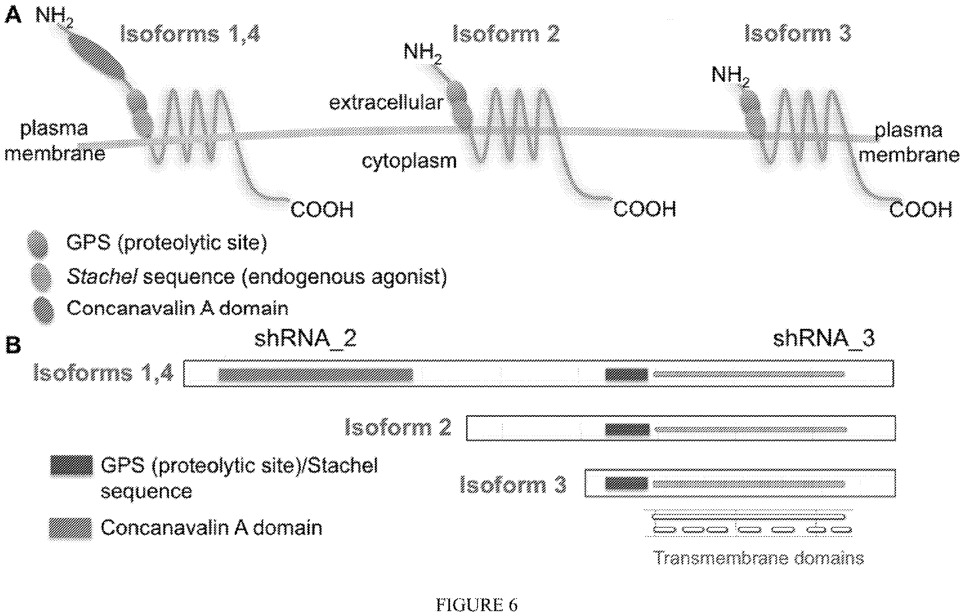

**Figures 6A-6B****.** Splice variants and protein structure of GPR133. **Figure 6A****.** GPR133 has 4 main isoforms (splice variants) (N terminal ectodomains of the four isoforms are provided in SEQ ID NOS: 1-4 while the full length isoforms are provided in SEQ ID NOS: 15-18), whose membrane topology are displayed. The isoforms differ in the N-terminal ectodomain. All isoforms contains a GPS site for proteolytic cleavage of the ectodomain, and a *Stachel* sequence, which acts as an endogenous agonist for the receptor. Only isoforms 1 and 4 contain a concanavalin A domain in the N-terminal ectodomain. **Figure 6B****.** The target sites for shRNA_2 and shRNA_3 are superimposed on the protein structure of the 4 isoforms. shRNA_3 targets all isoforms, whereas shRNA_2 targets isoforms 1 and 4 only.

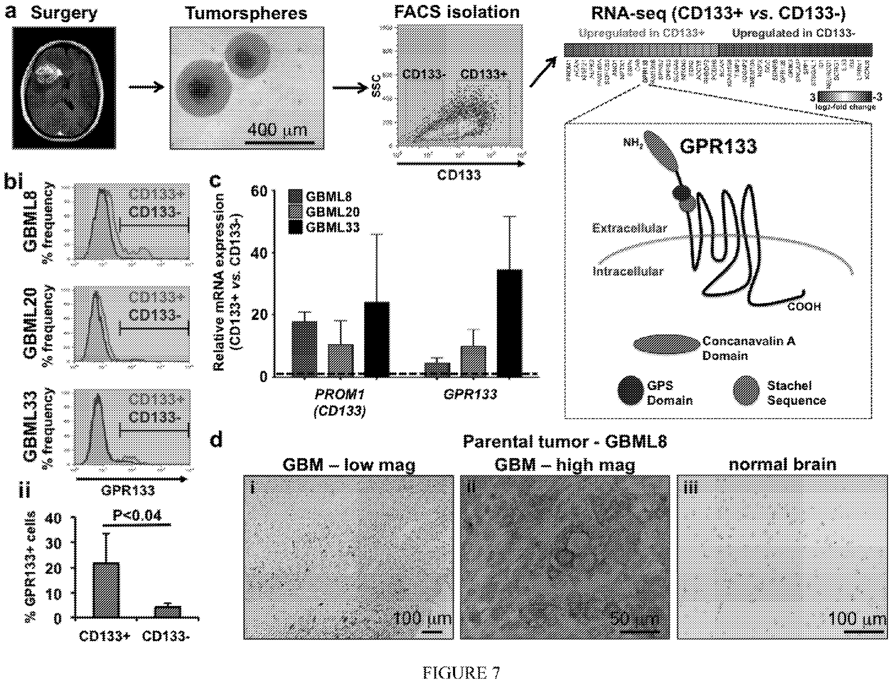

**Figures 7A-7D****.** GPR133 is expressed in the CD133+ cell population of human GBM. **Figure 7A****.** The experimental model consists of harvesting human GBM tissue during surgery and growing primary tumorsphere cultures. RNA-seq analysis of FACS-sorted CD133+ and CD133-cells from GBML8 revealed 314 differentially expressed genes. GPR133 was among the top 20 genes overexpressed in CD133+ cells. GPR133's membrane topology and important domains within the extracellular N-terminus are shown. **Figure 7B****. i**. Flow cytometry using antibodies against GPR133 and CD133 showed enrichment of GPR133 within the CD133+ cell population in 3 primary cultures, **ii**. Cumulative statistics showing the percentage of GPR133+ cells within the CD133+ and CD133- populations in 3 cultures. **Figure 7C**. Relative *PROM1* (*CD133*) and *GPR133* mRNA expression in CD133+ vs. CD133- populations in 3 primary cultures. **Figure 7D****.** Immunohistochemistry for GPR133 expression in GBML8's parental tumor and normal brain tissue.

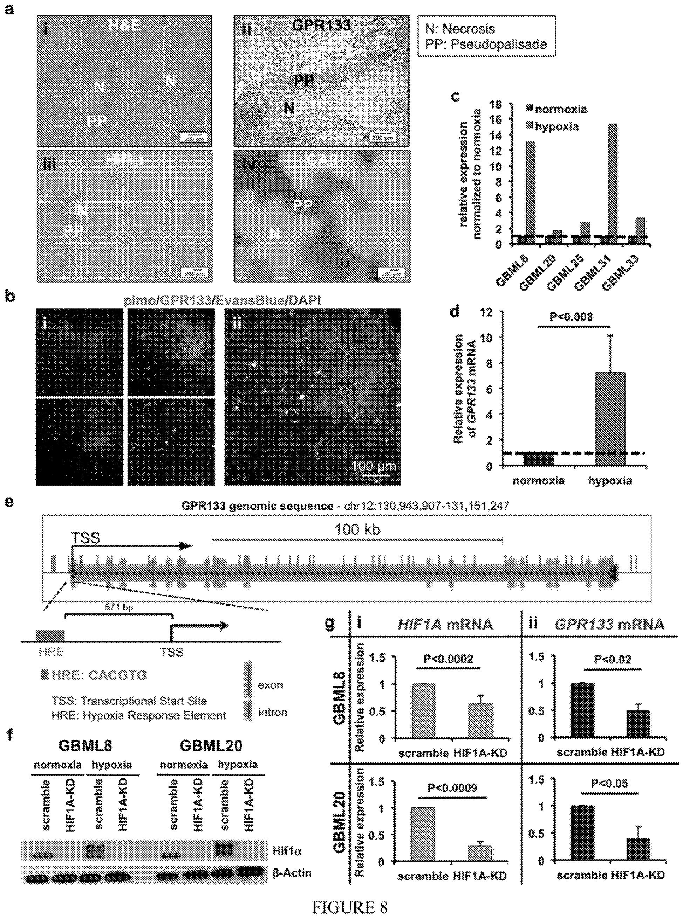

**Figures 8A-8G****.** GPR133 expression is upregulated by hypoxia. **Figure 8A****.** Immunohistochemistry of human GBM biospecimens reveals selective expression of GPR133 in pseudopalisading necrosis (PPN), along with hypoxic markers Hif1α and CA9. **Figure 8B****.** GPR133 immunoreactivity colocalizes with pimonidazole in tumor xenografts in the mouse brain. **Figure 8C****.** Hypoxia upregulates *GPR133* mRNA in 5 primary cultures. **Figure 8D****.** Cumulative upregulation of *GPR133* mRNA by hypoxia in 5 cultures. **Figure 8E****.** Analysis of the GPR133 genomic locus reveals numerous hypoxia response elements (HRE) motifs, including immediately upstream of the transcriptional start site (TSS). **Figure 8F****.** Western blot shows effects of Hif1α knockdown (HIF1A-KD) on Hif1α protein levels in 2 primary cultures. β-actin was used as loading control. **Figure 8G**. Hif1α knockdown downregulates *HIF1A* (**i**) and *GPR133* (**ii**) mRNA in 2 primary cultures.

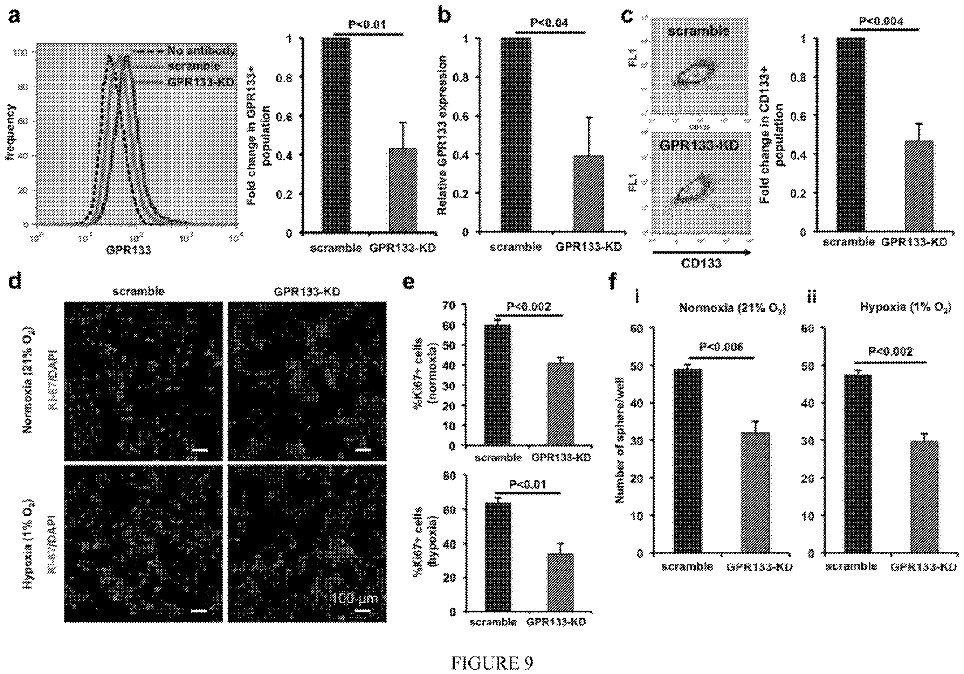

**Figures 9A-9F****.** *In vitro* effects of GPR133 knockdown in GBML20. **Figure 9A****.** GPR133 knockdown (GPR133-KD) reduces the number of GPR133+ cells, as shown by flow cytometry histograms. **Figure 9B****.** GPR133 knockdown decreases *GPR133* mRNA levels. **Figure 9C****.** Flow cytometry indicates reduction in the abundance of CD133+ cells after GPR133 knockdown. **Figures 9D****,E.** Knockdown of GPR133 reduces the percentage of Ki-67+ cells in both normoxia and hypoxia. **Figure 9F****.** GPR133 knockdown impairs tumorsphere formation in normoxia and hypoxia.

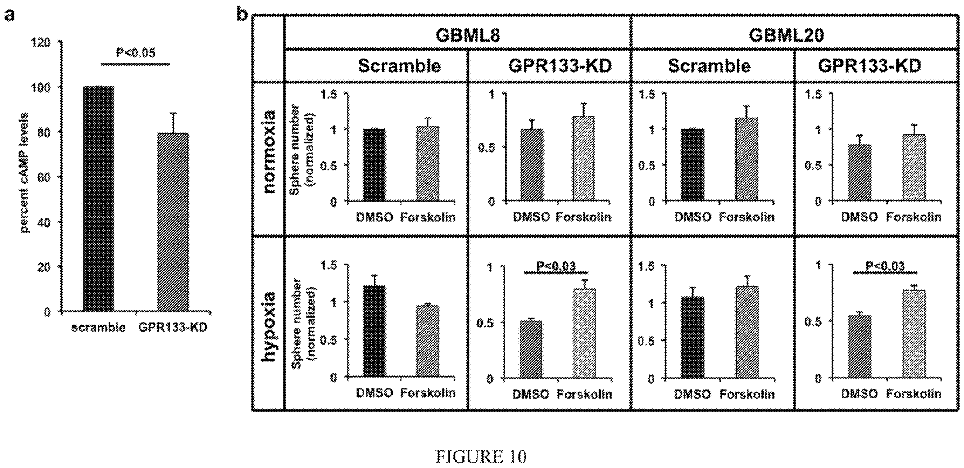

**Figures 10A-10B**. Further confirmation that GPR133 signaling is mediated by cAMP. **Figure 10A****.** GPR133 knockdown (GPR133-KD) decreases cAMP levels. **Figure 10B**. The GPR133 knockdown-induced impairment of tumorsphere formation in hypoxia is rescued by forskolin.

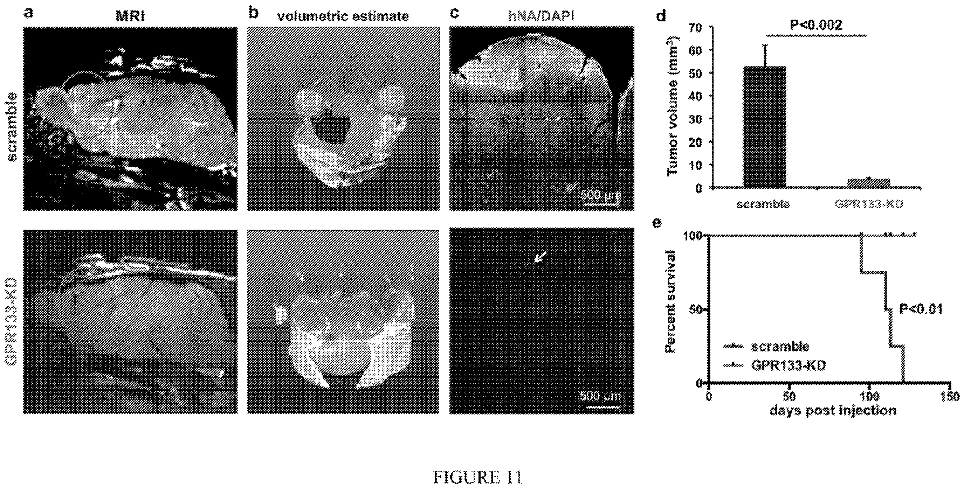

**Figures 11A-11E**. GPR133 knockdown prevents tumor formation and death *in vivo.* **Figures 11A****,B**. Representative MRI images (**A**) and tumor volumetric estimates (**B**) show dramatic reduction in tumor xenograft size in mice implanted with GBM cells bearing GPR133 shRNA knockdown constructs (GPR133-KD). **Figure 11C****.** Histology of tumor xenografts. Tumor xenografts were identified by human Nuclear Antigen immunoreactivity (hNA). The arrow indicates scattered tumor cells in the control condition, in the absence of formed tumor. **Figure 11D**. Cumulative statistics for tumor size obtained from MRI-based volumetric estimates. **Figure 11E**. Kaplan-Meier survival curves of the GPR133-KD and control groups.

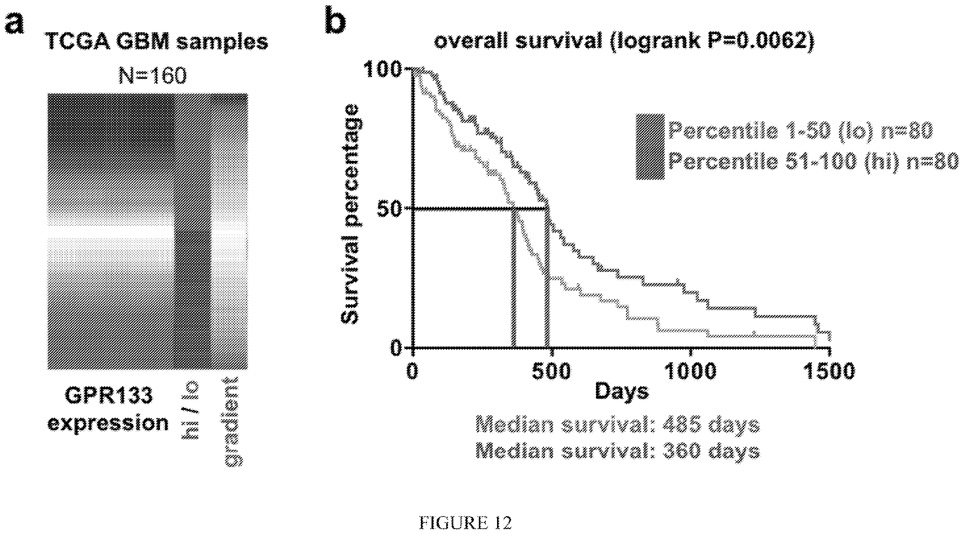

**Figures 12A-B**. Increased *GPR133* mRNA expression correlates with poor survival in patients. **Figure 12A****.** TCGA data from 160 patients with GBM were analyzed for *GPR133* expression. We studied outcomes in two cohorts (GPR133 hi and GPR133 lo) based on ranked *GPR133* mRNA levels. **Figure 12B****.** Kaplan-Meier curves of the two patient cohorts indicate an inverse relation between *GPR133* expression and survival.

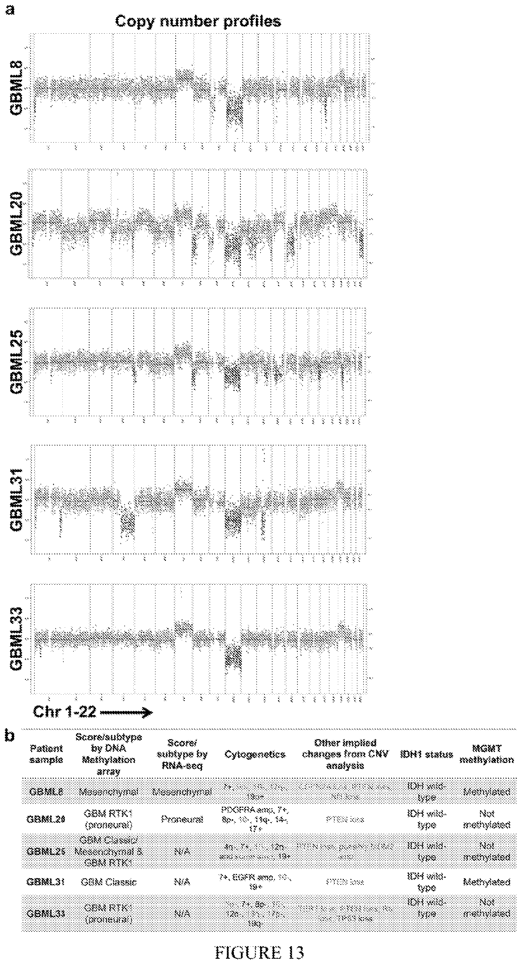

**Figures 13A-13B****.** DNA methylation-based classification of parental tumors. **Figure 13A****.** The parental tumors for the 5 primary cultures used in this study were analyzed with 450k arrays for DNA methylation. Copy number variation (CNV) profiles are shown. **Figure 13B****.** Molecular subtype by DNA methylation and RNA-seq (where available), cytogenetic profile, important CNV information, IDH1 status and MGMT promoter methylation status are shown.

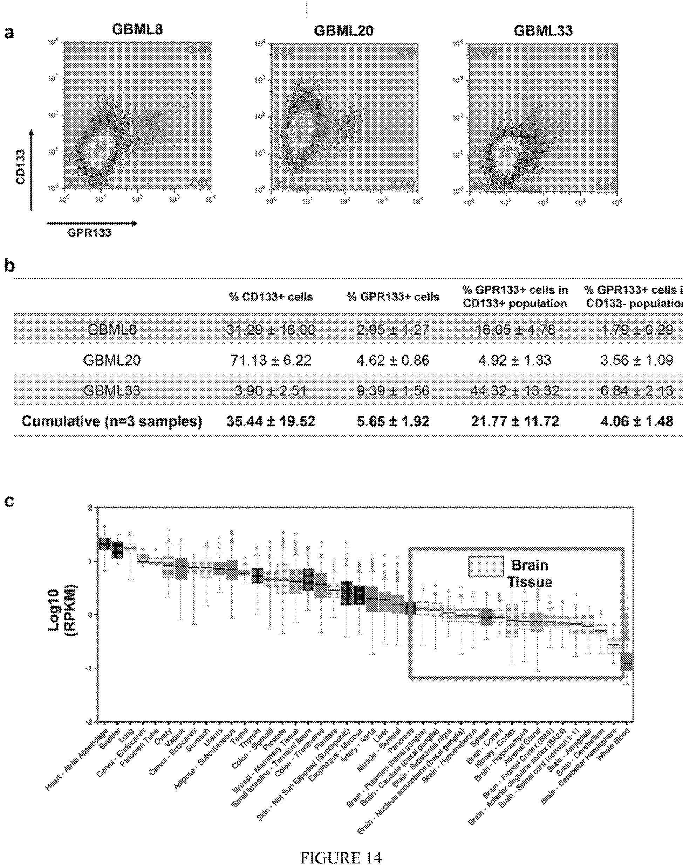

**Figures 14A-14C****.** GPR133 expression information. **Figure 14A****.** Representative flow cytometry analysis of 3 primary GBM cultures for CD133 and GPR133. **Figure 14B****.** Summary statistics describing the abundance of GPR133+ and CD133+ cells in 3 primary cultures based on flow cytometric quantitation. **Figure 14C****.** Analysis of GTEx data on human tissue-specific expression of *GPR133.* Normal brain tissue expresses very low amounts of *GPR133.*

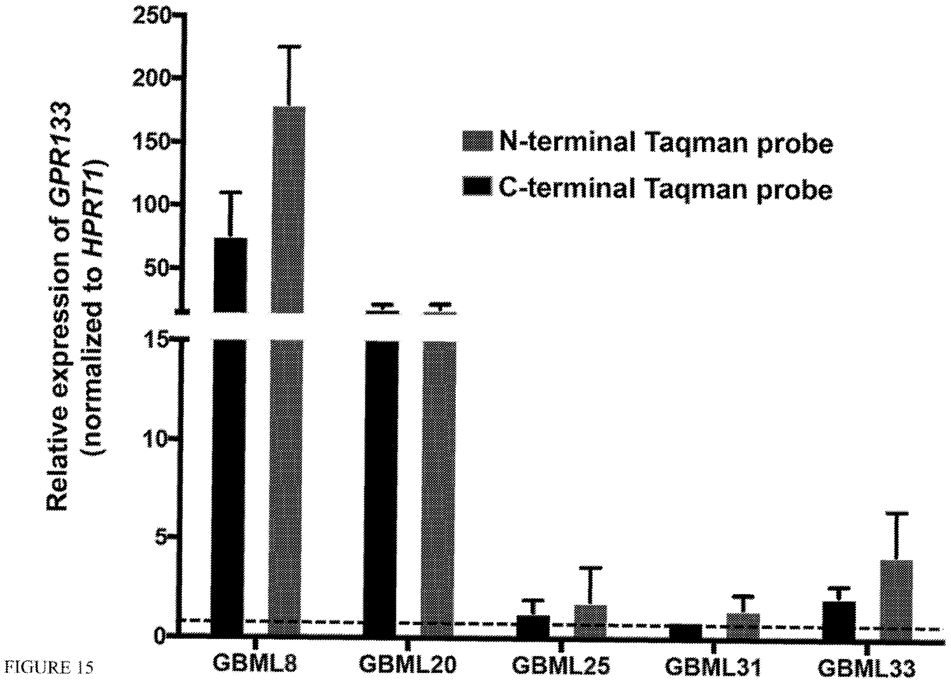

**Figure 15****.** Quantitation of *GPR133* mRNA with two different Taqman assays. The relative amounts of *GPR133* mRNA detected with two different Taqman assays did not differ, suggesting that the full-length *GPR133* splice variant is the dominant one in 5 primary GBM cultures.

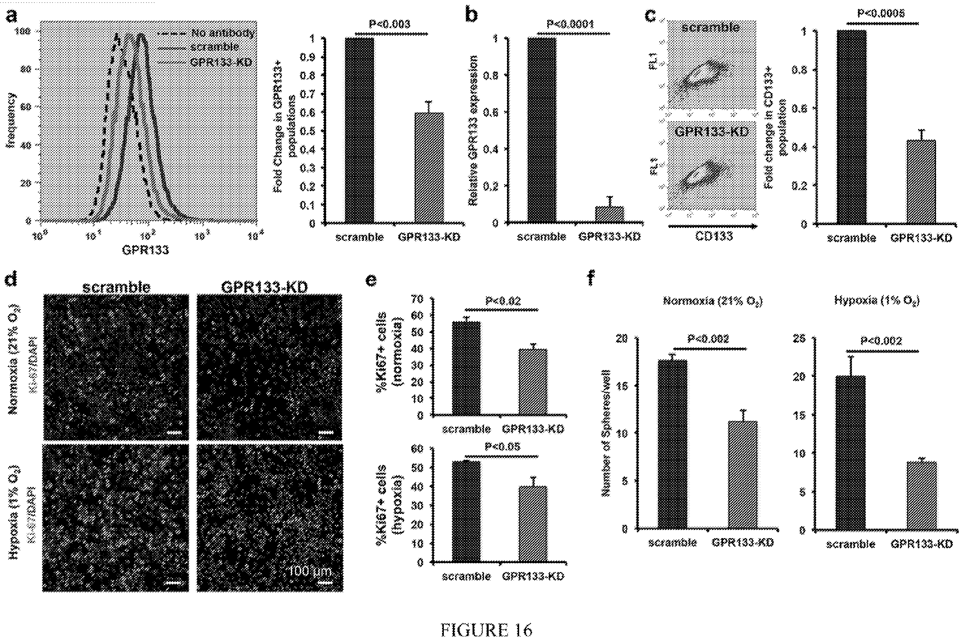

**Figures 16A-16F****.** *In vitro* effects of GPR133 knockdown in GBML8. The layout of this figure is identical to **Figure 9****,** but describes data obtained with GBML8. **Figure 16A****.** GPR133 knockdown (GPR133-KD) reduces the number of GPR133+ cells, as shown by flow cytometry. **Figure 16B****.** GPR133 knockdown decreases *GPR133* mRNA levels. **Figure 16C****.** Flow cytometry indicates reduction in the abundance of CD133+ cells after GPR133 knockdown. **Figures 16** **D,E.** Knockdown of GPR133 reduces the percentage of Ki-67+ cells in both normoxia and hypoxia. **Figure 16F****.** GPR133 knockdown impairs tumorsphere formation in normoxia and hypoxia.

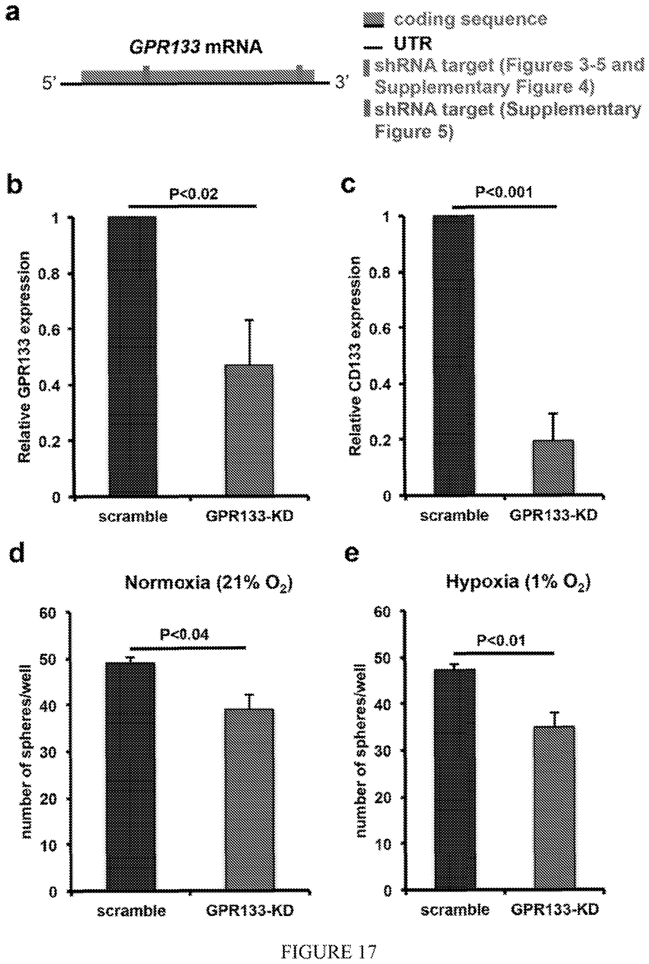

**Figures 17A-17E****.** *In vitro* effects of GPR133 knockdown with a different shRNA construct. **Figure 17A****.** Schematic showing the targets of two shRNA constructs on the full-length *GPR133* mRNA transcript. **Figures 17B****,C.** GPR133 knockdown with the second shRNA construct reduces *GPR133* (**B**) and *CD133* (**C**) mRNA levels in GBML20. **Figures 17D****,E.** GPR133 knockdown impairs tumorsphere formation in normoxia (**D**) and hypoxia (**E**) in GBML20.

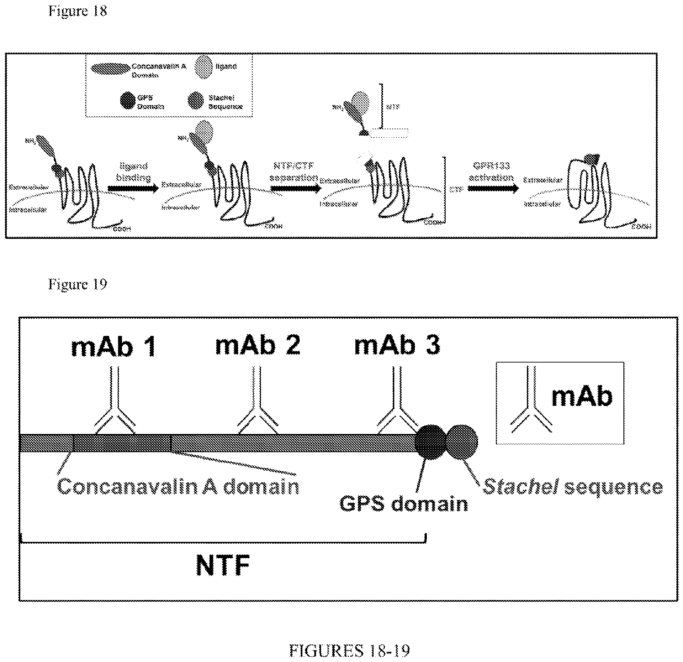

**Figure 18****.** Proposed mechanism of activation of full-length human GPR133. Full-length GPR133, like other adhesion G protein-coupled receptors, contains an autoproteolytic cleavage site (GPS) in the N-terminal ectodomain, with the cleavage occurring due to the GAIN domain on the extracellular N-terminus. The cleavage produces an N-terminal fragment (NTF) and a C-terminal fragment (CTF). It is theorized that the NTF and CTF remain non-covalently bound, until a yet to be discovered ligand binds the N-terminal ectodomain and leads to dissociation of the N-terminal fragment from the C-terminal fragment. This dissociation then allows for conformational changes involving the *Stachel* sequence, leading to receptor activation. The truncated isoform of GPR133, on the other hand, lacks the NTF, so that the *Stachel* sequence is theoretically free of conformational restraints related to the NTF, leading to ligand-independent activation of the receptor.
**Figure 19**. Monoclonal antibodies against the N-terminal ectodomain of human GPR133. Three different mouse monoclonal antibodies (mAb) are being raised against the N-terminal ectodomain, and 1 mouse mAb is being raised against the cytosolic C-terminus of GPR133. All antibodies will be used for GPR133 detection via immunofluorescence microscopy, immunohistochemistry microscopy or immunoblot. In addition, the antibodies targeting the N-terminal ectodomain will also be screened for potential inhibitory effects on GPR133 activation.

### DETAILED DESCRIPTION OF THE INVENTION

The present invention is based on discovery by the inventors of a method for inhibiting growth, self-renewal, or tumorigenicity of glioblastoma (GBM) cells by inhibiting GPR133 expression and/or function in GBM cells, including CD133+ GBM stem cells (GSCs).

### Definitions

The term "high-grade glioma" is used herein to refer to WHO grade III-IV undifferentiated or anaplastic malignant tumors that start in the brain or spine and arise from glial cells. Non-limiting examples of high-grade gliomas encompassed by the present invention include glioblastoma (GBM), gliosarcoma, anaplastic astrocytoma, and anaplastic oligodendroglioma.

As used herewith, the term "glioma cell" refers to any cancer cell within a high-grade glioma tumor. As used herewith, the term "glioblastoma (GBM) cell" refers to any cancer cell within a glioblastoma tumor.

The term "glioma stem cell (GSC)" refers to tumor cells with stem cell-like properties that have been found in gliomas such as GBM. These GSCs encompass a cellular hierarchy that parallels normal tissue and may be responsible for the maintenance and recurrence of gliomas in patients. The hallmarks of GSCs are that they must be able to self-renew, differentiate into tumor lineages and retain tumorigenicity.

The term "adenocarcinoma" is used herein to refer to cancers that occur in glandular or glandular-like tissue. Non-limiting examples of adenocarcinomas encompassed by the present invention include colorectal adenocarcinomas and breast adenocarcinomas, such as invasive ductal carcinoma, ductal carcinoma in situ, and invasive lobular carcinoma.

As used herein, the term "adenocarcinoma cell" refers to any cancer cell within an adenocarcinoma. Non-limiting examples of adenocarcinoma cells encompassed by the present invention include breast adenocarcinoma cells and colorectal carcinoma cells.

The term "lung squamous cell carcinoma" (also called squamous cell carcinoma of the lung or SCC) is used herein to refer to carcinomas of the lung squamous cells and are a type of non-small-cell lung carcinoma that most often arises centrally in larger bronchi and often metastasizes to locoregional lymph nodes (particularly the hilar nodes).

As used herein, the term "lung squamous cell carcinoma cell" refers to any cancer cell within a lung squamous cell carcinoma.

As used herewith, the term "GPR133" refers to G-protein coupled receptor 133 and, in case of a human protein, encompasses four protein isoforms which differ in the length of the N-terminal ectodomain (Figure 6). The sequences of the N-terminal ectodomains of the four human GPR133 isoforms are set forth in SEQ ID NOS: 1-4. The full sequences of the four human GPR133 isoforms are set forth in SEQ ID NOS: 15-18.

The terms "cell growth" and "growth of a cell" refer to the ability of a cell (*e.g.,* a glioma cell, an adenocarcinoma cell, a lung squamous cell carcinoma cell, etc.) to (i) grow (*i.e.*, to increase in cytoplasmic and organelle volume (G1 phase) and/or to increase in genetic material (G2 phase) following the replication during S phase) and (ii) divide to produce two "daughter cells" (M phase).

The term "self-renewal" refers to the ability of a cell (*e.g.,* a glioma cell, an adenocarcinoma cell, a lung squamous cell carcinoma cell, etc.) to go through cycles of cell division while maintaining the undifferentiated state.

The term "tumorigenicity" refers to the ability of a cancer cell (*e.g.,* a glioma cell, an adenocarcinoma cell, a lung squamous cell carcinoma cell, etc.) to produce tumors (*e.g*., as determined by using inoculation of NOD.SCID mice, or athymic (nude) mice/rats, or newborn mice/rats treated with either irradiation or anti-thymocyte globulin).

As used herein, the term "antibody" encompasses immunoglobulins of different classes (*i.e.*, IgA, IgG, IgM, IgD, and IgE) and subclasses (such as IgG1, IgG2 etc.) and includes, without limitation, chimeric antibodies, single-domain antibodies (sdAb, Nanobody), single chain antibodies, humanized antibodies, antibody fragments (*e.g.*, Fab, F(ab')₂, Fv, scFv fragments), single domain antibodies, single variable domain antibodies, immunoglobulin single variable domain (*e.g.*, comprising merely one variable domain V_{H} or V_{L} or larger polypeptides that comprise one or more monomers of an antibody single variable domain polypeptide sequence), monoclonal antibodies, polyclonal antibodies, multispecific antibodies (*e.g.*, bispecific antibodies). As used herein, the term "bispecific antibody" denotes a single polypeptide chain comprising two binding domains.

The term "about" means within an acceptable error range for the particular value as determined by one of ordinary skill in the art, which will depend in part on how the value is measured or determined, *i.e.*, the limitations of the measurement system. For example, "about" can mean within an acceptable standard deviation, per the practice in the art. Alternatively, "about" can mean a range of up to ±20%, preferably up to ±10%, more preferably up to ±5%, and more preferably still up to ±1 % of a given value. Alternatively, particularly with respect to biological systems or processes, the term can mean within an order of magnitude, preferably within 2-fold, of a value. Where particular values are described in the application and claims, unless otherwise stated, the term "about" is implicit and in this context means within an acceptable error range for the particular value.

In the context of the present invention insofar as it relates to any of the disease conditions recited herein, the terms "treat", "treatment", and the like mean to relieve or alleviate at least one symptom associated with such condition, or to slow or reverse the progression of such condition, or to arrest, delay the onset (*i.e.*, the period prior to clinical manifestation of a disease) and/or reduce the risk of developing or worsening a disease (*e.g.*, a high-grade glioma, a breast adenocarcinoma, a colorectal adenocarcinoma, or lung squamous cell carcinoma).

As used herein the term "therapeutically effective" applied to dose or amount refers to that quantity of a compound or pharmaceutical composition that is sufficient to result in a desired activity upon administration to a subject in need thereof. Note that when a combination of active ingredients is administered (*e.g.,* a combination of two or more inhibitors of GPR133) the effective amount of the combination may or may not include amounts of each ingredient that would have been effective if administered individually.

The phrase "pharmaceutically acceptable", as used in connection with compositions of the invention, refers to molecular entities and other ingredients of such compositions that are physiologically tolerable and do not typically produce untoward reactions when administered to a mammal (*e.g*., a human). Preferably, as used herein, the term "pharmaceutically acceptable" means approved by a regulatory agency of the Federal or a state government or listed in the U.S. Pharmacopeia or other generally recognized pharmacopeia for use in mammals, and more particularly in humans.

As used herein, the term "subject" refers to any mammal. In a preferred embodiment, the subject is human.

As used in this specification and the appended claims, the singular forms "a," "an," and "the" include plural references unless the context clearly dictates otherwise.

In accordance with the present invention there may be employed conventional molecular biology, microbiology, and recombinant DNA techniques within the skill of the art. Such techniques are explained fully in the literature. See, *e.g.,* Sambrook, Fritsch & Maniatis, Molecular Cloning: A Laboratory Manual, Second Edition (1989) Cold Spring Harbor Laboratory Press, Cold Spring Harbor, New York (herein "Sambrook *et al.*, 1989"); DNA Cloning: A practical Approach, Volumes I and II (D.N. Glover ed. 1985); Oligonucleotide Synthesis (MJ. Gait ed. 1984); Nucleic Acid Hybridization (B.D. Hames & S.J. Higgins eds.(1985»; Transcription and Translation (B.D. Hames & S.J. Higgins, eds. (1984»; Animal Cell Culture (R.I. Freshney, ed. (1986»; Immobilized Cells and Enzymes (1RL Press, (1986»; B. Perbal, A practical Guide To Molecular Cloning (1984); F.M. Ausubel et al. (eds.), Current Protocols in Molecular Biology, John Wiley & Sons, Inc. (1994); among others.

### Methods of the Disclosure

In one aspect, the disclosure relates to a method for inhibiting growth, self-renewal, or tumorigenicity of a cancer cell (e.g., a glioma cell [for example, a high-grade glioma cell such as a glioblastoma cell, CD133+ glioma stem cell (GSC)], an adenocarcinoma cell [for example, a breast adenocarcinoma cell or a colorectal adenocarcinoma cell], or a lung squamous cancer cell) comprising exposing said cancer cell to a GPR133 inhibitor that is capable of inhibiting expression or function of GPR133 in said cancer cell. In one embodiment, the invention provides a method for inhibiting growth, self-renewal, or tumorigenicity of a glioblastoma (GBM) cell, including CD133+ glioblastoma stem cell (GSC), comprising exposing said cell to a GPR133 inhibitor that inhibits expression or function of GPR133 in said GBM cell.

In another aspect, the disclosure relates to a method for treating a cancer (such as, e.g., a high-grade glioma [e.g., glioblastoma (GBM), gliosarcoma, anaplastic astrocytoma, anaplastic oligodendroglioma], a breast adenocarcinoma, a colorectal adenocarcinoma, or a lung squamous cell carcinoma) in a subject in need thereof, comprising administering to said subject a therapeutically effective amount of an inhibitor of expression or function of GPR133, wherein said inhibitor is administered in an amount which is effective for inhibiting expression or function of GPR133 in cancer cells of said subject. In one embodiment, the invention provides a method for treating glioblastoma (GBM) in a subject in need thereof comprising administering to said subject a therapeutically effective amount of an inhibitor of expression or function of GPR133, wherein said inhibitor is administered in an amount which is effective for inhibiting expression or function of GPR133 in GBM cells, including CD133+ GBM stem cells (GSCs).

In certain embodiments of any of the methods disclosed herein, GPR133 isoform 1 (Uniprot Q6QNK2.1; SEQ ID NO: 15) is selectively inhibited. In certain embodiments of any of the methods of the invention, the inhibitor of GPR133 is an interfering RNA (RNAi) molecule or an antibody specific for GPR133 as described in more detail herein. In certain embodiments of any of the methods of the invention, the cancer cells are CD133-expressing (CD133+) cells located within a hypoxic area of a tumor in the subject. In certain embodiments of any of the methods of the invention, the subject is human, and /or an experimental animal model.

GPR133 inhibitors useful in the methods disclosed herein include, without limitation, small molecules and biologics (*e.g.*, antibodies, peptides, nucleic acid-based inhibitors) which are described in more detail below. Such inhibitors can be delivered to tumors via any delivery method known in the art, including, without limitation, systemic delivery (*e.g*., intravenous, intramuscular, oral, intranasal, by inhalation, sublingual, mucosal, etc.), direct injection within tumors (*e.g*., via intraoperative injection within the tumor or in the walls of the resection cavity by the surgeon; or stereotactic implantation of catheters within the tumor, followed by convection-enhanced delivery (CED) of a GPR133 inhibitor), superselective intra-arterial infusion of a GPR133 inhibitor within the vascular territory of the tumor (*e.g.*, through an endovascular catheter), and viral vector delivery for nucleic acid-based inhibitors (*e.g*., using lentiviral vectors, which by virtue of a modified envelope, selectively infect or transduce CD133+ cancer cells, *see* Bayin et al., 2014, PlosOne, DOI:10,1371/Journal.pone.0116114; or retroviral replicating vectors, *see* Huang et al., 2013, Cancer Gene Ther. 20:544-551).

GPR133 inhibitors may also include cAMP regulators, as it has been shown herein that GPR133 acts via upregulation of cAMP. Non-limiting examples of a cAMP regulators suitable for use in the methods of the invention include forskolin, IBMX, phosphodiesterase inhibitors, cAMP analogs (*e.g*., 8-Bromo-cAMP, cAMPS-Sp and 8-pCPT-2-O-Me-cAMP-AM), and the protein kinase A inhibitor H-89.

GPR133 inhibitors may also include regulators of oxygen tension as well as regulatory molecules modulated by hypoxia, as it has been shown herein that GPR133 expression is regulated by oxygen tension and hypoxia. Non-limiting examples of regulatory molecules modulated by hypoxia which may be used as GPR133 inhibitors in the methods of the invention include inhibitors of Hif1α such as, e.g., chetomin, cryptotanshinone, FM19G11, and PX12.

It is also contemplated herein that more than one inhibitor of GPR133 can be used, and/or such one or more inhibitor(s) can be further combined with other therapeutic agents and/or therapies suitable for treatment of cancers, including, for example, high-grade gliomas, adenocarcinomas, and lung squamous cell carcinomas. Two or more active agents may be co-administered to generate additive or synergistic effects. Suitable therapeutically effective dosages for each agent may be lowered due to the additive action or synergy. In certain embodiments, the use of GPR133 inhibitors can be combined with, *e.g.*, radiation therapy, surgery, chemotherapy (*e.g.,* temozolamide, VEGF inhibitors [such as, *e.g.,* bevacizumab/Avastin], Cilengitide, rindopepimut and other EGFR modulators, nitrosoureas [such as, *e.g*., lomustine, carmustine/BCNU, CCNU], tyrosine kinase inhibitors), immune cell therapy (*e.g*., autologous, genetically engineered T-cells and/or dendritic cells [DCs]), cancer vaccines (*e.g*., cellular- or peptide-based), oncolytic viruses (*e.g*., Ad-RTS-IL-12), Stupp protocol (radiation + temozolamide), alternating electric field therapy (*e.g*., NovoTTF), etc.

### Inhibitors of GPR133

In conjunction with the above methods, the present invention provides inhibitors of GPR133 expression or function, which include, but are not limited to, biologics (*e.g.,* nucleic acid-based inhibitors, peptides, antibodies) and small molecules.

### Inhibitors of GPR133 Expression

Non-limiting examples of inhibitors of GPR133 expression include interfering RNA molecules (*e.g.*, shRNA or siRNA), dsRNA, RNA polymerase III transcribed DNAs, antisense nucleic acids, and genome editing methods such as, *e.g.*, methods which involve the use of Clustered Regularly Interspaced Short Palindromic Repeats (CRISPR)/Cas9 gene systems, methods which involve the use of zinc finger nucleases (ZFNs), methods which involve the use of transcription activator-like effector nucleases (TALENs), as well as methods which involve the use of any other nucleases that can cause DNA breaks or bind to DNA.

RNA interference (RNAi) is a process of sequence-specific post-transcriptional gene silencing by which double stranded RNA (dsRNA) homologous to a target locus can specifically inactivate gene function (Hammond et al., Nature Genet. 2001; 2:110-119; Sharp, Genes Dev. 1999; 13:139-141). This dsRNA-induced gene silencing is mediated by short double-stranded small interfering RNAs (siRNAs) generated from longer dsRNAs by ribonuclease III cleavage (Bernstein et al., Nature 2001; 409:363-366 and Elbashir et al., Genes Dev. 2001; 15:188-200). RNAi-mediated gene silencing is thought to occur *via* sequence-specific RNA degradation, where sequence specificity is determined by the interaction of an siRNA with its complementary sequence within a target RNA (see, *e.g*., Tuschl, Chem. Biochem. 2001; 2:239-245). RNAi can be activated by introduction of siRNAs (Elbashir, et al., Nature 2001; 411: 494-498) or short hairpin RNAs (shRNAs) bearing a fold back stem-loop structure (Paddison et al., Genes Dev. 2002; 16: 948-958; Sui et al., Proc. Natl. Acad. Sci. USA 2002; 99:5515-5520; Brummelkamp et al., Science 2002; 296:550-553; Paul et al., Nature Biotechnol. 2002; 20:505-508).

shRNA/siRNA comprises a double stranded structure typically containing 15 to 50 base pairs and preferably 21 to 25 base pairs and having a nucleotide sequence identical or nearly identical to an expressed target gene or RNA within the cell. The siRNA/shRNA inhibitors of the present invention are preferably short double stranded nucleic acid duplexes (or stem-loop structures in case of shRNA) comprising annealed complementary single stranded nucleic acid molecules. However, the invention also encompasses embodiments in which the siRNAs comprise an annealed RNA:DNA duplex, wherein the sense strand of the duplex is a DNA molecule and the antisense strand of the duplex is a RNA molecule. In some embodiments, duplexed siRNAs have a 2 or 3 nucleotide 3' overhang on each strand of the duplex. In some embodiments, siRNAs/shRNAs have 5'-phosphate and 3'-hydroxyl groups.

According to the present invention, siRNAs may be introduced to a target cell as an annealed duplex siRNA, or as single stranded sense and antisense nucleic acid sequences that, once within the target cell, anneal to form the siRNA duplex. Alternatively, the sense and antisense strands of the siRNA may be encoded on an expression construct that is introduced to the target cell. Upon expression within the target cell, the transcribed sense and antisense strands may anneal to reconstitute the siRNA. 100% sequence complementarity between the siRNA and the target nucleic acid is not required to practice the invention. Expression of shRNA in cells can be obtained by delivery of plasmids or through viral or bacterial vectors. A variety of viral vectors can be used to obtain shRNA expression in cells including adeno-associated viruses (AAVs), adenoviruses, retroviruses, and lentiviruses.

Two main isoforms of human GPR133 exist (Fig. 6): long and short. The long isoform contains a concanavalin A domain in the N-terminal ectodomain and has two variants (isoform 1 and isoform 4) which differ only in the 3' UTR (isoform 4 does not have one) and a small insertion in the N-terminal domain present in isoform 4. There exist two main variants of the short isoform (isoform 2 and isoform 3) that differ in the length of the N-terminal ectodomain.

Four human GPR133 isoforms produced by alternative splicing:

(The sequence of this isoform differs from Isoform 1 as follows:
Amino acids 1-314: Missing.
Amino acids 315-341: QTALNLTKTFLKAVGEILLLPGWIALS (SEQ ID NO: 19) → MEKGTELLVSPSQSGPGGDQPLLVKHR (SEQ ID NO: 20))

(The sequence of this isoform differs from Isoform 1 as follows:
Amino acids 1-481: Missing.
Amino acids 482-491: LITVHLKHRL (SEQ ID NO: 21) → MHRVCFLSFQ (SEQ ID NO: 22))

(The sequence of this isoform differs from Isoform 1 as follows: Amino acids 62-62: G → GASRTHKLTVLPSRNATFVYSNDSAYSNLSATV (SEQ ID NO: 23))

In one specific embodiment, three shRNA GPR133 inhibitors are provided: shRNA_1 which targets isoform 1 (long isoform) in the 3'-UTR (5'- GGGATCATAGATGTGAATTAA-3' SEQ ID NO: 5); shRNA_2 which targets the long isoforms 1 and 4 (exon 5) (5'-GGAGTCACGCTTCTCTATTAC-3' SEQ ID NO: 6); and shRNA_3 which recognizes all isoforms (5'-CCTGCAGGGACTGTTCATATT-3' SEQ ID NO: 7). As shown in Figure 3, all three shRNAs significantly reduce *GPR133* mRNA levels (determined by qRT-PCR).

In certain embodiments, isoform 1 of GPR133 is selectively inhibited (SEQ ID NO: 15; Uniprot Q6QNK2.1). Isoform 1 is the variant most commonly found in GBM (Figures 3 and 15). GPR133 isoform 1, like other adhesion G protein-coupled receptors, contains an autoproteolytic cleavage site (GPS) in the N-terminal ectodomain, with the cleavage occurring due to the GAIN domain on the extracellular N-terminus (Figure 18). The cleavage produces an N-terminal fragment (NTF) and a C-terminal fragment (CTF). Not wishing to be bound by any theory, it is hypothesized that the NTF and CTF remain non-covalently bound, until a yet to be discovered ligand binds the N-terminal ectodomain and leads to dissociation of the N-terminal fragment from the C-terminal fragment. This dissociation then allows for conformational changes involving the Stachel sequence, leading to receptor activation. The truncated isoforms of GPR133, on the other hand, lack the NTF, so that the Stachel sequence is theoretically free of conformational restraints related to the NTF, leading to ligand-independent activation of the receptor.

In certain embodiments, the disclosure relates to the use of genome editing methods for inhibiting expression of GPR133 gene. Non-limiting examples of useful genome editing methods include, e.g., methods which involve the use of Clustered Regularly Interspaced Short Palindromic Repeats (CRISPR)/Cas9 gene systems, methods which involve the use of zinc finger nucleases (ZFNs), methods which involve the use of transcription activator-like effector nucleases (TALENs), as well as methods which involve the use of any other nucleases that can cause DNA breaks or bind to DNA. Genome editing tools such as TALENs, ZFNs, and CRISPR/Cas9 system are examples of targeted nuclease systems: these systems have a DNA-binding member that localizes the nuclease to a target site. The site is then cut by the nuclease. TALENs and ZFNs have the nuclease fused to the DNA-binding member. CRISPR/Cas9 are cognates that find each other on the target DNA. The DNA-binding member is typically designed in light of the intended cognate sequence so as to obtain a nucleolytic action at or near an intended site. For more information on these genome editing methods see, *e.g.,* WO 2013163628, US 20140273235, EP 2336362, WO 2014093479, WO 2014089290, USP 8795965, US 20140357530, WO 2011091324, USP 8106255, US 20120192298, US 20110023159, and US20110281306.

In certain embodiments, the disclosure relates to the use of CRISPR/Cas9 system for inhibiting expression of GPR133 gene. The CRISPR/Cas9 has been used to create a simple, RNA-programmable method to mediate genome editing in mammalian cells, and to generate gene knockouts (via insertion/deletion) or knockins (via homology directed repair (HDR)). To create GPR133 gene disruptions, a single guide RNA (sgRNA) can be generated to direct the Cas9 nuclease to a specific genomic location. Cas9-induced double strand breaks are repaired via the non-homologous end joining DNA repair (NHEJ) or the homology directed repair (HDR) DNA repair pathway. The repair is error prone, and thus insertions and deletions (INDELs) may be introduced that can disrupt gene function. See *e.g.,* Sander & Joung, Nature Biotechnology, 32:347-355, 2014; Takara Clotech: The CRISPR/Cas9 system for targeted genome editing; and New England BioLabs: CRISPR/Cas9 and Targeted Genome Editing: A New Era in Molecular Biology).

Antisense oligonucleotides, including antisense DNA, RNA, and DNA/RNA molecules, act to directly block the translation of mRNA by binding to targeted mRNA and preventing protein translation. Preferably, antisense oligonucleotides are of at least about 15 bases and are complementary to unique regions of the target DNA sequence. Such antisense oligonucleotides can be synthesized, *e.g.,* by conventional techniques (see, *e.g.,* Dallas et al., (2006) Med. Sci. Monit. 12(4):RA67-74; Kalota et al., (2006) Handb. Exp. Pharmacol. 173:173-96; Lutzelburger et al., (2006) Handb. Exp. Pharmacol. 173:243-59).

RNA polymerase III-transcribed DNAs contain promoters, such as the U6 promoter. These DNAs can be transcribed to produce small hairpin RNAs in the cell that can function as siRNA or linear RNAs that can function as antisense RNA. The inhibitor may be polymerized *in vitro,* recombinant RNA, contain chimeric sequences, or derivatives of these groups. The inhibitor may contain ribonucleotides, deoxyribonucleotides, synthetic nucleotides, or any suitable combination such that the target RNA and/or gene is inhibited. In addition, these forms of nucleic acid may be single, double, triple, or quadruple stranded (see for example Bass (2001) Nature, 411, 428 429; Elbashir et al., (2001) Nature, 411, 494 498; and PCT Publication Nos. WO 00/44895, WO 01/36646, WO 99/32619, WO 00/01846, WO 01/29058, WO 99/07409, WO 00/44914).

Aptamer nucleic acid sequences are readily made that bind to a wide variety of target molecules. The aptamer nucleic acid sequences useful in the methods of the invention can be comprised entirely of RNA or partially of RNA, or entirely or partially of DNA and/or other nucleotide analogs. Aptamers are typically developed to bind particular ligands by employing known *in vivo* or *in vitro* (most typically, *in vitro*) selection techniques known as SELEX (Systematic Evolution of Ligands by Exponential Enrichment). Methods of making aptamers are described in, for example, Ellington and Szostak (1990) Nature 346:818, Tuerk and Gold (1990) Science 249:505, U.S. Pat. No. 5,582,981; PCT Publication No. WO 00/20040; U.S. Pat. No. 5,270,163; Lorsch and Szostak (1994) Biochem. 33:973; Mannironi et al., (1997) Biochem. 36:9726; Blind (1999) Proc. Nat'l. Acad. Sci. USA 96:3606-3610; Huizenga and Szostak (1995) Biochem. 34:656-665; PCT Publication Nos. WO 99/54506, WO 99/27133, and WO 97/42317; and U.S. Pat. No. 5,756,291.

Nucleic acid-based inhibitors may include one or more modifications, e.g., to increase intracellular stability and efficacy (e.g., modifications to the base moiety, sugar moiety, phosphate moiety, phosphate-sugar backbone, or a combination thereof). For example, the phosphodiester linkages may be modified to include at least one heteroatom other than oxygen, such as nitrogen or sulfur. In this case, for example, the phosphodiester linkage may be replaced by a phosphothioester linkage. Similarly, bases may be modified to block the activity of adenosine deaminase. Other examples of useful modifications are morpholino modifications and locked nucleic acids (LNA). Where the nucleic acid-based inhibitor molecule is produced synthetically, or by *in vitro* transcription, a modified nucleoside may be introduced during synthesis or transcription.

Non-limiting examples of modified base moieties include inosine, 5-fluorouracil, 5-bromouracil, 5-chlorouracil, 5-iodouracil, hypoxanthine, xanthine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5-methoxycarboxymethyluracil, 5-methoxyuracil, 2-methylthio-N6-isopentenyladenine, uracil-5-oxyacetic acid (v), pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine.

Non-limiting examples of modified sugar moieties include arabinose, 2-fluoroarabinose, xylulose, and hexose. Modified siRNAs may contain substituted sugar moieties comprising one of the following at the 2' position: OH, SH, SCH₃, F, OCN, O(CH₂)ₙNH₂ or O(CH₂)ₙCH₃ where n is from 1 to about 10; C₁ to C₁₀ lower alkyl, substituted lower alkyl, alkaryl or aralkyl; Cl; Br; CN; CF₃; OCF₃; O-; S-, or N-alkyl; O-, S-, or N-alkenyl; SOCH₃; SO₂CH₃; ONO₂; NO₂; N₃; NH₂; heterocycloalkyl; heterocycloalkaryl; aminoalkylamino; polyalkylamino; substituted sialyl; a fluorescein moiety; a reporter group; a group for improving the pharmacokinetic properties; or a group for improving the pharmacodynamic properties, and other substituents having similar properties. Modified nucleic acid-based inhibitors may also have sugar mimetics such as cyclobutyls or other carbocyclics in place of the pentofuranosyl group.

Non-limiting examples of modifications of phosphate backbone include a phosphorothioate, a phosphorodithioate, a phosphoramidothioate, a phosphoramidate, a phosphordiamidate, a methylphosphonate, a phosphotriester, an alkyl phosphotriester, and a formacetal or analog thereof, as well as chimeras between methylphosphonate and phosphodiester, short chain alkyl, or cycloalkyl intersugar linkages or short chain heteroatomic or heterocyclic intersugar linkages. Specific non-limiting examples include those with CH₂-NH-O-CH₂, CH₂-N(CH₃)-O-CH₂, CH₂-O-N(CH₃)-CH₂, CH₂-N(CH₃)-N(CH₃)-CH₂ and O-N(CH₃)-CH₂-CH₂ backbones (where phosphodiester is O-PO₂-O-CH₂). U.S. Patent No. 5,677,437 describes heteroaromatic oligonucleoside linkages. Nitrogen linkers or groups containing nitrogen can also be used to prepare oligonucleotide mimics (U.S. Patents Nos. 5,792,844 and 5,783,682). U.S. Patent No. 5,637,684 describes phosphoramidate and phosphorothioamidate oligomeric compounds.

Also envisioned are modified nucleic acid-based inhibitors having morpholino backbone structures in which the bases are linked to 6-membered morpholine rings, which are connected to other morpholine-linked bases *via* non-ionic phosphorodiamidate intersubunit linkages. Morpholino derivatives are highly resistant to nucleases and have good targeting predictability (U.S. Patent No. 5,034,506; Summerton, Biochim. Biophys. Acta 1999; 1489:141-158; Summerton and Weller, Antisense Nucleic Acid Drug Dev. 1997; 7:187-195; Arora et al., J. Pharmacol. Exp. Ther. 2000;292:921-928; Qin et al., Antisense Nucleic Acid Drug Dev. 2000; 10:11-16; Heasman et al., Dev. Biol. 2000; 222:124-134; Nasevicius and Ekker, Nat. Genet. 2000; 26:216-220).

Another type of a useful modification is the peptide-nucleic acid (PNA) backbone: the phosphodiester backbone of the oligonucleotide may be replaced with a polyamide backbone, the bases being bound directly or indirectly to the aza nitrogen atoms of the polyamide backbone (Nielsen et al., Science 1991;254:1497).

In other embodiments, locked nucleic acids (LNA) can be used (reviewed in, *e.g.,* Jepsen and Wengel, Curr. Opin. Drug Discov. Devel. 2004; 7:188-194; Crinelli et al., Curr. Drug Targets 2004; 5:745-752). LNA are nucleic acid analog(s) with a 2'-O, 4'-C methylene bridge. This bridge restricts the flexibility of the ribofuranose ring and locks the structure into a rigid C3-endo conformation, conferring enhanced hybridization performance and exceptional biostability.

Modified nucleic acid-based inhibitors can include appending groups such as, *e.g.,* peptides, or agents facilitating transport across the cell membrane (see, *e.g.,* Letsinger et al., Proc. Natl. Acad. Sci. USA 1989; 86:6553-6556; Lemaitre et al., Proc. Natl. Acad. Sci. USA 1987; 84:648-652; PCT Publication No. WO 88/09810), or blood-brain barrier (see, *e.g.,* PCT Publication No. WO 89/10134), etc.

Nucleic acid-based inhibitors of the invention can be synthesized by standard methods known in the art, *e.g.,* by use of an automated synthesizer. In one embodiment, RNA molecules can be chemically synthesized using appropriately protected ribonucleoside phosphoramidites and a conventional DNA/RNA synthesizer. In case of siRNA molecules, following chemical synthesis, single stranded RNA molecules are deprotected, annealed to form siRNAs, and purified *(e.g.,* by gel electrophoresis or HPLC). Commercial suppliers of synthetic RNA molecules or synthesis reagents include, *e.g*., Proligo (Hamburg, Germany), Dharmacon Research (Lafayette, Colo.), Pierce Chemical (part of Perbio Science, Rockford, Ill.), Glen Research (Sterling, Va.), ChemGenes (Ashland, Mass.) and Cruachem (Glasgow, UK).

Alternatively, standard procedures may be used for *in vitro* transcription of RNA from DNA templates carrying RNA polymerase promoter sequences (*e.g.*, T7 or SP6 RNA polymerase promoter sequences) (Donzé and Picard, Nucleic Acids Res. 2002; 30:e46; Yu et al., Proc. Natl. Acad. Sci. USA 2002; 99:6047-6052; Weintraub, H. et al., Trends in Genetics, Vol. 1 (1) 1986). In the case of siRNA molecules, the sense and antisense transcripts may be synthesized in two independent reactions and annealed later, or may be synthesized simultaneously in a single reaction. siRNA molecules may be formed within a cell by transcription of RNA from an expression construct introduced into the cell. For example, both a protocol and an expression construct for *in vivo* expression of siRNAs are described in Yu *et al.*, *supra.*

The expression constructs for *in vivo* production of nucleic acid-based inhibitors of the invention comprise encoding sequences operably linked to elements necessary for the proper transcription, including promoter elements and transcription termination signals. Non-limiting examples of promoters for use in such expression constructs include the polymerase-III H1-RNA promoter (see, *e.g.,* Brummelkamp *et al.*, *supra*) and the U6 polymerase-III promoter (see, *e.g.,* Sui *et al.*, *supra;* Paul, *et al. supra;* and Yu *et al.*, *supra*). The expression constructs can further comprise vector sequences that facilitate the cloning of the expression constructs. Standard vectors that may be used in practicing the current invention are known in the art (*e.g.,* pSilencer 2.0-U6 vector, Ambion Inc., Austin, TX).

### Antibody Inhibitors

The disclosure provides that the GPR133 inhibitors can also be antibodies, including, without limitation, monoclonal antibodies, polyclonal antibodies, recombinant antibodies, chimeric antibodies, naturally occurring antibodies, or specific antigen binding and/or functional domains, motifs, or fragments thereof. The antibodies of the invention include human antibodies, non-human animal antibodies from any animal species (*e.g*., mouse, rat, rabbit, chicken, dog, goat, camelids [dromedaries, camels, llamas and alpacas], and monkey), and/or humanized antibodies. The invention provides in the broadest scope anti-GPR133 antibodies, now known and/or commercially available, or later developed.

Techniques for the production of antibodies are well known in the art and described, *e.g.* in Harlow and Lane "Antibodies, A Laboratory Manual", Cold Spring Harbor Laboratory Press, 1988 and Harlow and Lane "Using Antibodies: A Laboratory Manual" Cold Spring Harbor Laboratory Press, 1999.

In certain embodiments, the antibodies are directed to the N-terminal ectodomain and/or the cytosolic C-terminus of GPR133 (Figure 18). In one embodiment, the anti-GPR133 antibody is directed to the Concanavalin A domain of the N-terminal ectodomain. Such anti-GPR133 antibody may recognize the sequence VNKGIYLKEEKGVTLLYYGR YNSSCISKPEQCGPEGVTFSFFWKTQGEQSRPIPSAYGGQVISNGFKVCSSGGRGSVELYT RDNSMTWEASFSPPGPYWTHVLFTWKSKEGLKVYVNGTLVIGSEQDQAK (SEQ ID NO: 8) (mAb 1 as depicted in Figure 18). In another embodiment, the anti-GPR133 antibody is directed to a sequence within the N-terminal ectodomain. Such anti-GPR133 antibody may recognize the sequence AVVLSLIDTIDTVMGHVSSNLHGSTPQVTVEGSSAMAEFSVAKI LPKTVNSSHYRFPAHGQSFIQIPHEAFHRHAWSTVVGLLYHSMHYYLNNIWPAHTKIA EAMHHQDC (SEQ ID NO: 9) (mAb 2 as depicted in Figure 18). In another embodiment, the anti-GPR133 antibody is directed to a sequence within the N-terminal ectodomain and adjacent to GPS and *Stachel* sequences. Such anti-GPR133 antibody may recognize the sequence TRKQHSEATNSSNRC (SEQ ID NO: 10) and/or CSSGEGVWSNHG (SEQ ID NO: 11) (mAb 3 as depicted in Figure 18). In another embodiment, the anti-GPR133 antibody is directed to a sequence in the cytosolic C-terminus. Such anti-GPR133 antibody may recognize the sequences CSSARTSNAKPFHSD (SEQ ID NO: 12) and/or LMNGTRPGMASTKLSC (SEQ ID NO: 13) (mAb 4 as depicted in Figure 18).

Non-limiting examples of commercially available anti-GPR133 antibodies include, but are not limited to, anti-GPR133 antibodies produced by LifeSpan Biociences, Inc. (*e.g*., Anti-GPR133 Antibody (N-Terminus) IHC-plus™ LS-A2034; Anti-GPR133 Antibody (Cytoplasmic Domain) IHC-plus™ LS-A2035; Anti-GPR133 Antibody (C-Terminus) IHC-plus™ LS-A2037; Anti-GPR133 Antibody (aa461-510) LS-C120684; Anti-GPR133 Antibody (aa477-507) LS-C166621; Anti-GPR133 Antibody (aa449-498) LS-C292008; Anti-GPR133 Antibody (aa477-507, FITC) LS-C235902; Anti-GPR133 Antibody (aa477-507, PE) LS-C235904; Anti-GPR133 Antibody (aa477-507, Azide-free, HRP) LS-C249116; Anti-GPR133 Antibody (aa477-507, Biotin) LS-C235901; Anti-GPR133 Antibody (aa477-507) LS-C321730; Anti-GPR133 Antibody (aa477-507, APC) LS-C235899; Anti-GPR133 Antibody (aa477-507, AP) LS-C235900); AbCAM® (Cambridge, MA) (*e.g*., ab103990, ab136251, ab67349, ab150640, and ab188637); and any GPR133 antibody products commercially available, *e.g.*, Browse EMD Millipore's Extensive Line of anti-GPR133 Mono- and Polyclonal Antibodies; Browse R&D Systems for anti-GPR133 antibodies; OriGene antibodies for GPR133 and/or OriGene Custom Antibody Services for GPR133; Novus Biologicals GPR133 antibodies; Cloud-Clone Corp. Antibodies for GPR133; ThermoFisher Antibodies for GPR133 (including pierce PA5-33624); and antibodies-online antibodies for GPR133.

The antibodies can also be either full length antibodies or single-domain antibodies. In one specific embodiment, the antibody is a single-domain antibody contains an antibody fragment consisting of a single monomeric variable antibody domain, and lacking the light chain and CH1 domain of the heavy chain. For instance, the single domain antibodies contain VHH fragments which are engineered from heavy chain antibodies in camelids, *e.g.*, dromedaries, camels, llamas and alpacas.

In one specific embodiment, the disclosure provides VHH single domain anti-GPR133 antibodies. The small size (about 15 kD) of these antibodies allows them to fit into epitopes that are normally not accessible to traditional antibodies and also enables such antibodies to penetrate tissues faster, even readily crossing the blood-brain barrier (BBB). Moreover, the camelid (*e.g*., Llama) VHH single domain antibodies are extremely stable and are resistant to both high acidity and temperature, even being able to fold back into a functional protein after denaturation.

The disclosure also encompasses bispecific antibodies, which bind to two different antigens (e.g., one arm binds to a specific epitope on GPR133 protein on glioma cells, and the other arm binds to the CD3 receptor or another protein on T cells or on dendritic cells (DCs) in order to facilitate glioma tumor cell killing by these cells). In certain embodiments, the recombinant bispecific antibodies are diabodies, which are a class of small bivalent and bispecific antibody fragments that can be expressed in bacteria (*e.g. E.coli*), yeast (*e.g. Pichia pastoris*), and cells from higher eukaryotic organisms in functional form and with high yields (up to 1 g/L). Diabodies comprise a heavy (VH) chain variable domain connected to a light chain variable domain (VL) on the same polypeptide chain (VH-VL) connected by a peptide linker that is too short to allow pairing between the two domains on the same chain. This forces pairing with the complementary domains of another chain and promotes the assembly of a dimeric molecule with two functional antigen binding sites.

To construct bispecific diabodies, the variable domains derived from antibody A and antibody B can be fused to create the two chains VHA-VLB, VHB-VLA. Each chain is inactive in binding to antigen, but recreates the functional antigen binding sites of antibodies A and B on pairing with the other chain.

Antibodies useful in the methods disclosed herein can be generated using any antibody producing methods, now known or later developed in the art. For example, phage display methods can be used. In phage display methods, functional antibody domains are displayed on the surface of phage particles which carry the nucleic acid sequences encoding them. In a particular embodiment, such phage can be utilized to display antigen binding domains expressed from a repertoire or combinatorial antibody library (*e.g.*, human or murine). In particular, DNA sequences encoding VH and VL domains are amplified from animal cDNA libraries (*e.g*., human or murine cDNA libraries of lymphoid tissues). The DNA encoding the V_{H} and V_{L} domains are recombined together with an scFv linker by PCR and cloned into a phagemid vector. The vector is electroporated in *E. coli* and the *E. coli* is infected with helper phage. Phage used in these methods are typically filamentous phage including fd and M13 binding domains expressed from phage with Fab, Fv or disulfide stabilized Fv antibody domains recombinantly fused to either the phage gene III or gene VIII protein. Phage expressing an antigen binding domain that binds to or-portions thereof can be selected or identified with antigen *e.g*., using labeled antigen or antigen bound or captured to a solid surface or bead. Alternatively it is also possible to produce the recombinant bispecific antibody constructs by hybridoma fusion.

Depending on the mode of application, bispecific antibodies can be administered to a patient. One of the problems which may occur is that bispecific antibodies are very quickly excreted from the body of the patient and accumulate in excreting organs like the kidney. One option to overcome this problem is to isolate *ex vivo* T cells from the blood of the patients for example by leukapheresis and to incubate T cells with the diabody. Such incubated mixtures can thereafter be re-administered to the patient.

For more information about the recombinant bispecific antibody and its application for eradication of glioblastoma stem cells, *see* WO2014128185 which discloses a recombinant bispecific antibody that binds to the CD133 antigen on tumor cells and to the CD3 T cell receptor; and Prasad et al., DOI: 10.1158/0008-5472, Cancer Res., 2015.

In case of andenocarcinoma or lung squamous cell carcinoma treatments relying on systemic delivery, anti-GPR133 antibodies may have to cross into or be transported (e.g., actively or passively) into such cancers. This may be possible using small antibodies described above. Alternatively, enhanced and/or targeted delivery of anti-GPR133 antibodies can be achieved by using bispecific antibodies with the second antigen being, *e.g.,* an adenocarcinoma-specific receptor or a lung squamous cell carcinoma-specific receptor. Non-limiting examples of such receptors include, *e.g.*, estrogen receptors, progesterone receptors and Her2 receptors which are expressed in breast adenocarcinomas, and folate receptor α which is expressed in lung squamous cell carcinomas. Alternatively, antibodies can be administered directly into the tumor, *e.g.*, by injection or using continuous peristaltic pumps.

In case of high-grade glioma treatments relying on systemic delivery, anti-GPR133 antibodies have to cross the blood-brain barrier (BBB). This may be possible using small antibodies described above. Alternatively, enhanced brain delivery of anti-GPR133 antibodies can be achieved by using bispecific antibodies with the second antigen being, *e.g.,* an endogenous BBB receptor, such as, *e.g*., insulin receptor (InsR), transferrin receptor (TfR), low-density lipoprotein receptor-related proteins 1 and 2 (Lrp1 and Lrp2), receptor-associated protein, apolipoprotein E, or melanotransferrin/p97 (see, *e.g.,* Yu et al., Sci. Transl. Med. 6, 261ra154 (2014); Pardridge and Boado, Methods Enzymol. 2012, 503:269-292; Yu and Watts, Neurotherapeutics. 2013, 10(3): 459-472). Alternatively, antibodies can be administered directly into the tumor, *e.g*., by injection or using continuous peristaltic pumps.

### Small Molecule Inhibitors

The present disclosure also encompasses various small molecule inhibitors of GPR133 gene expression and/or protein function. Small molecules are a diverse group of synthetic and natural substances generally having low molecular weights (preferably less than about 2000 Daltons, less than about 1000 Daltons, or less than about 500 Daltons). Small molecules, without limitation, may be, for example, nucleic acids, peptides, polypeptides, peptide nucleic acids, peptidomimetics, carbohydrates, lipids, or other organic (carbon containing) or inorganic molecules and may be synthetic or naturally occurring or optionally derivatized. Such small molecules may be a therapeutically deliverable substance or may be further derivatized to facilitate delivery or targeting.

The above compounds may be obtained by methods known to skilled practitioners. Synthesis, characterization, and biological activity data for some of these compounds are disclosed in, *e.g.*, Scifinder, Chem. Pharm. Bull. 49(8) 988-998 (2001); J Pharmacol Sci 96, 42-52 (2004).

Small molecule inhibitors of GPR133 can be isolated from natural sources (for example, plants, fungi, microbes and the like) or isolated from random or combinatorial chemical libraries of synthetic or natural compounds, or synthesized. *See* Werner et al., (2006) Brief Funct. Genomic Proteomic 5(1):32-6. Many random or combinatorial libraries are known in the art that can be used. Numerous means are currently used for random and directed synthesis of saccharide, peptide, and nucleic acid based compounds. Synthetic compound libraries are commercially available from Maybridge Chemical Co. (Trevillet, Cornwall, UK), Comgenex (Princeton, N.J.), Brandon Associates (Merrimack, N.H.), and Microsource (New Milford, Conn.). A rare chemical library is available from Aldrich (Milwaukee, Wis.). Alternatively, libraries of natural compounds in the form of bacterial, fungal, plant and animal extracts are available from, *e.g*., Pan Laboratories (Bothell, Wash.) or MycoSearch (N.C.), or are readily producible. Additionally, natural and synthetically produced libraries and compounds are readily modified through conventional chemical, physical, and biochemical means (Blondelle et al., (1996) Tib Tech 14:60).

Methods for preparing libraries of molecules are well known in the art and many libraries are commercially available. Libraries of interest in the invention include peptide libraries, randomized oligonucleotide libraries, synthetic organic combinatorial libraries, and the like. Degenerate peptide libraries can be readily prepared in solution, in immobilized form as bacterial flagella peptide display libraries or as phage display libraries. Peptide ligands can be selected from combinatorial libraries of peptides containing at least one amino acid. Libraries can be synthesized of peptoids and non-peptide synthetic moieties. Such libraries can further be synthesized which contain non-peptide synthetic moieties, which are less subject to enzymatic degradation compared to their naturally-occurring counterparts. Libraries are also meant to include for example but are not limited to peptide-on-plasmid libraries, polysome libraries, aptamer libraries, synthetic peptide libraries, synthetic small molecule libraries and chemical libraries. The libraries can also comprise cyclic carbon or heterocyclic structure and/or aromatic or polyaromatic structures substituted with one or more of the above-identified functional groups.

Examples of chemically synthesized libraries are described in Fodor et al., (1991) Science 251:767-773; Houghten et al., (1991) Nature 354:84-86; Lam et al., (1991) Nature 354:82-84; Medynski, (1994) BioTechnology 12:709-710; Gallop et al., (1994) J. Medicinal Chemistry 37(9):1233-1251; Ohlmeyer et al., (1993) Proc. Natl. Acad. Sci. USA 90:10922-10926; Erb et al., (1994) Proc. Natl. Acad. Sci. USA 91:11422-11426; Houghten et al., (1992) Biotechniques 13:412; Jayawickreme et al., (1994) Proc. Natl. Acad. Sci. USA 91:1614-1618; Salmon et al., (1993) Proc. Natl. Acad. Sci. USA 90:11708-11712; PCT Publication No. WO 93/20242, dated Oct. 14, 1993; and Brenner et al., (1992) Proc. Natl. Acad. Sci. USA 89:5381-5383.

Examples of phage display libraries are described in Scott et al., (1990) Science 249:386-390; Devlin et al., (1990) Science, 249:404-406; Christian, et al., (1992) J. Mol. Biol. 227:711-718; Lenstra, (1992) J. Immunol. Meth. 152:149-157; Kay et al., (1993) Gene 128:59-65; and PCT Publication No. WO 94/18318.

Screening the libraries can be accomplished by any variety of commonly known methods. See, for example, the following references, which disclose screening of peptide libraries: Parmley and Smith, (1989) Adv. Exp. Med. Biol. 251:215-218; Scott and Smith, (1990) Science 249:386-390; Fowlkes et al., (1992) BioTechniques 13:422-427; Oldenburg et al., (1992) Proc. Natl. Acad. Sci. USA 89:5393-5397; Yu et al., (1994) Cell 76:933-945; Staudt et al., (1988) Science 241:577-580; Bock et al., (1992) Nature 355:564-566; Tuerk et al., (1992) Proc. Natl. Acad. Sci. USA 89:6988-6992; Ellington et al., (1992) Nature 355:850-852; U.S. Pat. Nos. 5,096,815; 5,223,409; and 5,198,346, all to Ladner et al.; Rebar et al., (1993) Science 263:671-673; and PCT Pub. WO 94/18318.

Identification and screening of inhibitors of GPR133 can be further facilitated by X-ray crystallography, neutron diffraction, nuclear magnetic resonance spectrometry, and other techniques for structure determination. These techniques provide for the rational design or identification of inhibitors. Methods for screening and identifying GPR133 inhibitors are known in the art, and the disclosure contemplates any inhibitor screening and identification approaches, now known or later developed, in the art.

### Compositions and Methods of Administration

The disclosure further provides that the GPR133 inhibitors can be used as pharmaceutical compositions and can be optionally combined with other GPR133 inhibitors or other therapeutic molecules and/or treatments for cancers. In certain embodiments, a GPR133 inhibitor is used before, during, and/or after such other therapeutic molecules and/or treatments. Non-limiting examples of additional therapeutic molecules and treatments for cancers which can be combined with the use of GPR133 inhibitors include, *e.g.,* radiation therapy, surgery, chemotherapy (*e.g.,* temozolamide, VEGF inhibitors [such as, *e.g*., bevacizumab/Avastin], Cilengitide, rindopepimut and other EGFR modulators, nitrosoureas [such as, *e.g*., lomustine, carmustine/BCNU, CCNU], tyrosine kinase inhibitors), immune cell therapy (*e.g*., autologous, genetically engineered T-cells and/or dendritic cells [DCs]), cancer vaccines (*e.g*., cellular- or peptide-based), oncolytic viruses (*e.g*., Ad-RTS-IL-12), Stupp protocol (radiation + temozolamide), alternating electric field therapy (*e.g*., NovoTTF), etc.

In some embodiments, at least one inhibitor of GPR133 is formulated into a suitable pharmaceutical preparation such as, *e.g*., solution, suspension, tablet, dispersible tablet, pill, capsule, powder, sustained release formulation or elixir, for oral administration; sterile solution or suspension for parenteral administration; powdered or liquid spray, nose drops, a gel or ointment for intranasal administration; powdered or liquid spray for administration by inhalation; films for sublingual administration; patch for transdermal administration, etc. An inhibitor of GPR133 can be formulated into pharmaceutical compositions using any of the techniques and procedures known in the art (see, *e.g.,* Ansel Introduction to Pharmaceutical Dosage Forms, Fourth Edition 1985, 126).

In the compositions, effective concentrations of one or more inhibitors of GPR133 or pharmaceutically acceptable derivatives thereof is (are) mixed with a suitable pharmaceutical carrier or vehicle.

Pharmaceutically acceptable derivatives include acids, bases, enol ethers and esters, salts, esters, hydrates, solvates and prodrug forms. A suitable derivative is selected such that its pharmacokinetic properties are superior with respect to at least one characteristic to the corresponding parent agent. The inhibitor of GPR133 may be derivatized prior to formulation.

The amount of the inhibitor of GPR133 administered and the regimen of administration depends on absorption, inactivation and excretion rates of the active agent, the physicochemical characteristics of the agent, the severity of the condition to be alleviated, the age, condition, body weight, sex and diet of the patient, the disease state, other medications administered, and other factors known to those of skill in the art. An effective amount to treat the disease would broadly range (*e.g*., between about 0.001 mg and about 2000 mg per kg body weight of the recipient per day), and may be administered as a single dose or divided doses.

It is to be further understood that for any particular subject, specific dosage regimens should be adjusted over time according to the individual need and the professional judgment of the person administering or supervising the administration of the compositions.

The compositions are intended to be administered by a suitable route, including by way of example and without limitation, systemically (*e.g*., intravenously, intramuscularly, orally, intranasally, by inhalation, sublingually, mucosally, etc.), by direct injection within tumors (*e.g*., via intraoperative injection within the tumor or in the walls of the resection cavity by the surgeon; or stereotactic implantation of catheters within the tumor, followed by convection-enhanced delivery (CED) of a GPR133 inhibitor), by superselective intra-arterial infusion of a GPR133 inhibitor within the vascular territory of the tumor (*e.g*., through an endovascular catheter), and by viral vector delivery for nucleic acid-based inhibitors (*e.g*., using lentiviral vectors, which by virtue of a modified envelope, selectively infect or transduce CD133+ cancer cells, or retroviral replicating vectors). The compositions are in liquid, semi-liquid or solid form and are formulated in a manner suitable for each route of administration.

Solutions or suspensions can include any of the following components, in any combination: a sterile diluent, including by way of example without limitation, water for injection, saline solution, fixed oil, polyethylene glycol, glycerine, propylene glycol or other synthetic solvent; antimicrobial agents, such as benzyl alcohol and methyl parabens; antioxidants, such as ascorbic acid and sodium bisulfite; chelating agents, such as ethylenediaminetetraacetic acid (EDTA); buffers, such as acetates, citrates and phosphates; and agents for the adjustment of tonicity, such as sodium chloride or dextrose.

In instances in which the agents exhibit insufficient solubility, methods for solubilizing agents may be used. Such methods are known to those of skill in this art, and include, but are not limited to, using co-solvents, such as, *e.g.*, dimethylsulfoxide (DMSO), using surfactants, such as TWEEN®80, or dissolution in aqueous sodium bicarbonate. Pharmaceutically acceptable derivatives of the agents may also be used in formulating effective pharmaceutical compositions.

The composition can contain along with the active agent, for example and without limitation: a diluent such as lactose, sucrose, dicalcium phosphate, or carboxymethylcellulose; a lubricant, such as magnesium stearate, calcium stearate and talc; and a binder such as starch, natural gums, such as gum acacia gelatin, glucose, molasses, polyvinylpyrrolidone, celluloses and derivatives thereof, povidone, crospovidones and other such binders known to those of skill in the art. Liquid pharmaceutically administrable compositions can, for example, be prepared by dissolving, dispersing, or otherwise mixing an active agent as defined above and optional pharmaceutical adjuvants in a carrier, such as, by way of example and without limitation, water, saline, aqueous dextrose, glycerol, glycols, ethanol, and the like, to thereby form a solution or suspension. If desired, the pharmaceutical composition to be administered may also contain minor amounts of nontoxic auxiliary substances such as wetting agents, emulsifying agents, or solubilizing agents, pH buffering agents and the like, such as, by way of example and without limitation, acetate, sodium citrate, cyclodextrin derivatives, sorbitan monolaurate, triethanolamine sodium acetate, triethanolamine oleate, and other such agents. Actual methods of preparing such dosage forms are known, or will be apparent, to those skilled in this art (*e.g*., Remington's Pharmaceutical Sciences, Mack Publishing Company, Easton, Pa., 15th Edition, 1975). The composition or formulation to be administered will, in any event, contain a quantity of the active agent in an amount sufficient to alleviate the symptoms of the treated subject.

The active agents or pharmaceutically acceptable derivatives may be prepared with carriers that protect the agent against rapid elimination from the body, such as time release formulations or coatings. The compositions may include other active agents to obtain desired combinations of properties.

Oral pharmaceutical dosage forms include, by way of example and without limitation, solid, gel and liquid. Solid dosage forms include tablets, capsules, granules, and bulk powders. Oral tablets include compressed, chewable lozenges and tablets which may be enteric-coated, sugar-coated or film-coated. Capsules may be hard or soft gelatin capsules, while granules and powders may be provided in non-effervescent or effervescent form with the combination of other ingredients known to those skilled in the art.

In certain embodiments, the formulations are solid dosage forms, such as capsules or tablets. The tablets, pills, capsules, troches and the like can contain any of the following ingredients, or agents of a similar nature: a binder; a diluent; a disintegrating agent; a lubricant; a glidant; a sweetening agent; and a flavoring agent.

Examples of binders include, by way of example and without limitation, microcrystalline cellulose, gum tragacanth, glucose solution, acacia mucilage, gelatin solution, sucrose, and starch paste. Lubricants include, by way of example and without limitation, talc, starch, magnesium or calcium stearate, lycopodium and stearic acid. Diluents include, by way of example and without limitation, lactose, sucrose, starch, kaolin, salt, mannitol, and dicalcium phosphate. Glidants include, by way of example and without limitation, colloidal silicon dioxide. Disintegrating agents include, by way of example and without limitation, crosscarmellose sodium, sodium starch glycolate, alginic acid, corn starch, potato starch, bentonite, methylcellulose, agar and carboxymethylcellulose. Coloring agents include, by way of example and without limitation, any of the approved certified water soluble FD and C dyes, mixtures thereof; and water insoluble FD and C dyes suspended on alumina hydrate. Sweetening agents include, by way of example and without limitation, sucrose, lactose, mannitol and artificial sweetening agents such as saccharin, and any number of spray dried flavors. Flavoring agents include, by way of example and without limitation, natural flavors extracted from plants such as fruits and synthetic blends of agents which produce a pleasant sensation, such as, but not limited to peppermint and methyl salicylate. Wetting agents include, by way of example and without limitation, propylene glycol monostearate, sorbitan monooleate, diethylene glycol monolaurate, and polyoxyethylene laural ether. Emetic-coatings include, by way of example and without limitation, fatty acids, fats, waxes, shellac, ammoniated shellac and cellulose acetate phthalates. Film coatings include, by way of example and without limitation, hydroxyethylcellulose, sodium carboxymethylcellulose, polyethylene glycol 4000 and cellulose acetate phthalate.

If oral administration is desired, the agent could be provided in a composition that protects it from the acidic environment of the stomach. For example, the composition can be formulated in an enteric coating that maintains its integrity in the stomach and releases the active agent in the intestine. The composition may also be formulated in combination with an antacid or other such ingredient.

When the dosage unit form is a capsule, it can contain, in addition to material of the above type, a liquid carrier such as fatty oil. In addition, dosage unit forms can contain various other materials which modify the physical form of the dosage unit, for example, coatings of sugar and other enteric agents. The agents can also be administered as a component of an elixir, suspension, syrup, wafer, sprinkle, chewing gum or the like. A syrup may contain, in addition to the active agents, sucrose as a sweetening agent and certain preservatives, dyes and colorings and flavors. The active materials can also be mixed with other active materials which do not impair the desired action, or with materials that supplement the desired action, such as antacids, H2 blockers, and diuretics.

Pharmaceutically acceptable carriers included in tablets are binders, lubricants, diluents, disintegrating agents, coloring agents, flavoring agents, and wetting agents. Enteric-coated tablets, because of the enteric-coating, resist the action of stomach acid and dissolve or disintegrate in the neutral or alkaline intestines. Sugar-coated tablets are compressed tablets to which different layers of pharmaceutically acceptable substances are applied. Film-coated tablets are compressed tablets which have been coated with a polymer or other suitable coating. Multiple compressed tablets are compressed tablets made by more than one compression cycle utilizing the pharmaceutically acceptable substances previously mentioned. Coloring agents may also be used in the above dosage forms. Flavoring and sweetening agents are used in compressed tablets, sugar-coated, multiple compressed and chewable tablets. Flavoring and sweetening agents are useful in the formation of chewable tablets and lozenges.

Liquid oral dosage forms include aqueous solutions, emulsions, suspensions, solutions and/or suspensions reconstituted from non-effervescent granules and effervescent preparations reconstituted from effervescent granules. Aqueous solutions include, for example, elixirs and syrups. Emulsions are either oil-in-water or water-in-oil.

Elixirs are clear, sweetened, hydroalcoholic preparations. Pharmaceutically acceptable carriers used in elixirs include solvents. Syrups are concentrated aqueous solutions of a sugar, for example, sucrose, and may contain a preservative. An emulsion is a two-phase system in which one liquid is dispersed in the form of small globules throughout another liquid. Pharmaceutically acceptable carriers used in emulsions are non-aqueous liquids, emulsifying agents and preservatives. Suspensions use pharmaceutically acceptable suspending agents and preservatives. Pharmaceutically acceptable substances used in non-effervescent granules, to be reconstituted into a liquid oral dosage form, include diluents, sweeteners and wetting agents. Pharmaceutically acceptable substances used in effervescent granules, to be reconstituted into a liquid oral dosage form, include organic acids and a source of carbon dioxide. Coloring and flavoring agents may be used in any of the above dosage forms.

Solvents include, by way of example and without limitation, glycerin, sorbitol, ethyl alcohol and syrup. Examples of preservatives include, without limitation, glycerin, methyl and propylparaben, benzoic acid, sodium benzoate and alcohol. Non-aqueous liquids utilized in emulsions include, by way of example and without limitation, mineral oil and cottonseed oil. Emulsifying agents include, by way of example and without limitation, gelatin, acacia, tragacanth, bentonite, and surfactants such as polyoxyethylene sorbitan monooleate. Suspending agents include, by way of example and without limitation, sodium carboxymethylcellulose, pectin, tragacanth, Veegum and acacia. Diluents include, by way of example and without limitation, lactose and sucrose. Sweetening agents include, by way of example and without limitation, sucrose, syrups, glycerin and artificial sweetening agents such as saccharin. Wetting agents include, by way of example and without limitation, propylene glycol monostearate, sorbitan monooleate, diethylene glycol monolaurate, and polyoxyethylene lauryl ether. Organic acids include, by way of example and without limitation, citric and tartaric acid. Sources of carbon dioxide include, by way of example and without limitation, sodium bicarbonate and sodium carbonate. Coloring agents include, by way of example and without limitation, any of the approved certified water soluble FD and C dyes, and mixtures thereof. Flavoring agents include, by way of example and without limitation, natural flavors extracted from plants, such as fruits, and synthetic blends of agents which produce a pleasant taste sensation.

For a solid dosage form, the solution or suspension, in for example propylene carbonate, vegetable oils or triglycerides, is encapsulated in a gelatin capsule. Such solutions, and the preparation and encapsulation thereof, are disclosed in US 4,328,245, US 4,409,239, and US 4,410,545. For a liquid dosage form, the solution (*e.g*., in a polyethylene glycol) may be diluted with a sufficient quantity of a pharmaceutically acceptable liquid carrier (*e.g*., water) to be easily measured for administration.

Alternatively, liquid or semi-solid oral formulations may be prepared by dissolving or dispersing the active agent or salt in vegetable oils, glycols, triglycerides, propylene glycol esters (e.g., propylene carbonate) and other such carriers, and encapsulating these solutions or suspensions in hard or soft gelatin capsule shells. Other useful formulations include those set forth in US RE28819 and US 4,358,603. Briefly, such formulations include, but are not limited to, those containing an agent provided herein, a dialkylated mono- or poly-alkylene glycol, including, but not limited to, 1,2-dimethoxymethane, diglyme, triglyme, tetraglyme, polyethylene glycol-350-dimethyl ether, polyethylene glycol-550-dimethyl ether, polyethylene glycol-750-dimethyl ether wherein 350, 550 and 750 refer to the approximate average molecular weight of the polyethylene glycol, and one or more antioxidants, such as butylated hydroxytoluene (BHT), butylated hydroxyanisole (BHA), propyl gallate, vitamin E, hydroquinone, hydroxycoumarins, ethanolamine, lecithin, cephalin, ascorbic acid, malic acid, sorbitol, phosphoric acid, thiodipropionic acid and its esters, and dithiocarbamates.

Other formulations include, but are not limited to, aqueous alcoholic solutions including a pharmaceutically acceptable acetal. Alcohols used in these formulations are any pharmaceutically acceptable water-miscible solvents having one or more hydroxyl groups, including, but not limited to, propylene glycol and ethanol. Acetals include, but are not limited to, di(lower alkyl) acetals of lower alkyl aldehydes, such as acetaldehyde diethyl acetal.

Tablets and capsules formulations may be coated as known by those of skill in the art in order to modify or sustain dissolution of the active ingredient. Thus, for example and without limitation, they may be coated with a conventional enterically digestible coating, such as phenylsalicylate, waxes and cellulose acetate phthalate.

Parenteral administration, generally characterized by injection, either subcutaneously, intramuscularly or intravenously, is also contemplated herein. Injectables can be prepared in conventional forms, either as liquid solutions or suspensions, solid forms suitable for solution or suspension in liquid prior to injection, or as emulsions. Suitable excipients include, by way of example and without limitation, water, saline, dextrose, glycerol or ethanol. In addition, if desired, the pharmaceutical compositions to be administered may also contain minor amounts of non-toxic auxiliary substances, such as wetting or emulsifying agents, pH buffering agents, stabilizers, solubility enhancers, and other such agents, such as, for example, sodium acetate, sorbitan monolaurate, triethanolamine oleate and cyclodextrins.

Implantation of a slow-release or sustained-release system, such that a constant level of dosage is maintained (*e.g.*, US 3,710,795) is also contemplated herein. Briefly, an inhibitor of Nt5e or AIR is dispersed in a solid inner matrix (*e.g*., polymethylmethacrylate, polybutylmethacrylate, plasticized or unplasticized polyvinylchloride, plasticized nylon, plasticized polyethyleneterephthalate, natural rubber, polyisoprene, polyisobutylene, polybutadiene, polyethylene, ethylene-vinylacetate copolymers, silicone rubbers, polydimethylsiloxanes, silicone carbonate copolymers, hydrophilic polymers such as hydrogels of esters of acrylic and methacrylic acid, collagen, cross-linked polyvinylalcohol and cross- linked partially hydrolyzed polyvinyl acetate) that is surrounded by an outer polymeric membrane (*e.g*., polyethylene, polypropylene, ethylene/propylene copolymers, ethylene/ethyl acrylate copolymers, ethylene/vinylacetate copolymers, silicone rubbers, polydimethyl siloxanes, neoprene rubber, chlorinated polyethylene, polyvinylchloride, vinylchloride copolymers with vinyl acetate, vinylidene chloride, ethylene and propylene, ionomer polyethylene terephthalate, butyl rubber epichlorohydrin rubbers, ethylene/vinyl alcohol copolymer, ethylene/vinyl acetate/vinyl alcohol terpolymer, and ethylene/vinyloxyethanol copolymer) that is insoluble in body fluids. The agent diffuses through the outer polymeric membrane in a release rate controlling step. The percentage of active agent contained in such parenteral compositions is highly dependent on the specific nature thereof, as well as the activity of the agent and the needs of the subject.

Lyophilized powders can be reconstituted for administration as solutions, emulsions, and other mixtures or formulated as solids or gels. The sterile, lyophilized powder is prepared by dissolving an agent provided herein, or a pharmaceutically acceptable derivative thereof, in a suitable solvent. The solvent may contain an excipient which improves the stability or other pharmacological component of the powder or reconstituted solution, prepared from the powder. Excipients that may be used include, but are not limited to, dextrose, sorbital, fructose, corn syrup, xylitol, glycerin, glucose, sucrose or other suitable agent. The solvent may also contain a buffer, such as citrate, sodium or potassium phosphate or other such buffer known to those of skill in the art at, typically, about neutral pH. Subsequent sterile filtration of the solution followed by lyophilization under standard conditions known to those of skill in the art provides the desired formulation. Generally, the resulting solution will be apportioned into vials for lyophilization. Each vial will contain, by way of example and without limitation, a single dosage (10-1000 mg, such as 100- 500 mg) or multiple dosages of the agent. The lyophilized powder can be stored under appropriate conditions, such as at about 4°C to room temperature. Reconstitution of this lyophilized powder with water for injection provides a formulation for use in parenteral administration. For reconstitution, about 1-50 mg, such as about 5-35 mg, for example, about 9-30 mg of lyophilized powder, is added per mL of sterile water or other suitable carrier. The precise amount depends upon the selected agent. Such amount can be empirically determined.

The inhibitors of GPR133 or pharmaceutically acceptable derivatives thereof may be formulated as aerosols for application *e.g.,* by inhalation or intranasally *(e.g.,* as described in US 4,044,126, 4,414,209, and 4,364,923). These formulations can be in the form of an aerosol or solution for a nebulizer, or as a microfine powder for insufflation, alone or in combination with an inert carrier such as lactose. In such a case, the particles of the formulation will, by way of example and without limitation, have diameters of less than about 50 microns, such as less than about 10 microns.

The agents may be also formulated for local or topical application, such as for application to the skin and mucous membranes (*e.g*., intranasally), in the form of nasal solutions, gels, creams, and lotions.

Other routes of administration, such as transdermal patches are also contemplated herein. Transdermal patches, including iontophoretic and electrophoretic devices, are well known to those of skill in the art. For example, such patches are disclosed in US 6,267,983, 6,261,595, 6,256,533, 6,167,301, 6,024,975, 6,010,715, 5,985,317, 5,983,134, 5,948,433, and 5,860,957.

In one embodiment, in order to enhance brain delivery of the inhibitor of GPR133, the patient is treated in a manner so as to increase the selective permeability of the blood-brain barrier (BBB). Treatments to selectively increase the permeability of the BBB in a patient include, but are not limited to, the administration of about 1 to about 1000 µg/kg body weight, preferably about 10 to about 100 µg/kg bodyweight, of IGF-I (*e.g.,* as a bolus injection to a patient about 0.5 to 10 hours, preferably about 1 hour, before the inhibitor administration).

Inhibitors of GPR133 for use in the present invention may be packaged as articles of manufacture containing packaging material and a label that indicates that the inhibitor is used for inhibiting the expression and/or function of GPR133 for treatment of a cancer.

### Reporter CHO-based Cell Lines for Use in the Invention

GPR133 inhibitors of the invention can be tested in a reporter Chinese hamster ovary (CHO)-based cell line. Three exemplary CHO cell lines that report GPR133 activation have been developed herein. All three cell lines inducibly express GPR133 on the basis of a tetracycline-on (tet-on) promoter. This design allows for induction of GPR133 expression after incubation with doxycycline, a tetracycline analog.

Based on the finding that GPR133 signaling is mediated by elevations in cytosolic cAMP and that cAMP activates transcription of genes with a cAMP response element (CRE) in their promoter, two of the three reporter cell lines utilize CRE-luciferase constructs to report GPR133 activation as quantifiable bioluminescence. The third reporter cell line utilizes ChAMPion system (Axxam Inc.) to report GPR133 activation via a Ca²⁺-activated fluorescent photoprotein in a manner agnostic to whether GPR133 is coupled to Gₛ (leading to cAMP elevation) or G_{q} (leading to Ca²⁺ elevation) proteins.

The defining features of the three exemplary reporter cell lines are: (i) inducible transcription of GPR133 long isoform 1 in CHO cells with a CRE-luciferase reporter; (ii) inducible transcription of truncated (short) GPR133 isoform 3 in CHO cells with a CRE-luciferase reporter; and (iii) inducible transcription of truncated (short) GPR133 isoform in CHO cells with a ChAMPion reporter.

The reporter cell lines utilize inducible long or truncated (short) isoforms of GPR133. The short isoform 3 lacks the vast majority of the N-terminal ectodomain of GPR133 but retains the Stachel sequence, which is believed to act as an endogenous agonist (Liebscher et al, Cell Reports, 2014).

The reason for pursuing reporter cell lines for both long and truncated isoforms of GPR133 is that they can facilitate discovery of different types of modulators of GPR133 activation (e.g., by small molecule drug screening for inhibitors; by screening of antibodies or other biologics for inhibitory effects; by screening tissue fractions, including extracellular matrix, for receptor activation, etc.). Long GPR133 isoforms, like other adhesion G protein-coupled receptors, contain an autoproteolytic cleavage site (GPS) in the N-terminal ectodomain, with the cleavage occurring due to the GAIN domain on the extracellular N-terminus. The cleavage produces an N-terminal fragment (NTF) and a C-terminal fragment (CTF). It is hypothesized that the NTF and CTF remain non-covalently bound, until a yet to be discovered ligand binds the N-terminal ectodomain and leads to dissociation of the N-terminal fragment from the C-terminal fragment. This dissociation then allows for conformational changes involving the Stachel sequence, leading to receptor activation (Figure 17). The truncated isoform 3 of GPR133, on the other hand, lack the NTF, so that the Stachel sequence is theoretically free of conformational restraints related to the NTF, leading to ligand-independent activation of the receptor.

Long GPR133 isoform reporter cell line can be used, for example, for ligand-binding domain mapping, ligand discovery and deorphanization, and allosteric inhibitor discovery. Truncated GPR133 isoform reporter cell can be used, for example, for antagonists of *Stachel* sequence binding and receptor activation.

### Mouse Models of Glioblastoma for Use in the Invention

GPR133 inhibitors of the invention can be tested in mouse models of GBM, which, include, but are not limited to, human GBM tumor xenografts, genetically modified mice, and mice injected with viruses expressing oncogenes.

In human GBM tumor xenografts, the brain of immunodeficient mice is injected with human GBM cells, which form a tumor xenograft. The advantage of this model is that the tumor consists of human GBM cells, which are genetically identical to actual patient tumors. The disadvantage of the model is that mice need to be immunosuppressed to develop tumor xenografts, thereby eliminating the immune system as a variable in testing GPR133 inhibitors.

In another specific embodiment, genetically modified mice are used for testing GPR133 inhibitors. In this model, a number of genetically modified mouse strains develop spontaneous GBM tumors. Common modifications are the deletion of tumor suppressor genes, which include, but are not limited to, p53 (see, e.g., Shchors et al., Proc Natl Acad Sci USA., 2013; 110(16):E1480-9), NF1 (see, e.g., Alcantara Llaguno et al., Cancer Cell., 2015; 28(4):429-40) and PTEN (see, e.g., Chen J, et al., Nature, 2012; 488(7412):522-6). The tumors are composed of oncogenically transformed mouse cells. A potential limitation of this model is that inhibitors of human GPR133 may not have an effect on mouse GPR133. The advantage of this system is that mice do not need to be immunosuppressed and the immune system is intact.

In yet another specific embodiment, mice are injected with viruses expressing oncogenes into select areas of the mouse brain, causing oncogenic transformation. An example of this model is the RCAS-tva system (Hambardzumyan et al., 2009, Transl. Oncol. 2009; 2:89-95). Often times, this approach is paired with germline deletion of a tumor suppressor gene. A potential limitation of this model is that inhibitors of human GPR133 may not have an effect on mouse GPR133. The advantage of this system is that mice do not need to be immunosuppressed and the immune system is intact.

### Diagnostic and Screening Methods of the Invention

In one embodiment, the disclosure relates to a method for determining whether a subject diagnosed with a cancer is at an increased risk for progression of said cancer and/or reduced survival comprising:
(a) determining an expression level of GPR133 in cancer cells of the subject,
(b) comparing the expression level of GPR133 determined in step (a) to a database's mean value of GPR133 mRNA expression in the same type of cancer, and
(c) determining that the subject is at an increased risk for cancer progression and/or reduced survival if the expression level of GPR133 in cancer cells of the subject is higher than the database's mean value of GPR133 mRNA expression in the same type of cancer.

In one specific embodiment, the database is NIH The Cancer Genome Atlas (TCGA) database (accessible, e.g., via https://tcga-data.nci.nih.gov/tcga/). One specific example of how the calculation of a mean GPR133 mRNA expression level in a tumor type using TCGA database can be done is provided below (see also, e.g., Cline et al., Scientific reports 3, 2652, doi: 10.1038/srep02652 (2013)):
1. Go to Cancer Browser (e.g., https://genome-cancer.ucsc.edu or https://tcga-data.nci.nih.gov/tcga/);
2. Select dataset with a given cancer's gene expression (RNA-seq data) (e.g., GBM gene expression (IlluminaHiSeq));
3. Enter GPR133 as the gene of interest;
4. Download GPR133 mRNA gene expression data annotated for sample type (e.g., normal tissue, primary tumor, recurrent tumor);
5. Analyze in Excel gene expression data among tumor samples.

In another embodiment, the disclosure relates to a method for determining whether a subject diagnosed with a cancer is at an increased risk for progression of said cancer and/or reduced survival comprising:
(a) determining an expression level of GPR133 in cancer cells of the subject,
(b) comparing the expression level of GPR133 determined in step (a) to GPR133 expression level in corresponding normal cells of the same tissue origin, and
(c) determining that the subject is at an increased risk for cancer progression and/or reduced survival if the expression level of GPR133 in cancer cells of the subject is higher than in the corresponding normal cells.

In a further embodiment, the provides a method of identifying a GPR133 inhibitor for treatment of a cancer, comprising contacting a candidate agent with a CD133-expressing (CD133+) cancer cell and determining expression level or a function of GPR133 in said cancer cell, wherein an inhibition of expression level or function of GPR133, as compared with said level or function prior to the agent exposure, indicates that said agent is a GPR133 inhibitor.

### EXAMPLES

The present invention is also described and demonstrated by way of the following examples.

### EXAMPLE 1: A novel signaling pathway that promotes self-renewal and tumorigenicity in CD133+ GSCs

Within any given human GBM tumor, there exist at least two distinct subtypes of gliolbastoma stem cells (GSCs). One of these GSC subtypes (referred to as Notch+) requires the Notch signaling pathway for self-renewal and tumorigenicity. Notch+ GSCs are metabolically dependent on oxidative phosphorylation and have the unique ability to differentiate to tumor-derived pericytes, thereby giving rise to large tumor vessels that generate a normoxic microenvironment and support their aerobic metabolism. A second GSC subtype (referred to CD133+), which is marked by cell surface expression of the CD133 glycoprotein and does not require the Notch pathway for self-renewal or tumorigenesis, has a restricted differentiation program and cannot generate pericytes. This restriction translates to poor tumor vascularity and hypoxic microenvironmental conditions. Remarkably though, CD133+ GSCs selectively employ a transcriptional network of hypoxia-regulated genes that allow them to utilize anaerobic glycolysis to grow in hypoxic conditions (Fig. 1).

The Notch signaling is required for the self-renewal of Notch+, but not CD133+, GSCs. To understand which signaling mechanisms subserve self-renewal and tumorigenicity in CD133+ GSCs, an RNA-seq comparison of the transcriptome in the two GSC subtypes was performed. We investigated the function of GPR133, a 7-transmembrane G protein-coupled receptor (GPCR) (Fig. 2A) not previously described in GBM was investigated, since the transcriptional data showed up to 8-fold enrichment of *GPR133* transcript in CD133+ cells.

In the investigated system described herein, it was found that *GPR133* transcript is significantly enriched in CD133+ GSCs (compared to CD133- cells) in all 3 primary GBM cultures tested (Fig. 2B). Using a commercially available antibody against the N-terminus of GPR133 (Lifespan Biosciencecs, Cat # LS-A2034, which recognizes the N-terminal ectodomain of GPR133), it was found that GPR133 expression in human GBM tumors is confined to cells (cell membrane staining) in areas of pseudopalisading necrosis (12/12 biospecimens tested) (Fig. 2Ci, ii), which are considered hypoxic (Fig. 2E). There was no GPR133 expression in normal brain (Fig. 2D), consistent with prior observations that *GPR133* transcript is confined to the basal ganglia and pituitary gland within the brain.

It was also found that *GPR133* is transcriptionally activated by hypoxia *in vitro* (Fig. 3A) and its enrichment in CD133+ GSCs correlates with enrichment of the hypoxia-regulated CA9 and *VEGFA* transcripts (Fig. 2B). Importantly, shRNA-mediated GPR133 knockdown reduces the amounts of *PROM1* (*CD133*) transcript, decreases the abundance of CD133+ cells and attenuates tumorsphere formation, under both normoxic and hypoxic conditions in primary GBM cultures *in vitro* (Fig. 3B-C). *In vivo,* GPR133 knockdown reduces tumor xenograft size and increases survival of transplanted mice (Fig. 4).

Previous observations in HEK293 and COS7 cells have indicated that GPR133 signaling is mediated by Gs-dependent activation of adenylate cyclase and subsequent elevation in intracellular cAMP. In certain studies using colorimetric assays for intracellular cAMP, it was found that cAMP is elevated in CD133+ cells compared to CD133- cells, and GPR133 knockdown reduces intracellular cAMP levels, which under control conditions, are significantly higher in CD133+ GSCs, suggesting that GPR133 signaling causes elevation in intracellular cAMP (Fig. 5A-5B). These findings, in conjunction with the observations demonstrating metabolic adaptations to hypoxia in CD133+ GSCs, suggest that GPR133 is essential to the self-renewal of CD133+ GSCs and their response to hypoxia via cAMP-mediated signaling cascades.

### EXAMPLE 2: GPR133 represents a novel therapeutic target in GBM

The finding that the cancer stem cell population in GBM is heterogeneous represents a paradigm shift in understanding the disease process and the way to design future treatment for GBM. As described above, GPR133 is an important regulator of the self-renewal of CD133+ GSCs, much like Notch signaling is important to the self-renewal and tumorigenicity of Notch+ GSCs. In particular, GPR133 is necessary for the self-renewal and growth of CD133+ GSCs under hypoxic conditions, as occurs in poorly vascularized areas of GBM, as supported by the following findings:
- GPR133 is specifically expressed within the CD133+ population in GBM, along with other hypoxia-driven genes;
- GPR133 is transcriptionally upregulated by hypoxia;
- GPR133 is selectively expressed in hypoxic areas of human GBM tumors;
- GPR133 knockdown reduces the abundance of CD133+ cells and *PROM1* (*CD133*) transcript *in vitro;*
- GPR133 knockdown decreases tumor growth in both normoxia and hypoxia *in vitro;*
- GPR133 knockdown decreases tumor growth *in vivo;*
- GPR133 signaling is mediated by elevations in intracellular cAMP.

Therefore, these findings suggest that within any given GBM tumor there exist at least two different types of GSCs. One subtype (Notch+) depends on Notch signaling for its self-renewal, and another one CD133+ GSCs depend on GPR133 and its downstream signaling via cAMP for their self-renewal and tumorigenic potential.

Inhibiting GPR133 function is therefore an attractive therapeutic approach in treating GBM, either alone or in combination with (a) Notch inhibitors or (b) other agents used in treating GBM. New small molecule and biologic *GPR133* inhibitors can be screened, tested, and developed in the preclinical animal models of GBM, as described above.

### EXAMPLE 3: GPR133 expression is upregulated in CD133+ GSCs

In order to identify novel genes that CD133+ GSCs require for tumorigenicity, an RNA-seq comparison of FACS-sorted CD133+ and CD133- cells from a primary human GBM culture developed from a surgical biospecimen (GBML8) (**Figure 7a**)⁴⁵ was performed. A total of 314 genes (266 upregulated and 48 downregulated in CD133+ cells; **Tables 1a,b**) were identified that were differentially expressed between the two cell groups (fold change cut-off: 1.5, FDR<0.05). GPR133, an orphan adhesion GPCR, which was among the top 20 genes overexpressed in CD133+ cells (**Figure 7a**) was chosen for further study. GPR133 has a long N-terminal ectodomain, which includes a concanavalin A domain, as well as an autoproteolytic GPS site and the endogenous *Stachel* sequence agonist (**Figure 7a**)⁴³**.** It was hypothesized that GPR133 and its downstream effectors might represent a critical signaling pathway regulating tumorigenicity of CD133+ GSCs.

First, the upregulation of GPR133 in the CD133+ population was confirmed, using flow cytometry (**Figure 7bi****,ii**) and qRT-PCR (**Figure 7c**) in 3 different primary patient samples (GBML8, GBML20 and GBML33; **Supplementary** **Figure 1**). Despite the variable percentage of CD133+ cells within these primary cultures (35.44 ± 19.52 %, n=3 cultures), we observed that GPR133 surface expression was enriched in CD133+ cells when compared to CD133- cells by flow cytometry (**Figure 7bi****,** **Figure 13a****-b**). Within the CD133+ population, 21.77 ± 11.72% of the cells were GPR133+, whereas in the CD133- population only 4.06 ± 1.48% of the cells expressed GPR133 on the cell surface cells (n=3 patient samples, p<0.04, t-test; **Figure 7bii**, **Figure 14a****-b**). Quantitation of mRNA levels with qRT-PCR revealed 16.30 ± 9.22- fold upregulation of *GPR133* mRNA levels in CD133+ cells, along with 17.43 ± 3.90-fold upregulation of *PROM1* (*CD133*) mRNA (n=3 cultures; **Figure 7c**)**.** When histological specimens from GBM patients were analyzed, it was observed that 9/9 tumors expressed GPR133 (**Figure 7di**). Under high magnification, the most intense immunostaining localized to the plasma membrane (**Figure 7dii**). No staining was observed in normal human brain specimens (**Figure 7diii**). The *in silico* analysis using GTex Portal⁴⁶ (http://www.gtexportal.org) also showed that *GPR133* expression is very low in the human brain (**Figure 14c**)¹⁷**.** These results suggest that GPR133 is selectively expressed within the CD133+ cell population of GBM and not in normal brain.

### EXAMPLE 4: GPR133 expression is upregulated in hypoxia in Hiflα-dependent manner

When the nine human GBM biospecimens were analyzed for GPR133 expression, it was observed that the staining localized in areas of PPN, which are believed to be hypoxic and stain positive for the hypoxia markers Hif1α and Carbonic Anhydrase 9 (CA9) (**Figure 8ai****-iv**)⁴⁸**.** Furthermore, xenograft tumors generated from FACS-sorted CD133+ human GBM cells revealed that GPR133 immunoreactivity overlaps with hydroxy-probe (pimonidazole) staining, which identifies hypoxic regions within poorly perfused regions of the tumor, as analyzed by intravenously injected Evans Blue (**Figure 8bi****-ii**) (n=3 animals). These findings suggest that *GPR133* expression is regulated by oxygen tension.

Previous reports suggested a hypoxic niche for CD133+ GBM cells^{22,23}. Furthermore, as revealed by RNA-seq experiments, upregulation of GPR133 correlates with the upregulation of CA9, a well-known target of Hif1α (**Figure 7a****, Table 1a**)**.** All of these data raise the possibility that GPR133 expression might be regulated by hypoxia, and specifically by Hif1α-mediated transcription. To test this hypothesis, human GBM cells were first subjected to *in vitro* hypoxia (1% O₂) for 24 hours. Irrespective of initial *GPR133* levels, five of five primary GBM cultures (**Figure 13**) showed upregulation of *GPR133* mRNA upon hypoxia (7.23 ± 2.88 -fold, P<0.008, Mann-Whitney) (**Figure 8c****,d**). *In silico* analysis of the genomic locus of *GPR133* revealed numerous hypoxia response elements (HRE; motif: CACGTG), including an HRE 571 bp upstream of the transcriptional start site, further supporting this hypothesis (**Figure 8e**). In order to test whether Hif1α levels affect the expression level of *GPR133,* Hif1α expression was knocked down in 2 primary GBM samples using short hairpin RNA (shRNA). This approach caused strong downregulation of Hif1α protein levels, as shown by Western blot (**Figure 8f**), and *HIF1A* mRNA (0.64 ± 0.14 and 0.29 ± 0.08 of control, in GBML8 and GBML20, respectively; t-test, P<0.002) (**Figure 8gi**). Interestingly, upon Hif1α knockdown, downregulation of *GPR133* mRNA was observed to 0.49 ± 0.12 and 0.40 ± 0.21 of control in GBML8 and GBML20, respectively (t-test, P<0.05, **Figure 8gii**). Although it remains to be explored whether *GPR133* is a direct transcriptional target of Hif1α, this finding suggests that *GPR133* transcription is regulated by hypoxia via Hif1α.

### EXAMPLE 5: GPR133 knockdown depletes CD133+ GSCs, impairs in vitro tumorsphere formation and reduces cellular proliferation.

*In silico* analysis with UniProt (http://www.uniprot.org) reveals that GPR133 might have multiple isoforms, which vary in the length of the large N-terminal extracellular domain. To investigate which isoform is relevant in GBM biology, qRT-PCR was performed on 5 primary GBM cultures using two different Taqman probes, one against the full N-terminal ectodomain and one against the C-terminal cytoplasmic domain of *GPR133.* The analysis revealed no significant difference between levels of mRNA amplified with either probe, suggesting that the full-length isoform is the predominant form of GPR133 in GBM (**Figure 16**).

These results demonstrate that *GPR133* is selectively expressed in CD133+ GSCs and upregulated by hypoxia in Hif1α-dependent manner. This hypoxia-dependent regulation of GPR133 expression suggests that signaling mediated by GPR133 could be critical for the self-renewal of CD133+ GSCs, which reside in hypoxic microenvironments of GBM^{22,23}. To test this hypothesis and thereby assess the functional role of GPR133 in regulating stem-like properties of CD133+ GSCs, shRNA-mediated knockdown of GPR133 was utilized in GBML20 and GBML8 (**Figure 9** and **Figure 16**, respectively). The knockdown construct used against GPR133 is designed to target the 3' end of the coding portion of the mRNA. As a validation of the knockdown efficiency, GPR133 expression levels were tested in cells lentivirally infected with the knockdown construct (GPR133-KD), compared to a scrambled shRNA control. A decrease in GPR133 expression was observed, as analyzed by flow cytometry in both primary lines tested (GBML20: 0.43 ± 0.13 of control, P<0.01, t-test; GBML8: 0.59 ± 0.06 of control, P<0.003, t-test) (**Figure 9a** and **Figure 16a**, respectively). Knockdown was further confirmed by quantitating *GPR133* mRNA levels with qRT-PCR (GBML20: 0.39 ± 0.20 of control, P<0.04, t-test; GBML8: 0.08 ± 0.06 of control, P<0.0001, t-test) (**Figure 9b** and **Figure 16b**, respectively).

To investigate the role of GPR133 in regulating CD133+ GSC, it was tested whether GPR133 knockdown can influence the percentage of CD133+ GSCs *in vitro.* In line with the hypothesis that GPR133 is critical for self-renewal of CD133+ GSCs, it was observed that GPR133 knockdown leads to significant decrease of CD133+ GSCs (GBML20: 0.47 ± 0.09 of control, P<0.004, t-test; GBML8: 0.43 ± 0.06 of control, P<0.0005, t-test) (**Figure 9c** and **Figure 16c**, respectively).

The proliferative capacity of GBM cells upon GPR133 knockdown was then tested. Compared to scrambled shRNA controls, it was observed that GPR133-KD GBM cells had reduced proliferation measured by Ki-67 immunofluorescence staining both under normoxic (GBML20: 0.67 ± 0.02 of control, P<0.002, t-test) and hypoxic (GBML20: 0.56 ± 0.10 of control, P<0.01, t-test) conditions (**Figure 9d****-e**). These results were also reproducible with GBML8 under both normoxic (0.71 ± 0.08 of control, P<0.02, t-test) and hypoxic (0.75 ± 0.09 of control, P<0.05, t-test) conditions (**Figure 16d****-e**).

In order to assess the role of GPR133 in regulating clonogenic tumor growth, a property ascribed to GSCs, *in vitro* tumorsphere formation assays were performed. When cells were seeded in low densities (4 cells/µL), it was observed that GPR133-KD reduced the number of spheres compared to scrambled shRNA-bearing controls under both normoxic (GBML20: p<0.006, t-test; GBML8: P<0.002, t-test) (**Figure 9fi** and **Figure 16fi****,** respectively) and hypoxic (GBML20: P<0.002, t-test; GBML8: P<0.002, t-test) (**Figure 9fii** and **Figure 16fii****,** respectively) conditions. These results suggest that GPR133 is required for the self-renewal of CD133+ GSCs and *in vitro* sphere formation.

To validate these GPR133 knockdown findings and rule out off-target effects of the shRNA used, some key experiments were repeated using a different shRNA construct, which targets the 5' coding region of the *GPR133* transcript encoding the full length N-terminal ectodomain (**Figure 17a**). Using this second shRNA construct in GBML20, downregulation in *GPR133* and *CD133* mRNA levels (*GPR133*: 0.47 ± 0.16 of control, P<0.02, t-test; *CD133:* 0.47 ± 0.16 of control, P<0.02, t-test) upon knockdown was observed (**Figure 17b****-c**). Diminished tumorsphere formation in both normoxic (P<0.04, t-test, **Figure 17d**) and hypoxic (P<0.01, t-test, **Figure 17e**) conditions was also observed.

### EXAMPLE 6: GPR133 acts via upregulation of cAMP

Previous literature on GPR133 suggested that it activates Gas and adenylyl cyclase, leading to accumulation of cAMP⁴⁴. To test whether GPR133 signaling in GBM is also mediated by cAMP, cAMP levels in GBM cells were first analyzed using a colorimetric assay. The assay demonstrated a 20.77 ± 8.89% downregulation of cAMP upon GPR133 knockdown (P<0.05, t-test) (**Figure 10a**), suggesting that canonical GPR133 signaling in GBM is indeed mediated by cAMP.

We then used an activator of adenylyl cyclase, forskolin (10 µM), to test whether it can rescue the effects of GPR133 knockdown. We observed that forskolin restored the impairment in tumorsphere formation under hypoxic conditions in 2 primary GBM cultures (GBML8 and GBML20, P<0.03, t-test, **Figure 10b**). These results argue that GPR133's action during the hypoxic response is mediated by cAMP and is critical for tumorsphere formation *in vitro.*

### EXAMPLE 7: GPR133 knockdown impairs tumorigenicity

The *in vitro* data suggest that GPR133 is critical for proliferation and GSC sphere formation. To confirm this hypothesis, the *in vivo* tumorigenicity of GBM cells after GPR133 knockdown was tested. GBML20 cells that were transduced with either scrambled or GPR133-KD shRNA constructs were injected into the brains of NOD.SCID mice (5x10⁵ cells/animal, n=4 animals/group). MRI analysis and volumetric tumor analysis with AMIRA software 1 month after injections revealed that control cells were able to form large tumors, as opposed to GPR133-KD cells, which showed impaired tumor formation (**Figure 11a****,b**)**.** All control animals expired within 4 months after implantation, whereas GPR133-KD animals survived without any clinical deterioration. Histology at the time of death for scramble mice, or sacrifice for GPR133-KD mice, showed a dramatic difference in tumor volume (**Figure 11c**)**.** Expansive and brain-infiltrating tumor formation were observed in mice injected with scramble control GBM cells (n=4 animals) (**Figure 11c****, upper panel**). In contrast, only a few scattered human cells were found in animals injected with GPR133-KD cells, identified by human nuclear antigen staining (hNA) at the site of injection (n=4 animals) (**Figure 11c****, lower panel**).

Cumulatively, quantitation of tumor volumes from the MRI images showed that scramble controls gave rise to significantly larger tumors, compared to GPR133-KD cells (P<0.002, t-test, n=4 animals/group (**Figure 11d**)**.** Furthermore, GPR133-KD led to a drastic survival difference compared to animals injected with GBM cells bearing scrambled shRNA (P<0.01, **Figure 11e**). These results show that GPR133 is critical for tumorigenicity.

### EXAMPLE 8: GPR133 expression correlates with poor prognosis in GBM

In order to understand the clinical relevance of these findings, the UCSC Cancer Genome Browser was used to analyze existing RNA-seq data from 160 GBM samples in the TCGA database⁴⁹. After ranking the samples according to expression levels of *GPR133,* the samples were divided into two groups: GPR133 lo (percentile 1-50, light gray) and GPR133 hi (percentile 51-100, dark gray) (**Figure 12a**). Then, the survival of these two groups was analyzed using Kaplan-Meier survival curves. It was found that, in agreement with the mouse data, high GPR133 expression correlates with poor prognosis and reduced survival (median survival 360 days vs. 485 days, P=0.0062) (**Figure 12b**). These data confirm that GPR133 has pro-tumorigenic function and suggest that its inhibition represents an attractive and novel therapeutic approach.

### EXAMPLE 9: GPR133 expression correlates with poor prognosis in other cancers

Similar effects of GPR133 expression levels on patient survival in other cancer types, using the same analysis of TCGA data (accessible, e.g., via https://tcga-data.nci.nih.gov/tcga/). The cancer types include: breast adenocarcinoma, colorectal adenocarcinoma and lung squamous cell carcinoma. These findings suggest that GPR133 inhibition may be clinically beneficial in other malignancies, as well.

### DISCUSSION

Understanding the molecular mechanisms that underlie the response of cancer cells to hypoxia is critical for the discovery of new therapeutic targets, especially in tumors such as GBM, which exhibits extensive hypoxia. In GBM, hypoxia and its downstream cellular responses, such as increased stem-like phenotypes and angiogenesis, are associated with tumor progression⁵⁰. The idea that areas of PPN represent a hypoxic niche for CD133+ GSCs has been supported by previous literature^{22,23}. However, the key molecular events that regulate GSCs' response to hypoxia remain unclear. The above data show that GPR133, an orphan adhesion GPCR, is enriched in PPN *in vivo* and CD133+ GSCs *in vitro,* regulated transcriptionally by oxygen tension and necessary for tumor growth. It is possible that GPR133 represents an important component of GBM's hypoxic response and plays an essential pro-tumorigenic role.

Besides limited knowledge on the activation of the protein by the *Stachel* sequence⁴³ and downstream signaling via cAMP⁴⁴, the biology of GPR133 is overall poorly understood. Evidence for a role of GPR133 in normal cellular biology or disease processes has been lacking. In addition, relative contributions of intracellular signaling via G proteins vs. cell-to-cell signaling via the N-terminal ectodomain have not been determined.

The data herein is the first demonstration of GPR133's role in oncogenesis and provides insights into possible cellular functions. GPR133 acts as a crucial regulator of tumor growth. In GBM, hypoxia induces upregulation of *GPR133* mRNA via a mechanism that requires Hif1α. This regulatory mechanism is consistent with GPR133's localization in PPN, which are also enriched for Hif1α and its transcriptional target CA9, a known marker of hypoxia⁴⁸. The descrived tumor xenografts models also validate the predilection of GPR133 for hypoxic tumor microenvironments, as evidenced by its co-localization with pimonidazole. Given that *GPR133* gene bears numerous HREs, and that Hif1α knockdown downregulates *GPR133* transcription, it is hypothesized that *GPR133* is a direct target of Hif1α and a novel component of the hypoxia-induced transcriptional response.

The above-described experiments provide a strong indication that GPR133 expression is required for tumor growth. Using lentiviral shRNA constructs, it was found that reduced GPR133 expression decreases the number of CD133+ GSCs, tumorsphere formation and cellular proliferation *in vitro.* Notably, GPR133 knockdown abolished tumor formation *in vivo* and prevented death of mouse hosts. In agreement with the above studies in mice, analysis of TCGA data shows that increased expression in human GBM correlates with worse/poor survival. These findings indicate a crucial role for GPR133 in GBM growth and suggest that it represents an appealing therapeutic target. Further experiments will be required to test whether genetic inhibition of GPR133 causes regression of tumors that have already formed.

Furthermore, these findings suggest that GPR133 signaling in GBM is mediated by elevations in cAMP. GPR133 knockdown reduces cAMP levels in GBM cells *in vitro.* Forskolin, which activates adenylate cyclase and raises cAMP levels, rescues the knockdown phenotype *in vitro.* These data are in agreement with previous observations in HEK293 and Cos-7 cells, which suggested coupling of GPR133 to cAMP production⁴⁴. The issue of how cAMP signaling regulates tumor growth in GBM has been relatively understudied and equivocal in prior literature⁵¹⁻⁵³. Further experiments will be needed to elucidate the downstream effects of GPR133 on GBM's gene expression, signaling and metabolism.

In summary, the above data show that GPR133 plays an essential pro-tumorigenic role in GBM, especially within hypoxic microenvironments. Despite current gaps in knowledge about GPR133's signaling pathway and activation mechanisms, it is intriguing to consider that GPR133 may act as a sensor of hypoxia in the tumor microenvironment, responsible for translating extracellular stimuli into a specific cellular response. Given GPR133's important role in tumorigenesis in GBM, its extracellular localization and the general 'druggability' of GPCRs, it is proposed that GPR133 represents an enticing novel therapeutic target in GBM and in other cancers In addition, inhibition of GPR133 may be used to target the hypoxic response that is triggered by, and likely accounts for the failure of, anti-angiogenic therapy in GBM. Finally, the above-described results may be applicable to other malignancies with similar responses to hypoxia.

### Materials and Methods

### Primary Tumor Cultures

Fresh tumor tissue was procured from patients undergoing surgery for resection of GBM after informed consent (IRB protocol S12-01130). Primary human GBM cultures were obtained as previously described^{45,54,55}. Tumorsphere cultures were maintained in suspension on non-adherent plates in Neurobasal media, supplemented with N2, B27 (without vitamin A), 20 ng/mL of human recombinant EGF and 20 ng/ml FGF2 (Life Technologies and R&D Systems)⁵⁴. Molecular subtyping of parental tumors was performed with DNA methylation analysis⁵⁶. DNA was extracted from formalin-fixed paraffin embedded tissue and analyzed using Infinium 450k DNA methylation array. Tumors were classified according to their methylation profile⁵⁶. **Figure 13** shows the copy number variation profiles and key genomic changes in parental tumors for the 5 primary cultures used.

### Flow Cytometry

Cells were dissociated with Accutase (Stem Cell Technologies). CD133 staining was performed with fluorophore-conjugated AC133 antibody (Miltenyi), which recognizes the CD133/1 epitope. GPR133 flow cytometric analysis was performed using a GPR133 antibody that detects the extracellular domain (LSBiosciences). Goat anti-rabbit Alexa488 conjugated secondary antibody was used for detection (Life Technologies). The LSRII analyzer and FACSAria cell sorter (BD Biosciences) were used for flow cytometry and fluorescence-assisted cell sorting (FACS) experiments, respectively.

### RNA isolation, Library Preparation, RNA-sequencing and Bioinformatics

RNA was isolated from 30,000 FACS-isolated GBM cells using mirRNeasy Micro RNA isolation kit (Qiagen). RNA-Seq libraries were prepared using the Epicentre (Illumina) TotalScript RNA-Seq kit, starting from 5 ng of DNAse I-treated total RNA, and using oligo-(dT) as the primer for cDNA synthesis. The libraries were pooled equimolarly and run on a HiSeq 2500 sequencing system (Illumina), as paired 50 nucleotide reads. Sequencing results were de-multiplexed and converted to FASTQ format using Illumina Bcl2FastQ software. Paired-end reads were aligned to the human genome (build hg19/GRCh37) using the splice-aware STAR aligner⁵⁷. PCR duplicates were removed using the Picard toolkit (http://broadinstitute.github.io/picard). HTSeq package was utilized to generate counts for each gene based on how many aligned reads overlap its exons⁵⁸. These counts were then used to test for differential expression using negative binomial generalized linear models implemented by the DESeq2 R package⁵⁹.

### qRT-PCR Expression Analysis

Lysates and cDNA were prepared from 10,000 FACS-isolated cells using TaqMan Gene expression Cells-to-Ct kit using the manufacturer's protocol (Ambion, Life Technologies). Taqman gene expression probes against *PROM1, GPR133,* and *HIF1A* (Ambion, Life Technologies) were used to analyze changes in gene expression with qRT-PCR (Applied Biosystems, StepOne Real-time PCR System). Fold changes in expression were calculated using the ΔΔCt method. The *HPRT1* and *GAPDH* genes were used to normalize results. **Table 2** summarizes the Taqman assays used in this study.

### Animals and Stereotactic Injections into Mouse Brain

Mice were housed within NYU Langone Medical Center's Animal Facilities. All procedures were performed according to our IACUC-approved protocol (IACUC# 120310-03), as previously described⁴⁵. Briefly, 6-8 week old NOD.SCID (Jackson Labs) male mice were stereotactically injected with 5 µl of a suspension of human GBM cells (100,000 cells/µl) in the frontal lobe (2 mm off the midline and 2 mm anterior to coronal suture).

### Small Animal MRI

Tumor formation was analyzed 1 month after injection of tumor cells into the brains of NOD.SCID mice. MRIs were performed as previously described⁴⁰. Stacked images were processed using ImageJ software. Tumor volumes were calculated with Amira Software (FEI Software).

### Pimonidazole and Evans Blue injections for analyzing hypoxia and perfusion in vivo

Animals were injected i.p. with 60 mg/kg pimonidazole (Hydroxy-Probe) 90 minutes prior to sacrifice. 2% Evans blue (EB) was injected at 6 µL/g of body weight. Animals were sacrificed 5 minutes later⁶¹. Brains were collected directly into 4% paraformaldehyle with 0.05% glutaraldehyde. Brains were mounted in OCT (Tissue-Tek) and sectioned frozen as described below.

### Immunofluorescence Staining and Microscopic Analysis

When the experimental end-point was reached, animals were anesthetized with Ketamine/Xylazine (10 mg/kg and 100 mg/kg, respectively) and systemically perfused with first Phosphate-buffered Saline (PBS) and then 4% paraformaldehyde. Isolated brain tissue was mounted in OCT (Tissue-Tek) and 30 µm-thick frozen sections were obtained using a cryostat (Leica). Sections were blocked with 10% (w/v) BSA (Sigma) and 0.1% Triton X-100 (Sigma) in PBS for 2 hours at room temperature. The primary antibodies and the dilutions used for immunostaining are summarized in **Table 3.** Staining was performed in blocking solution for 18 hours at 4°C. Alexa488, Alexa555 and Alexa647 - conjugated secondary antibodies were used for fluorescent labeling (Life Technologies). Nuclear chromatin was counterstained with DAPI (Sigma). Pimonidazole staining was performed according to the manufacturer's instructions (Hypoxyprobe). Epifluorescence microscopy was performed on an Eclipse E800 fluorescent microscope (Nikon). For confocal imaging, 30 µm z-stacks were obtained with an LSM700 confocal microscope (Zeiss). Image analyses were performed on ImageJ and Adobe Photoshop.

### 24-Hour Hypoxia Treatment

Cells were treated with hypoxic gas mixture (1% O₂, 5%CO₂, balanced with N₂) for 24 hours at 37°C using a hypoxia chamber (Stem Cell Technologies).

### Plasmids and Constructs

Short hairpin RNA (shRNA) constructs against *GPR133* and *HIF1A* were cloned into the EcoRI and Agel sites of pLK0.1_puro (*GPR133* C-terminal: 5'-CCTGCAGGGACTGTTCATATT-3' (SEQ ID NO: 7), *GPR133* N-terminal: 5'-GGAGTCACGCTTCTCTATTAC-3' (SEQ ID NO: 6), and *HIF1A* 5'-GCCGCTCAATTTATGAATATT-3')²⁹ (SEQ ID NO: 24). A scrambled shRNA sequence (5'-CCTAAGGTTAAGTCGCCCTCG -3') (SEQ ID NO: 14) was used as a control.

### Lentivirus Production

Lentiviruses were generated in Lenti-X 293 HEK (Clontech) producer cells, concentrated and titered with qPCR-based assays (ABM), as previously described⁴⁵.

### Viral Transduction

Primary GBM tumorsphere cultures were dissociated with Accutase (Innovative Cell Technologies). Cells (300 cells/µl) were incubated at 37°C overnight with lentivirus in protamine sulfate (4 µg/mL) at a multiplicity of infection (MOI) of 5. Three days after transduction, transduced cells were selected with 1 µg/mL puromycin (Life Technologies) for 1 week. Some of the experiments with HIF1A-KD were performed 3 days after transduction without selection.

### Western Blotting

GBM cells were lysed in Lysis Buffer (150 mM NaCl, 50 mM Tris pH 7.4, 1 mM EDTA, 0.1% Triton-X100, 10% glycerol) supplemented with complete protease inhibitor cocktail (Roche). Protein extracts were quantified with Bradford assay. Proteins were separated by SDS-PAGE and transferred to nitrocellulose membranes (Biorad). Membranes were probed with anti-Hif1α (Bethyl Laboratories) and anti-β-Actin (Santa Cruz Biotechnology) primary antibodies. Signal was detected with appropriate HRP-conjugated secondary antibodies (Life Technologies) suited for chemiluminescence (Thermo Scientific).

### cAMP Measurements

cAMP levels were measured using cAMP-Glo assay (Promega). The manufacturer's protocol was followed.

### Statistical Analysis

Statistical comparisons included Student's two-tailed t-test and Mann-Whitney test. Statistical significance cutoff was set at P<0.05. Prism (GraphPad) and SPSS software (IBM) were used for statistical analyses. Population statistics were represented as mean ± standard error (SE) of the mean.

### TCGA analysis

The UCSC Cancer Browser (https://genome-cancer.ucsc.edu) was used for analysis of TCGA information on 160 GBM patients with available RNA-seq data. After ranking patients by *GPR133* expression levels, we classified them into two groups, each with 80 patients: GPR133 lo (percentile 1-50) and GPR133 hi (percentile 51-100). Survival data on these patients were then exported to Prism for generation of Kaplan-Meier curves and statistical analysis. Survival comparison was performed with logrank (Manterl-Cox) test.

**Table 1a. Genes upregulated in CD133+ vs. CD133- GBM cells.**

| **Gene** | **log2 change** | **fold change** | **FDR-adjusted p value** |
|---|---|---|---|
| *PROM1* | 4.02359 | 16.26377211 | 0.004 |
| *ACAN* | 3.77966 | 13.73380995 | 0.004 |
| *IGSF21* | 3.6893 | 12.90000751 | 0.004 |
| *ALPK2* | 3.50818 | 11.37803879 | 0.025 |
| *FAM180A* | 3.4572 | 10.98299791 | 0.004 |
| *SORCS3* | 3.37402 | 10.36767145 | 0.004 |
| *AN01* | 3.17698 | 9.044119158 | 0.004 |
| *NPTX1* | 3.15352 | 8.898239933 | 0.004 |
| *ESPN* | 3.15049 | 8.879571141 | 0.016 |
| *CA9* | 3.07609 | 8.433257464 | 0.004 |
| *GPR133* | 3.0061 | 8.033897194 | 0.004 |
| *FAM150B* | 2.98885 | 7.938409582 | 0.004 |
| *SPNS2* | 2.94832 | 7.718497304 | 0.004 |
| *DHRS3* | 2.92511 | 7.595316059 | 0.047 |
| *SLC6A6* | 2.89976 | 7.463022316 | 0.004 |
| *NRXN3* | 2.82332 | 7.077893236 | 0.004 |
| *EDN2* | 2.67708 | 6.39560128 | 0.007 |
| *ADCY8* | 2.56274 | 5.908287377 | 0.004 |
| *RHBDF2* | 2.55968 | 5.895769001 | 0.004 |
| *PCDH8* | 2.46046 | 5.503921906 | 0.004 |
| *SHANK2* | 2.45849 | 5.49641143 | 0.004 |
| *GRIP2* | 2.30228 | 4.932366489 | 0.004 |
| *CHI3L1* | 2.25978 | 4.789184449 | 0.045 |
| *GMPR* | 2.24373 | 4.736199986 | 0.004 |
| *HAPLN3* | 2.21446 | 4.641078179 | 0.004 |
| *SORCS2* | 2.17459 | 4.514574438 | 0.004 |
| *LOC154761* | 2.11182 | 4.322362289 | 0.004 |
| *TPPP3* | 2.10823 | 4.3116199 | 0.004 |
| *SPAG4* | 2.10036 | 4.288163755 | 0.007 |
| *COL15A1* | 2.09563 | 4.274127662 | 0.004 |
| *LOXL2* | 2.08287 | 4.236491575 | 0.004 |
| *SLC14A1* | 2.08245 | 4.235258419 | 0.004 |
| *CAV1* | 2.05299 | 4.149650983 | 0.004 |
| *CDH5* | 2.05091 | 4.14367255 | 0.004 |
| *PRSS35* | 2.04238 | 4.119245183 | 0.036 |
| *MMP17* | 2.03999 | 4.112426801 | 0.004 |
| *HIC1* | 2.0392 | 4.110175509 | 0.023 |
| *MAP7D2* | 2.02911 | 4.081529826 | 0.004 |
| *LMO1* | 2.0216 | 4.060338483 | 0.004 |
| *SMAD7* | 2.00634 | 4.017616893 | 0.004 |
| *ESRRB* | 1.999 | 3.997228372 | 0.016 |
| *MT1E* | 1.99553 | 3.987625708 | 0.018 |
| *C1QTNF5* | 1.99517 | 3.986630788 | 0.007 |
| *OLFM1* | 1.98693 | 3.963925918 | 0.004 |
| *TNFAIP2* | 1.98364 | 3.954896665 | 0.048 |
| *EDN1* | 1.98353 | 3.954595131 | 0.011 |
| *LOC284578* | 1.98208 | 3.950622509 | 0.007 |
| *GABRG3* | 1.98182 | 3.949910599 | 0.018 |
| *NELL2* | 1.97029 | 3.918468773 | 0.004 |
| *FAM50B* | 1.95733 | 3.883426067 | 0.034 |
| *OLFML2A* | 1.9427 | 3.844244242 | 0.004 |
| *AQP1* | 1.93211 | 3.816129156 | 0.023 |
| *H1F0* | 1.92254 | 3.790898948 | 0.004 |
| *NDRG1* | 1.91194 | 3.763147922 | 0.004 |
| *APLN* | 1.8974 | 3.72541204 | 0.004 |
| *ELFN1* | 1.89274 | 3.713398127 | 0.013 |
| *WNT7A* | 1.86552 | 3.643992526 | 0.004 |
| *STC1* | 1.84502 | 3.592579295 | 0.004 |
| *NXPH4* | 1.84486 | 3.592180887 | 0.004 |
| *CDH7* | 1.84446 | 3.591185061 | 0.018 |
| *MY01B* | 1.83421 | 3.565760981 | 0.004 |
| *OGDHL* | 1.81885 | 3.527998628 | 0.004 |
| *MAP3K5* | 1.80557 | 3.49567243 | 0.004 |
| *SNCB* | 1.78044 | 3.435309303 | 0.004 |
| *DUSP26* | 1.7681 | 3.406050912 | 0.039 |
| *SERPINF1* | 1.75522 | 3.375777926 | 0.007 |
| *OPCML* | 1.74628 | 3.354923802 | 0.004 |
| *PCSK1* | 1.7144 | 3.281601372 | 0.004 |
| *KCNK3* | 1.71425 | 3.281260195 | 0.018 |
| *SELM* | 1.71122 | 3.274376006 | 0.025 |
| *DDIT4L* | 1.69559 | 3.239093245 | 0.004 |
| *AMPD3* | 1.67555 | 3.194411122 | 0.004 |
| *INHBB* | 1.67535 | 3.193968313 | 0.004 |
| *CEBPD* | 1.67203 | 3.186626648 | 0.004 |
| *CDK18* | 1.66736 | 3.176328224 | 0.004 |
| *ADM* | 1.65296 | 3.144781975 | 0.004 |
| *WNT7B* | 1.64147 | 3.119835582 | 0.004 |
| *CYB5R2* | 1.63011 | 3.095365988 | 0.023 |
| *GALNTL4* | 1.625 | 3.084421651 | 0.004 |
| *ADAMTS7* | 1.6153 | 3.063753017 | 0.013 |
| *LGALS3* | 1.61346 | 3.059848026 | 0.004 |
| *SIK1* | 1.60679 | 3.045734107 | 0.004 |
| *FOSL2* | 1.60012 | 3.031685291 | 0.004 |
| *CAPG* | 1.5931 | 3.016969266 | 0.004 |
| *SGIP1* | 1.58736 | 3.004989604 | 0.004 |
| *NRP1* | 1.57982 | 2.989325506 | 0.004 |
| *ACCS* | 1.57952 | 2.988703958 | 0.021 |
| *MRVI1* | 1.5783 | 2.98617766 | 0.004 |
| *KBTBD11* | 1.57501 | 2.979375578 | 0.004 |
| *SLC16A3* | 1.573 | 2.975227525 | 0.004 |
| *CRABP2* | 1.5722 | 2.973578166 | 0.004 |
| *HSF4* | 1.5596 | 2.947721039 | 0.004 |
| *AKAP12* | 1.55173 | 2.931684799 | 0.004 |
| *COL23A1* | 1.54094 | 2.909840348 | 0.032 |
| *HSD11B2* | 1.54088 | 2.909719334 | 0.039 |
| *CNGA3* | 1.53308 | 2.894030246 | 0.013 |
| *BAMBI* | 1.52091 | 2.869720041 | 0.039 |
| *ALDOC* | 1.51809 | 2.86411615 | 0.004 |
| *VWA1* | 1.47682 | 2.783345494 | 0.011 |
| *EGLN3* | 1.47663 | 2.782978957 | 0.004 |
| *CHST15* | 1.47479 | 2.779431834 | 0.021 |
| *MFSD4* | 1.47137 | 2.772850818 | 0.007 |
| *PYGL* | 1.47036 | 2.770910283 | 0.004 |
| *PDZD2* | 1.46249 | 2.755835932 | 0.004 |
| *IL17RC* | 1.46152 | 2.753983661 | 0.045 |
| *ITPR1* | 1.44994 | 2.731966892 | 0.004 |
| *CLIC6* | 1.43228 | 2.69872879 | 0.038 |
| *RNF182* | 1.42411 | 2.683489048 | 0.004 |
| *GSN* | 1.41736 | 2.670963017 | 0.004 |
| *EFR3B* | 1.41381 | 2.664398731 | 0.004 |
| *ADSSL1* | 1.40184 | 2.642383745 | 0.004 |
| *INSIG2* | 1.39879 | 2.636803386 | 0.004 |
| *S1PR3* | 1.39323 | 2.62666097 | 0.004 |
| *TCEAL5* | 1.39179 | 2.624040524 | 0.007 |
| *CA12* | 1.38766 | 2.61653943 | 0.004 |
| *EPB49* | 1.36479 | 2.575388356 | 0.034 |
| *MLLT4-AS1* | 1.36347 | 2.573033071 | 0.021 |
| *FAM69C* | 1.36208 | 2.570555213 | 0.021 |
| *TF* | 1.34861 | 2.546666424 | 0.030 |
| *BHLHE40* | 1.34337 | 2.537433477 | 0.004 |
| *PPFIA4* | 1.34201 | 2.535042616 | 0.004 |
| *FAM110C* | 1.3388 | 2.52940841 | 0.047 |
| *BEST3* | 1.33022 | 2.514410148 | 0.004 |
| *MIAT* | 1.31799 | 2.493185107 | 0.004 |
| *NT5E* | 1.3026 | 2.466730322 | 0.004 |
| *FAM115C* | 1.29649 | 2.456305491 | 0.004 |
| *FGF11* | 1.2949 | 2.453599878 | 0.038 |
| *OBSL1* | 1.29456 | 2.453021706 | 0.004 |
| *ELL2* | 1.28918 | 2.4438911 | 0.004 |
| *TMEM123* | 1.2846 | 2.436144991 | 0.004 |
| *ZNF395* | 1.28442 | 2.435841061 | 0.004 |
| *PCOLCE2* | 1.28285 | 2.43319172 | 0.007 |
| *ID2* | 1.27942 | 2.427413691 | 0.004 |
| *SLC2A1* | 1.2714 | 2.413957038 | 0.004 |
| *STAC2* | 1.26583 | 2.404655129 | 0.004 |
| *LOC100216479* | 1.26444 | 2.40233942 | 0.004 |
| *GPR12* | 1.26042 | 2.395654735 | 0.004 |
| *PFKFB4* | 1.24961 | 2.377771366 | 0.004 |
| *HSPB6* | 1.24708 | 2.373605212 | 0.004 |
| *RCOR2* | 1.2457 | 2.371335842 | 0.004 |
| *MAF* | 1.24142 | 2.364311296 | 0.004 |
| *GATM* | 1.23682 | 2.35678475 | 0.004 |
| *ZNF334* | 1.23033 | 2.346206505 | 0.023 |
| *STC2* | 1.21213 | 2.316794367 | 0.004 |
| *NTRK2* | 1.20587 | 2.30676334 | 0.004 |
| *SLC43A3* | 1.20298 | 2.302147068 | 0.004 |
| *EHD2* | 1.19712 | 2.292815075 | 0.004 |
| *SH2D4A* | 1.19615 | 2.291274012 | 0.016 |
| *IGFBP5* | 1.19567 | 2.290511808 | 0.004 |
| *GYPC* | 1.19441 | 2.288512227 | 0.007 |
| *FAM49A* | 1.19233 | 2.285215151 | 0.004 |
| *MGST1* | 1.18968 | 2.281021429 | 0.045 |
| *HILPDA* | 1.18768 | 2.277861453 | 0.004 |
| *PTPRD* | 1.17923 | 2.2645588 | 0.004 |
| *SEMA5B* | 1.17591 | 2.259353479 | 0.016 |
| *SLC2A3* | 1.16547 | 2.24306278 | 0.004 |
| *LMO2* | 1.16336 | 2.239784608 | 0.018 |
| *NRN1* | 1.14803 | 2.216110778 | 0.004 |
| *YWHAH* | 1.13928 | 2.202710659 | 0.004 |
| *EMP2* | 1.13889 | 2.202115287 | 0.004 |
| *GABBR2* | 1.13096 | 2.19004422 | 0.004 |
| *ARHGEF3* | 1.12941 | 2.187692548 | 0.004 |
| *CNR1* | 1.12917 | 2.187328644 | 0.004 |
| *SFXN3* | 1.12198 | 2.176454711 | 0.007 |
| *KIAA1671* | 1.11606 | 2.167542077 | 0.004 |
| *TSC22D3* | 1.11344 | 2.163609294 | 0.004 |
| *MAFB* | 1.11026 | 2.158845501 | 0.034 |
| *EFS* | 1.10827 | 2.155869723 | 0.004 |
| *CD9* | 1.10732 | 2.154450572 | 0.004 |
| *PSTPIP2* | 1.09879 | 2.141749869 | 0.045 |
| *LOC254559* | 1.09859 | 2.14145298 | 0.030 |
| *PPP1R3C* | 1.0874 | 2.124907441 | 0.004 |
| *ST3GAL1* | 1.08427 | 2.120302344 | 0.004 |
| *PRPH* | 1.08074 | 2.115120709 | 0.043 |
| *FABP5* | 1.07707 | 2.109746997 | 0.004 |
| *PLOD2* | 1.06946 | 2.098647697 | 0.004 |
| *RARA* | 1.05355 | 2.075631017 | 0.030 |
| *TRIM2* | 1.04904 | 2.069152531 | 0.004 |
| *MED15* | 1.04897 | 2.069052138 | 0.004 |
| *CD97* | 1.04399 | 2.061922345 | 0.004 |
| *FKBP9L* | 1.03953 | 2.055557886 | 0.036 |
| *HK2* | 1.03389 | 2.047537678 | 0.004 |
| *PDK1* | 1.03177 | 2.044531088 | 0.004 |
| *NFATC4* | 1.02656 | 2.037160995 | 0.013 |
| *TET1* | 1.02306 | 2.032224802 | 0.011 |
| *SPEG* | 1.01486 | 2.020706793 | 0.028 |
| *MEST* | 1.00816 | 2.011344214 | 0.004 |
| *PPAP2B* | 1.00257 | 2.003565952 | 0.004 |
| *MT1X* | 1.00088 | 2.001220311 | 0.038 |
| *EPAS1* | 0.995335 | 1.993543381 | 0.004 |
| *ENO2* | 0.993995 | 1.991692603 | 0.004 |
| *TMEM45A* | 0.991327 | 1.988012737 | 0.007 |
| *B3GALNT1* | 0.98677 | 1.981743164 | 0.004 |
| *CHPF* | 0.983777 | 1.977636122 | 0.004 |
| *LINGO1* | 0.983445 | 1.977181071 | 0.004 |
| *CSPG4* | 0.98313 | 1.976749418 | 0.011 |
| *GRIK1* | 0.978111 | 1.969884442 | 0.004 |
| *TGFA* | 0.976867 | 1.968186591 | 0.004 |
| *SIPA1 L2* | 0.975836 | 1.966780559 | 0.004 |
| *FLNB* | 0.968271 | 1.956494426 | 0.018 |
| *CD109* | 0.964911 | 1.951943102 | 0.023 |
| *LRRC49* | 0.961732 | 1.947646702 | 0.007 |
| *MLLT3* | 0.955775 | 1.939621297 | 0.034 |
| *VEGFA* | 0.955466 | 1.939205909 | 0.004 |
| *MXI1* | 0.938912 | 1.917081937 | 0.004 |
| *MIR210HG* | 0.935642 | 1.912741617 | 0.039 |
| *ZFP36L2* | 0.932293 | 1.908306624 | 0.004 |
| *SYTL2* | 0.930037 | 1.90532486 | 0.004 |
| *LGR6* | 0.926289 | 1.900381411 | 0.034 |
| *TMEM198* | 0.925968 | 1.899958623 | 0.043 |
| *MDK* | 0.9257 | 1.899605713 | 0.004 |
| *APOLD1* | 0.918636 | 1.890327233 | 0.004 |
| *FLJ46906* | 0.9126 | 1.882434935 | 0.007 |
| *RND3* | 0.912391 | 1.882162251 | 0.045 |
| *KCNE4* | 0.911253 | 1.880678184 | 0.004 |
| *SORL1* | 0.904571 | 1.871987758 | 0.004 |
| *SCARB1* | 0.902987 | 1.869933546 | 0.011 |
| *NPNT* | 0.902121 | 1.868811426 | 0.013 |
| *FKBP9* | 0.899069 | 1.864862162 | 0.004 |
| *NT5C3* | 0.896159 | 1.861104417 | 0.004 |
| *PDE4D* | 0.894443 | 1.85889206 | 0.032 |
| *C1orf198* | 0.885987 | 1.848028492 | 0.004 |
| *PPARGC1B* | 0.885446 | 1.847335625 | 0.004 |
| *FGFR1* | 0.869414 | 1.826920683 | 0.004 |
| *CABIN1* | 0.863656 | 1.819643716 | 0.013 |
| *ERO1L* | 0.858669 | 1.813364567 | 0.018 |
| *MEG3* | 0.858232 | 1.812815372 | 0.023 |
| *IFITM3* | 0.857451 | 1.811834274 | 0.028 |
| *LOC730101* | 0.855987 | 1.809996616 | 0.043 |
| *FRAS1* | 0.848884 | 1.801107136 | 0.004 |
| *AMD1* | 0.846678 | 1.798355199 | 0.004 |
| *TMEM22* | 0.843261 | 1.794100863 | 0.025 |
| *RORA* | 0.843026 | 1.793808647 | 0.013 |
| *MT3* | 0.842154 | 1.792724753 | 0.004 |
| *SBF2* | 0.841085 | 1.791396882 | 0.004 |
| *ARL4C* | 0.84046 | 1.790620986 | 0.004 |
| *IQSEC1* | 0.839238 | 1.789104926 | 0.032 |
| *HJURP* | 0.836282 | 1.785442905 | 0.013 |
| *C11orf41* | 0.829182 | 1.77667771 | 0.023 |
| *EYA2* | 0.828153 | 1.77541095 | 0.025 |
| *NOTCH3* | 0.826995 | 1.773986462 | 0.013 |
| *KATNAL1* | 0.826141 | 1.772936666 | 0.018 |
| *CSGALNACT1* | 0.819128 | 1.764339261 | 0.011 |
| *PSMD5* | 0.814537 | 1.758733636 | 0.021 |
| *S1PR2* | 0.814434 | 1.758608077 | 0.050 |
| *JAM2* | 0.8112 | 1.75467033 | 0.032 |
| *RP9P* | 0.80781 | 1.7505521 | 0.030 |
| *CXCR4* | 0.806631 | 1.749122097 | 0.025 |
| *VOPP1* | 0.796003 | 1.736284066 | 0.007 |
| *NCALD* | 0.794744 | 1.73476952 | 0.011 |
| *CAV2* | 0.78674 | 1.725171754 | 0.021 |
| *C14orf43* | 0.785832 | 1.724086311 | 0.016 |
| *NEDD4L* | 0.783422 | 1.721208655 | 0.023 |
| *GRB10* | 0.782665 | 1.720305752 | 0.007 |
| *TNIP1* | 0.773008 | 1.708828958 | 0.023 |
| *TSKU* | 0.771003 | 1.706455745 | 0.043 |
| *AK4* | 0.758256 | 1.691444688 | 0.004 |
| *PPFIBP2* | 0.754097 | 1.686575614 | 0.023 |
| *LPL* | 0.75126 | 1.683262292 | 0.016 |
| *CCNG2* | 0.74887 | 1.680476071 | 0.045 |
| *FEM1C* | 0.737755 | 1.667578874 | 0.039 |
| *DDIT4* | 0.711344 | 1.637328726 | 0.036 |
| *PSPH* | 0.702172 | 1.626952352 | 0.016 |
| *C5orf62* | 0.687785 | 1.610808511 | 0.038 |
| *BNIP3L* | 0.662611 | 1.582944858 | 0.043 |
| *ARHGAP21* | 0.660533 | 1.580666489 | 0.045 |

**Table 1b. Genes downregulated in CD133+ vs. CD133- GBM cells.**

| **Gene** | **log2 change** | **fold change** | **FDR-adjusted p value** |
|---|---|---|---|
| *KCNJ6* | 2.82636 | 7.092823266 | 0.004 |
| *LRRN1* | 2.40211 | 5.285756626 | 0.004 |
| *ID3* | 2.35582 | 5.118850954 | 0.004 |
| *IL33* | 2.2916 | 4.895987925 | 0.011 |
| *SCRG1* | 1.97385 | 3.928149942 | 0.004 |
| *NEUROD1* | 1.86324 | 3.638238197 | 0.004 |
| *ID1* | 1.75405 | 3.37304134 | 0.004 |
| *ST6GAL1* | 1.67493 | 3.193038614 | 0.004 |
| *SPP1* | 1.5431 | 2.914200218 | 0.004 |
| *SNCAIP* | 1.52535 | 2.87856542 | 0.011 |
| *GRIK3* | 1.47713 | 2.783943631 | 0.004 |
| *GPR126* | 1.39706 | 2.633643372 | 0.039 |
| *EDNRB* | 1.39034 | 2.621404521 | 0.004 |
| *DCC* | 1.38744 | 2.616140458 | 0.016 |
| *HOPX* | 1.36608 | 2.577692195 | 0.013 |
| *TMEM108* | 1.35151 | 2.551790695 | 0.004 |
| *IQGAP2* | 1.29641 | 2.456169288 | 0.004 |
| *TIMP3* | 1.26842 | 2.408975966 | 0.032 |
| *KIAA1598* | 1.22756 | 2.341706069 | 0.004 |
| *BCAN* | 1.22132 | 2.331599504 | 0.004 |
| *PLAT* | 1.20322 | 2.302530074 | 0.004 |
| *CDH6* | 1.19191 | 2.284549972 | 0.004 |
| *POSTN* | 1.17074 | 2.251271415 | 0.004 |
| *VGF* | 1.16919 | 2.248853998 | 0.004 |
| *TNFRSF19* | 1.14168 | 2.206378035 | 0.004 |
| *HSPA12A* | 1.07027 | 2.099826312 | 0.004 |
| *SLC1A2* | 1.05194 | 2.073315973 | 0.007 |
| *KCNQ2* | 1.03411 | 2.047849936 | 0.004 |
| *CD24* | 1.01029 | 2.014315962 | 0.004 |
| *LAMB1* | 0.957362 | 1.941756102 | 0.018 |
| *FLRT3* | 0.944393 | 1.924379052 | 0.013 |
| *PVRL3* | 0.937611 | 1.915353922 | 0.004 |
| *BDNF* | 0.93551 | 1.912566618 | 0.041 |
| *VCAN* | 0.903931 | 1.871157501 | 0.048 |
| *SHROOM3* | 0.887019 | 1.849350912 | 0.013 |
| *LRRC17* | 0.885076 | 1.84686191 | 0.041 |
| *FST* | 0.884673 | 1.846346083 | 0.048 |
| *RCAN1* | 0.873007 | 1.831476259 | 0.016 |
| *ANXA1* | 0.871862 | 1.830023278 | 0.004 |
| *ODZ2* | 0.869303 | 1.826780127 | 0.016 |
| *C8orf46* | 0.800004 | 1.741105954 | 0.048 |
| *ALCAM* | 0.77189 | 1.707505233 | 0.034 |
| *WISP1* | 0.754421 | 1.686954427 | 0.032 |
| *EYA4* | 0.741246 | 1.671618928 | 0.023 |
| *SACS* | 0.740583 | 1.670850901 | 0.021 |
| *DLC1* | 0.725233 | 1.653167607 | 0.038 |
| *COL5A2* | 0.719459 | 1.646564469 | 0.039 |
| *SPRY4* | 0.708049 | 1.633593465 | 0.03 |

**Table 2. Taqman Assays used in the study.**

| **Target** | **TaqMan Assay** |
|---|---|
| *GPR133-*C-terminal | Hs00914797_m1 |
| *GPR133-*N-terminal | Hs00138665_m1 |
| *HPRT1* | Hs28000695_m1 |
| *GAPDH* | Hs02758991_m1 |
| *PROM1 (CD133)* | Hs00195682_m1 |
| *HIF1A* | Hs00153153 m1 |

**Table 3. Antibodies used for Immunofluorescence staining.**

| **Antigen** | **Company** | **Dilution** |
|---|---|---|
| **GPR133** | LSBio | 1/200 |
| **Ki67** | Abcam | 1/200 |
| **Hiflα** | Bethyl Labs | 1/200 |
| **CA9** | Abcam | 1/200 |
| **Hydroxy-probe (pimo)** | HydroxyProbe | 1/100 |
| **hNA** | Millipore | 1/200 |

### References

1. Vanti WB, Nguyen T, Cheng R, Lynch KR, George SR, O'Dowd BF. Novel human G-protein-coupled receptors. Biochemical and biophysical research communications. 2003;305(1):67-71. PubMed PMID: 12732197.
2. Harmar AJ. Family-B G-protein-coupled receptors. Genome biology. 2001;2(12):REVIEWS3013. Epub 2002/01/16. PubMed PMID: 11790261; PubMed Central PMCID: PMC138994.
3. Bjarnadottir TK, Fredriksson R, Hoglund PJ, Gloriam DE, Lagerstrom MC, Schioth HB. The human and mouse repertoire of the adhesion family of G-protein-coupled receptors. Genomics. 2004;84(1):23-33. doi: 10.1016/j.ygeno.2003.12.004. PubMed PMID: 15203201.
4. Chan YF, Jones FC, McConnell E, Bryk J, Bunger L, Tautz D. Parallel selection mapping using artificially selected mice reveals body weight control loci. Current biology: CB. 2012;22(9):794-800. doi: 10.1016/j.cub.2012.03.011. PubMed PMID: 22445301.
5. Kim JJ, Park YM, Baik KH, Choi HY, Yang GS, Koh I, Hwang JA, Lee J, Lee YS, Rhee H, Kwon TS, Han BG, Heath KE, Inoue H, Yoo HW, Park K, Lee JK. Exome sequencing and subsequent association studies identify five amino acid-altering variants influencing human height. Human genetics. 2012;131(3):471-8. doi: 10.1007/s00439-011-1096-4. PubMed PMID: 21959382.
6. Kim YK, Moon S, Hwang MY, Kim DJ, Oh JH, Kim YJ, Han BG, Lee JY, Kim BJ. Gene-based copy number variation study reveals a microdeletion at 12q24 that influences height in the Korean population. Genomics. 2012. doi: 10.1016/j.ygeno.2012.11.002. PubMed PMID: 23147675.
7. Marroni F, Pfeufer A, Aulchenko YS, Franklin CS, Isaacs A, Pichler I, Wild SH, Oostra BA, Wright AF, Campbell H, Witteman JC, Kaab S, Hicks AA, Gyllensten U, Rudan I, Meitinger T, Pattaro C, van Duijn CM, Wilson JF, Pramstaller PP, Consortium E. A genome-wide association scan of RR and QT interval duration in 3 European genetically isolated populations: the EUROSPAN project. Circ Cardiovasc Genet. 2009;2(4):322-8. doi: 10.1161/CIRCGENETICS.108.833806. PubMed PMID: 20031603; PubMed Central PMCID: PMC2760953.
8. Tonjes A, Koriath M, Schleinitz D, Dietrich K, Bottcher Y, Rayner NW, Almgren P, Enigk B, Richter O, Rohm S, Fischer-Rosinsky A, Pfeiffer A, Hoffmann K, Krohn K, Aust G, Spranger J, Groop L, Bluher M, Kovacs P, Stumvoll M. Genetic variation in GPR133 is associated with height: genome wide association study in the self-contained population of Sorbs. Human molecular genetics. 2009;18(23):4662-8. doi: 10.1093/hmg/ddp423. PubMed PMID: 19729412; PubMed Central PMCID: PMC2773272.
9. Bohnekamp J, Schoneberg T. Cell adhesion receptor GPR133 couples to Gs protein. The Journal of biological chemistry. 2011;286(49):41912-6. doi: 10.1074/jbc.C111.265934. PubMed PMID: 22025619; PubMed Central PMCID: PMC3234928.
10. Bjarnadottir TK, Geirardsdottir K, Ingemansson M, Mirza MA, Fredriksson R, Schioth HB. Identification of novel splice variants of Adhesion G protein-coupled receptors. Gene. 2007;387(1-2):38-48. doi: 10.1016/j.gene.2006.07.039. PubMed PMID: 17056209.
11. Liebscher I, Schon J, Petersen SC, Fischer L, Auerbach N, Demberg LM, Mogha A, Coster M, Simon KU, Rothemund S, Monk KR, Schoneberg T. A Tethered Agonist within the Ectodomain Activates the Adhesion G Protein-Coupled Receptors GPR126 and GPR133. Cell reports. 2014;9(6):2018-26. Epub 2014/12/24. doi: 10.1016/j.celrep.2014.11.036. PubMed PMID: 25533341; PubMed Central PMCID: PMC4277498.
12. Stupp, R. et al. Radiotherapy plus concomitant and adjuvant temozolomide for glioblastoma. The New England journal of medicine 352, 987-996, doi:10.1056/NEJMoa043330 (2005).
13. Singh, S. K. et al. Identification of human brain tumour initiating cells. Nature 432, 396-401, doi:10.1038/nature03128 (2004).
14. Chen, J. et al. A restricted cell population propagates glioblastoma growth after chemotherapy. Nature 488, 522-526, doi:10.1038/nature11287 (2012).
15. Bao, S. et al. Glioma stem cells promote radioresistance by preferential activation of the DNA damage response. Nature 444, 756-760, doi:10.1038/nature05236 (2006).
16. Bayin, N. S., Modrek, A. S. & Placantonakis, D. G. Glioblastoma stem cells: Molecular characteristics and therapeutic implications. World J Stem Cells 6, 230-238, doi:10.4252/wjsc.v6.i2.230 (2014).
17. Hardee, M. E. & Zagzag, D. Mechanisms of glioma-associated neovascularization. The American journal of pathology 181, 1126-1141, doi:10.1016/j.ajpath.2012.06.030 (2012).
18. Rong, Y., Durden, D. L., Van Meir, E. G. & Brat, D. J. 'Pseudopalisading' necrosis in glioblastoma: a familiar morphologic feature that links vascular pathology, hypoxia, and angiogenesis. Journal of neuropathology and experimental neurology 65, 529-539 (2006).
19. Borovski, T. et al. Tumor microvasculature supports proliferation and expansion of glioma-propagating cells. International journal of cancer. Journal international du cancer 125, 1222-1230, doi:10.1002/ijc.24408 (2009).
20. Calabrese, C. et al. A perivascular niche for brain tumor stem cells. Cancer cell 11, 69-82, doi:10.1016/j.ccr.2006.11.020 (2007).
21. Christensen, K., Schroder, H. D. & Kristensen, B. W. CD133 identifies perivascular niches in grade II-IV astrocytomas. Journal of neuro-oncology 90, 157-170, doi: 10.1007/s11060-008-9648-8 (2008).
22. Christensen, K., Schroder, H. D. & Kristensen, B. W. CD133+ niches and single cells in glioblastoma have different phenotypes. Journal of neuro-oncology 104, 129-143, doi:10.1007/s11060-010-0488-y (2011).
23. Seidel, S. et al. A hypoxic niche regulates glioblastoma stem cells through hypoxia inducible factor 2 alpha. Brain: a journal of neurology 133, 983-995, doi: 10.1093/brain/awq042 (2010).
24. Bar, E. E., Lin, A., Mahairaki, V., Matsui, W. & Eberhart, C. G. Hypoxia increases the expression of stem-cell markers and promotes clonogenicity in glioblastoma neurospheres. The American journal of pathology 177, 1491-1502, doi:10.2353/ajpath.2010.091021 (2010).
25. Hjelmeland, A. B. et al. Acidic stress promotes a glioma stem cell phenotype. Cell death and differentiation 18, 829-840, doi:10.1038/cdd.2010.150 (2011).
26. Heddleston, J. M., Li, Z., McLendon, R. E., Hjelmeland, A. B. & Rich, J. N. The hypoxic microenvironment maintains glioblastoma stem cells and promotes reprogramming towards a cancer stem cell phenotype. Cell Cycle 8, 3274-3284 (2009).
27. Heddleston, J. M. et al. Hypoxia inducible factors in cancer stem cells. British journal of cancer 102, 789-795, doi:10.1038/sj.bjc.6605551 (2010).
28. Li, Z. et al. Hypoxia-inducible factors regulate tumorigenic capacity of glioma stem cells. Cancer cell 15, 501-513, doi:10.1016/j.ccr.2009.03.018 (2009).
29. Mendez, O. et al. Knock down of HIF-1alpha in glioma cells reduces migration in vitro and invasion in vivo and impairs their ability to form tumor spheres. Molecular cancer 9, 133, doi: 10.1186/1476-4598-9-133 (2010).
30. Qiang, L. et al. HIF-1 alpha is critical for hypoxia-mediated maintenance of glioblastoma stem cells by activating Notch signaling pathway. Cell death and differentiation 19, 284-294, doi:10.1038/cdd.2011.95 (2012).
31. Batchelor, T. T. et al. Phase II study of cediranib, an oral pan-vascular endothelial growth factor receptor tyrosine kinase inhibitor, in patients with recurrent glioblastoma. Journal of clinical oncology : official journal of the American Society of Clinical Oncology 28, 2817-2823, doi:10.1200/JCO.2009.26.3988 (2010).
32. Lu-Emerson, C. et al. Lessons from anti-vascular endothelial growth factor and anti-vascular endothelial growth factor receptor trials in patients with glioblastoma. Journal of clinical oncology : official journal of the American Society of Clinical Oncology 33, 1197-1213, doi:10.1200/JCO.2014.55.9575 (2015).
33. Jain, R. K. Antiangiogenesis strategies revisited: from starving tumors to alleviating hypoxia. Cancer cell 26, 605-622, doi:10.1016/j.ccell.2014.10.006 (2014).
34. Paola, B. et al. CD133 is essential for glioblastoma stem cell maintenance. Stem Cells, doi:10.1002/stem.1317 (2013).
35. Vanti, W. B. et al. Novel human G-protein-coupled receptors. Biochemical and biophysical research communications 305, 67-71 (2003).
36. Bjarnadottir, T. K. et al. The human and mouse repertoire of the adhesion family of G-protein-coupled receptors. Genomics 84, 23-33, doi:10.1016/j.ygeno.2003.12.004 (2004).
37. Promel, S. et al. The GPS motif is a molecular switch for bimodal activities of adhesion class G protein-coupled receptors. Cell reports 2, 321-331, doi:10.1016/j.celrep.2012.06.015 (2012).
38. Monk, K. R. et al. Adhesion G Protein-Coupled Receptors: From In Vitro Pharmacology to In Vivo Mechanisms. Molecular pharmacology 88, 617-623, doi: 10.1124/mol.115.098749 (2015).
39. Marroni, F. et al. A genome-wide association scan of RR and QT interval duration in 3 European genetically isolated populations: the EUROSPAN project. Circ Cardiovasc Genet 2, 322-328, doi: 10.1161/CIRCGENETICS.108.833806 (2009).
40. Kim, Y. K. et al. Gene-based copy number variation study reveals a microdeletion at 12q24 that influences height in the Korean population. Genomics 101, 134-138, doi:10.1016/j.ygeno.2012.11.002 (2013).
41. Kim, J. J. et al. Exome sequencing and subsequent association studies identify five amino acid-altering variants influencing human height. Human genetics 131, 471-478, doi:10.1007/s00439-011-1096-4 (2012).
42. Tonjes, A. et al. Genetic variation in GPR133 is associated with height: genome wide association study in the self-contained population of Sorbs. Human molecular genetics 18, 4662-4668, doi:10.1093/hmg/ddp423 (2009).
43. Liebscher, I. et al. A Tethered Agonist within the Ectodomain Activates the Adhesion G Protein-Coupled Receptors GPR126 and GPR133. Cell reports 9, 2018-2026, doi:10.1016/j.celrep.2014.11.036 (2014).
44. Bohnekamp, J. & Schoneberg, T. Cell adhesion receptor GPR133 couples to Gs protein. The Journal of biological chemistry 286, 41912-41916, doi:10.1074/jbc.C111.265934 (2011).
45. Bayin, N. S. et al. Selective Lentiviral Gene Delivery to CD133-Expressing Human Glioblastoma Stem Cells. PloS one 9, e116114, doi:10.1371/journal.pone.0116114 (2014).
46. Consortium, G. T. Human genomics. The Genotype-Tissue Expression (GTEx) pilot analysis: multitissue gene regulation in humans. Science 348, 648-660, doi:10.1126/science.1262110 (2015).
47. Human genomics. The Genotype-Tissue Expression (GTEx) pilot analysis: multitissue gene regulation in humans. Science 348, 648-660, doi:10.1126/science.1262110 (2015).
48. McIntyre, A. et al. Carbonic anhydrase IX promotes tumor growth and necrosis in vivo and inhibition enhances anti-VEGF therapy. Clinical cancer research : an official journal of the American Association for Cancer Research 18, 3100-3111, doi:10.1158/1078-0432.CCR-11-1877 (2012).
49. Cline, M. S. et al. Exploring TCGA Pan-Cancer data at the UCSC Cancer Genomics Browser. Scientific reports 3, 2652, doi:10.1038/srep02652 (2013).
50. Jensen, R. L. Brain tumor hypoxia: tumorigenesis, angiogenesis, imaging, pseudoprogression, and as a therapeutic target. Journal of neuro-oncology 92, 317-335, doi:10.1007/s11060-009-9827-2 (2009).
51. Feng, H. et al. Protein kinase A-dependent phosphorylation of Dock180 at serine residue 1250 is important for glioma growth and invasion stimulated by platelet derived-growth factor receptor alpha. Neuro-oncology 17, 832-842, doi:10.1093/neuonc/nou323 (2015).
52. Sugimoto, N. et al. Targeted activation of PKA and Epac promotes glioblastoma regression. Mol Clin Oncol 1, 281-285, doi:10.3892/mco.2013.65 (2013).
53. Kang, T. W. et al. Growth arrest and forced differentiation of human primary glioblastoma multiforme by a novel small molecule. Scientific reports 4, 5546, doi:10.1038/srep05546 (2014).
54. Lee, J. et al. Tumor stem cells derived from glioblastomas cultured in bFGF and EGF more closely mirror the phenotype and genotype of primary tumors than do serum-cultured cell lines. Cancer cell 9, 391-403, doi:10.1016/j.ccr.2006.03.030 (2006).
55. Basu-Roy, U. et al. Sox2 antagonizes the Hippo pathway to maintain sternness in cancer cells. Nat Commun 6, 6411, doi:10.1038/ncomms7411 (2015).
56. Sturm, D. et al. Hotspot mutations in H3F3A and IDH1 define distinct epigenetic and biological subgroups of glioblastoma. Cancer cell 22, 425-437, doi:10.1016/j.ccr.2012.08.024 (2012).
57. Dobin, A. et al. STAR: ultrafast universal RNA-seq aligner. Bioinformatics 29, 15-21, doi:10.1093/bioinformatics/bts635 (2013).
58. Anders, S., Pyl, P. T. & Huber, W. HTSeq--a Python framework to work with high-throughput sequencing data. Bioinformatics 31, 166-169, doi:10.1093/bioinformatics/btu638 (2015).
59. Love, M. I., Huber, W. & Anders, S. Moderated estimation of fold change and dispersion for RNA-seq data with DESeq2. Genome biology 15, 550, doi:10.1186/s13059-014-0550-8 (2014).
60. Walchli, T. et al. Quantitative assessment of angiogenesis, perfused blood vessels and endothelial tip cells in the postnatal mouse brain. Nature protocols 10, 53-74, doi:10.1038/nprot.2015.002 (2015).

### LIST OF SEQUENCES:

| **SEQ ID NO** | **TYPE** | **SOURCE** | **SEQUENCE** |
|---|---|---|---|
| 1 | Protein | Human | |
| 2 | Protein | Human | |
| 3 | Protein | Human | |
| 4 | Protein | Human | |
| | | | |
| 5 | DNA | Synthetic | GGGATCATAGATGTGAATTAA |
| 6 | DNA | Synthetic | GGAGTCACGCTTCTCTATTAC |
| 7 | DNA | Synthetic | CCTGCAGGGACTGTTCATATT |
| 8 | Protein | Human | |
| 9 | Protein | Human | |
| 10 | Protein | Human | TRKQHSEATNSSNRC |
| 11 | Protein | Human | CSSGEGVWSNHG |
| 12 | Protein | Human | CSSARTSNAKPFHSD |
| 13 | Protein | Human | LMNGTRPGMASTKLSC |
| 14 | DNA | Synthetic | CCTAAGGTTAAGTCGCCCTCG |
| 15 | Protein | Human | |
| 16 | Protein | Human | |
| | | | |
| 17 | Protein | Human | |
| 18 | Protein | Human | |
| | | | |
| 19 | Protein | Human | QTALNLTKTFLKAVGEILLLPGWIALS |
| 20 | Protein | Human | MEKGTELLVSPSQSGPGGDQPLLVKHR |
| 21 | Protein | Human | LITVHLKHRL |
| 22 | Protein | Human | MHRVCFLSFQ |
| 23 | Protein | Human | GASRTHKLTVLPSRNATFVYSNDSAYSNLSATV |
| 24 | DNA | Synthetic | GCCGCTCAATTTATGAATATT |

## Claims

1. A GPR133 inhibitor for use in inhibiting growth, self-renewal, or tumorigenicity of a glioma cancer cell by exposing said cancer cell to the GPR133 inhibitor.

2. The GPR133 inhibitor for use according to claim 1, wherein said glioma cell is a high-grade glioma cell.

3. The GPR133 inhibitor for use according to claim 1, wherein said high-grade glioma cell is a glioblastoma cell.

4. The GPR133 inhibitor for use according to claim 1, wherein said glioma cancer cell is a CD133-expressing (CD133+) cell.

5. The GPR133 inhibitor for use according to any one of claims 1-4, wherein said glioma cancer cell is a glioma stem cell.

6. The GPR133 inhibitor for use according to any one of claims 1-5, wherein said GPR133 inhibitor is selected from the group consisting of a nucleic acid-based molecule, an antibody, a peptide, and a small molecule.

7. The GPR133 inhibitor for use according to claim 6, wherein said GPR133 inhibitor is selected from the group consisting of interfering RNA (RNAi) molecules, dsRNA, RNA polymerase III transcribed DNAs, and antisense nucleic acids.

8. The GPR133 inhibitor for use according to claim 7, wherein said RNAi molecule is shRNA or siRNA.

9. The GPR133 inhibitor for use according to claim 8, wherein said shRNA comprises a nucleic acid sequence selected from the group consisting of GGGATCATAGATGTGAATTAA (SEQ ID NO: 5), GGAGTCACGCTTCTCTATTAC (SEQ ID NO: 6), and CCTGCAGGGACTGTTCATATT (SEQ ID NO: 7).

10. The GPR133 inhibitor for use according to any one of claims 1-5, wherein said GPR133 inhibitor is selected from the group consisting of genome editing methods for inhibiting expression of the GPR133 gene, which involve the use of Clustered Regularly Interspaced Short Palindromic Repeats (CRISPR)/Cas9 gene systems, methods which involve the use of zinc finger nucleases (ZFNs), and methods which involve the use of transcription activator-like effector nucleases (TALENs).

11. The GPR133 inhibitor for use according to claim 6, wherein said GPR133 inhibitor is an antibody.

12. The GPR133 inhibitor for use according to claim 11, wherein said antibody recognizes an amino acid sequence selected from the group consisting of: AVVLSLIDTIDTVMGHVSSNLHGSTPQVTVEGSSAMAEFSVAKILPKTVNSSHYRFPAHGQS FIQIPHEAFHRHAWSTVVGLLYHSMHYYLNNIWPAHTKIAEAMHHQDC (SEQ ID NO: 9), TRKQHSEATNSSNRC (SEQ ID NO: 10), CSSGEGVWSNHG (SEQ ID NO: 11), CSSARTSNAKPFHSD (SEQ ID NO: 12), and LMNGTRPGMASTKLSC (SEQ ID NO: 13).

13. The GPR133 inhibitor for use according to claim 11, wherein said antibody selectively recognizes GPR133 isoform 1 (Uniprot Q6QNK2.1; SEQ ID NO: 15).

14. An *in vitro* method for determining whether a subject diagnosed with a glioma is at an increased risk for progression of said glioma and/or reduced survival comprising:
(a) determining an expression level of GPR133 in glioma cancer cells of the subject,
(b) comparing the expression level of GPR133 determined in step (a) to GPR133 expression level in corresponding normal cells of the same tissue origin or to a database's mean value of GPR133 mRNA expression in the same type of cancer, and
(c) determining that the subject is at an increased risk for cancer progression and/or reduced survival if the expression level of GPR133 in cancer cells of the subject is higher than in the corresponding normal cells or the database's mean value of GPR133 mRNA expression in the same type of cancer.

## Patentansprüche

1. Ein GPR133-Inhibitor zur Verwendung bei der Inhibierung des Wachstums, der Selbsterneuerung oder der Kanzerogenität einer Gliomkrebszelle durch Aussetzen der Krebszelle dem GPR133-Inhibitor.

2. Der GPR133-Inhibitor zur Verwendung nach Anspruch 1, wobei die Gliomzelle eine hochgradige Gliomzelle ist.

3. Der GPR133-Inhibitor zur Verwendung nach Anspruch 1, wobei die hochgradige Gliomzelle eine Glioblastomzelle ist.

4. Der GPR133-Inhibitor zur Verwendung nach Anspruch 1, wobei die Gliomkrebszelle eine CD133-exprimierende (CD133+)-Zelle ist.

5. Der GPR133-Inhibitor zur Verwendung nach einem der Ansprüche 1-4, wobei die Gliomkrebszelle eine Gliomstammzelle ist.

6. Der GPR133-Inhibitor zur Verwendung nach einem der Ansprüche 1-5, wobei der GPR133-Inhibitor ausgewählt ist aus der Gruppe bestehend aus einem Nukleinsäure-basierten Molekül, einem Antikörper, einem Peptid und einem kleinen Molekül.

7. Der GPR133-Inhibitor zur Verwendung nach Anspruch 6, wobei der GPR133-Inhibitor ausgewählt ist aus der Gruppe bestehend aus RNAi ("interfering RNA")-Molekülen, dsRNA, RNA-Polymerase III transkribierten DNAs und Antisinn-Nukleinsäuren.

8. Der GPR133-Inhibitor zur Verwendung nach Anspruch 7, wobei das RNAi-Molekül shRNA oder siRNA ist.

9. Der GPR133-Inhibitor zur Verwendung nach Anspruch 8, wobei die shRNA eine Nukleinsäuresequenz ausgewählt ist aus der Gruppe bestehend aus GGGATCATAGATGTGAATTAA (SEQ ID NR: 5), GGAGTCACGCTTCTCTATTAC (SEQ ID NR: 6) und CCTGCAGGGACTGTTCATATT (SEQ ID NR: 7) ist.

10. Der GPR133-Inhibitor zur Verwendung nach einem der Ansprüche 1-5, wobei der GPR133-Inhibitor ausgewählt ist aus der Gruppe bestehend aus Genom-editierenden Verfahren zur Inhibierung der Expression des GPR133-Inhibitors, die die Verwendung von CRISPR ("*Clustered Regularly Interspaced Short Palindromic Repeats*")/Cas9-Gen-Systemen einschließen, Verfahren, die die Verwendung von Zinkfingernukleasen (ZFNs) einschließen, und Verfahren, die die Verwendung von Transkriptionsaktivator-ähnliche Effektor-Nukleasen (TALENs) einschließen.

11. Der GPR133-Inhibitor zur Verwendung nach Anspruch 6, wobei der GPR133-Inhibitor ein Antikörper ist.

12. Der GPR133-Inhibitor zur Verwendung nach Anspruch 11, wobei der Antikörper eine Aminosäuresequenz ausgewählt aus der Gruppe bestehend aus: AVVLSLIDTIDTVMGHVSSNLHGSTPQVTVEGSSAMAEFSVAKILPKTVNSSHYRFPAHGQS-FIQIPHEAFHRHAWSTVVGLLYHSMHYYLNNIWPAHTKIAEAMHHQDC (SEQ ID NR: 9), TRKQHSEATNSSNRC (SEQ ID NR: 10), CSSGEGVWSNHG (SEQ ID NR: 11), CSSARTSNAKPFHSD (SEQ ID NR: 12) und LMNGTRPGMASTKLSC (SEQ ID NR: 13) erkennt.

13. Der GPR133-Inhibitor zur Verwendung nach Anspruch 11, wobei der Antikörper selektiv die GPR133-Isoform 1 (Uniprot Q6QNK2.1; SEQ ID NR: 15) erkennt.

14. Ein *in vitro*-Verfahren zur Bestimmung ob bei einem Individuum, bei dem ein Glioma diagnostiziert wurde, ein erhöhtes Risiko für das Fortschreiten des Glioms und/oder verminderte Überlebenschancen bestehen, umfassend:
(a) Bestimmen des Expressionslevels an GPR133 in Gliomkrebszellen des Individuums,
(b) Vergleichen des in Schritt (a) bestimmten Expressionslevels an GPR133 mit dem GPR133-Expressionslevel in den entsprechenden normalen Zellen vom gleichen Gewebeursprung oder mit einem Datenbank-Mittelwert der GPR133 mRNA-Expression in der gleichen Krebsart, und
(c) Bestimmen, dass bei einem Individuum ein erhöhtes Risiko für das Fortschreiten des Glioms und/oder verminderte Überlebenschancen bestehen wenn der Expressionslevel an GPR133 in den Krebszellen des Individuums höher ist als in den entsprechenden normalen Zellen vom gleichen Gewebeursprung oder mit einem Datenbank-Mittelwert der GPR133 mRNA-Expression in der gleichen Krebsart.

## Revendications

1. Inhibiteur GPR133 pour l'utilisation dans l'inhibition de la croissance, de l'auto-renouvellement, ou de la tumorigénicité d'une cellule cancéreuse de gliome par exposition de ladite cellule cancéreuse à l'inhibiteur GPR133.

2. Inhibiteur GPR133 pour l'utilisation selon la revendication 1, ladite cellule de gliome étant une cellule de gliome de haut grade.

3. Inhibiteur GPR133 pour l'utilisation selon la revendication 1, ladite cellule de gliome de haut grade étant une celle de glioblastome.

4. Inhibiteur GPR133 pour l'utilisation selon la revendication 1, ladite cellule cancéreuse de gliome étant une cellule exprimant CD133 (CD133+).

5. Inhibiteur GPR133 pour l'utilisation selon l'une quelconque des revendications 1 à 4, ladite cellule cancéreuse de gliome étant une cellule souche de gliome.

6. Inhibiteur GPR133 pour l'utilisation selon l'une quelconque des revendications 1 à 5, ledit inhibiteur GPR133 étant sélectionné dans le groupe constitué d'une molécule à base d'acide nucléique, d'un anticorps, d'un peptide, et d'une petite molécule.

7. Inhibiteur GPR133 pour l'utilisation selon la revendication 6, ledit inhibiteur GPR133 étant sélectionné dans le groupe constitué des molécules d'ADN interférant (ARNi), d'ADNsd, des ADN transcrits de l'ARN polymérase III, et des acides nucléiques antisens.

8. Inhibiteur GPR133 pour l'utilisation selon la revendication 7, ladite molécule d'ARNi étant l'ARNsh ou l'ARNsi.

9. Inhibiteur GPR133 pour l'utilisation selon la revendication 8, ledit ARNsh comprenant une séquence d'acides nucléiques sélectionnée dans le groupe constitué de GGGATCATAGATGTGAATTAA (SEQ ID n° : 5), GGAGTCACGCTTCTCTATTAC (SEQ ID n° : 6), et CCTGCAGGGACTGTTCATATT (SEQ ID n° : 7).

10. Inhibiteur GPR133 pour l'utilisation selon l'une quelconque des revendications 1 à 5, ledit inhibiteur GPR133 étant sélectionné dans le groupe constitué des procédés d'édition de génome pour inhiber l'expression du gène GPR133, qui impliquent l'utilisation des systèmes de répétition palindromiques courts régulièrement interespacés en amas/gène Cas9, des procédés qui impliquent l'utilisation des nucléases en doigt de zinc (ZFN), et des procédés qui impliquent l'utilisation des nucléases effectrices type activateur de transcription (TALEN).

11. Inhibiteur GPR133 pour l'utilisation selon la revendication 6, ledit inhibiteur GPR133 étant un anticorps.

12. Inhibiteur GPR133 pour l'utilisation selon la revendication 11, ledit anticorps reconnaissant une séquence d'acides aminés sélectionnée dans le groupe constitué de : AVVLSLIDTIDTVMGHVSSNLHGSTPQVTVEGSSAMAEFSVAKILPKTVNSSHYRF PAHGQSFIQIPHEAFHRHAWSTVVGLLYHSMHYYLNNIWPAHTKIAEAMHHQDC (SEQ ID n° : 9), TRKQHSEATNSSNRC (SEQ ID n° : 10), CSSGEGVWSNHG (SEQ ID n° : 11), CSSARTSNAKPFHSD (SEQ ID n° : 12), et LMNGTRPGMASTKLSC (SEQ ID n° : 13).

13. Inhibiteur GPR133 pour l'utilisation selon la revendication 11, ledit anticorps reconnaissant sélectivement l'isoforme 1 de GPR133 (Uniprot Q6QNK2.1 ; SEQ ID n° : 15) .

14. Procédé *in vitro* de détermination du fait qu'un sujet ayant reçu un diagnostic de gliome présente un risque accru de progression dudit gliome et/ou de survie réduite comprenant :
(a) la détermination d'un niveau d'expression de GPR133 dans des cellules cancéreuses de gliome du sujet,
(b) la comparaison du niveau d'expression de GPR133 déterminé dans l'étape (a) au niveau d'expression de GPR133 dans des cellules normales correspondantes du tissu de même origine ou d'une valeur moyenne de base de données de l'expression de l'ARNm de GPR133 dans le même type de cancer, et
(c) la détermination que le sujet présente un risque accru de progression du cancer et/ou de survie réduite si le niveau d'expression de GPR133 dans les cellules cancéreuses du sujet est supérieur à celui dans les cellules normales correspondantes ou à la valeur moyenne de la base de données de l'expression de l'ARNm de GPR133 dans le même type de cancer.
